# EUROPEAN PATENT SPECIFICATION

(11) **EP 4 136 238 B1**
(45) Date of publication and mention of the grant of the patent: **10.12.2025**
(21) Application number: 21788960.9
(22) Date of filing: 14.04.2021
(51) Int. Cl.: C12N 15/63, A61P 35/00, A61P 25/16, C12Q 1/6806

(54) **A SYSTEM FOR THREE-WAY COMBINATORIAL CRISPR SCREENS FOR ANALYSING TARGET INTERACTIONS AND METHODS THEREOF**
SYSTEM FÜR DREIFACH KOMBINATORISCHE CRISPR-SCREENS ZUM ANALYSIEREN VON ZIELINTERAKTIONEN UND VERFAHREN DAVON
SYSTÈME POUR ÉCRANS CRISPR COMBINATOIRES À TROIS VOIES POUR ANALYSER DES INTERACTIONS CIBLES ET PROCÉDÉS ASSOCIÉS

(30) Priority: 16.04.2020 US 202063010877 P
(43) Date of publication of application: 22.02.2023
(73) Proprietor: The University of Hong Kong, Hong Kong Special Administrative Region (CN)
(72) Inventor: WONG, Siu Lun, Hong Kong, Pokfulam Road Hong Kong (CN); ZHOU, Peng, Hong Kong, Pokfulam Road Hong Kong (CN); HO, SikYin, Hong Kong, Pokfulam Road Hong Kong (CN)
(74) Representative: HGF
(86) International application number: PCT/CN2021/087250
(87) International publication number: WO 2021/208971

(56) References cited:
- WO-A1-2017/075294
- WO-A1-2018/112423
- WO-A2-2016/070037
- CN-A- 106 637 421
- KYUHO HAN ET AL: "Synergistic drug combinations for cancer identified in a CRISPR screen for pairwise genetic interactions", NATURE BIOTECHNOLOGY, vol. 35, no. 5, 20 March 2017 (2017-03-20), New York, pages 463 - 474, XP055544433, ISSN: 1087-0156, DOI: 10.1038/nbt.3834
- FRAMPTON JAMES E.: "Olaparib: A Review of Its Use as Maintenance Therapy in Patients with Ovarian Cancer", BIODRUGS , vol. 29, no. 2, 22 April 2015 (2015-04-22), NZ , pages 143 - 150, XP009538570, ISSN: 1173-8804, DOI: 10.1007/s40259-015-0125-6
- ALAN S. L.WONG, GIGI C. G. CHOI, CHERYL H. CUI, GABRIELA PREGERMIG, PAMELA MILANI, MIRIAM ADAM, SAMUEL D. PERLIA,SAMUEL W. KAZER, : "Multiplexed barcoded CRISPR-Cas9 screening enabled by CombiGEM", PROCEEDINGS OF THE NATIONAL ACADEMY OF SCIENCES, NATIONAL ACADEMY OF SCIENCES, vol. 113, no. 9, 1 March 2016 (2016-03-01), pages 2544 - 2549, XP002775745, ISSN: 0027-8424, DOI: 10.1073/pnas.1517883113

## Description

### 1. FIELD

Provided herein is a system for CRISPR-based multi-gene knockout screening.

### 2. BACKGROUND

Despite the promise of combination therapies to enhance treatment efficacy for various diseases (Al-Lazikani et al., 2012), only a limited number of effective combinations, especially those comprising three or more drugs (Table S1), have been discovered so far. Drug combination effect is difficult to predict due to unanticipated synergy or antagonism, and is not simply the sum of the effects brought by each drug (Borisy et al., 2003). Microplate arrays are coupled to robotics systems to screen large panels of drug combinations. However, as the number of experiments grows exponentially with the number of drugs and the order of combinatorial complexity being studied, such approach can become prohibitively expensive. RNA interference and Clustered Regularly Interspaced Short Palindromic Repeats (CRISPR)-based genetic perturbation systems have been applied to facilitate the screening of effective drug target pairs (Doench, 2018; Wong et al., 2016b), but no library assembly and screening method has been validated to simultaneously evaluate more than two targets. This could be attributed to the relatively low and variable cleavage efficiency for polycistronic systems to express multiple RNAs (Han et al., 2017; Xu et al., 2017), and/or the characterization of high-order combinations requires large-scale oligo synthesis and high sequencing costs (elaborated in Design). Mathematical models have been developed for predicting three-way and higher-order drug interactions (Cokol et al., 2017; Wood et al., 2012; Zimmer et al., 2016), but high-throughput methods are needed to experimentally validate sets of potential combinations. Breaking through the bottlenecks, here we establish and validate an extensible platform (named CombiGEM-CRISPR v2.0) for rapid screening of disease-alleviating gene knockouts to study high-order genetic interactions, identify potential therapeutic target combinations, and deploy their matching drug regimens for further testing. WO2016/070037 discloses methods and compositions that enable rapid generation of high-order combinations of genetic elements comprising a guide RNA and a scaffold sequence, and a barcode, and provides methods for screening in which several genes are targeted by guide RNAs.

### 3. SUMMARY

The invention herein is as described in the claims.

Provided is a CRISPR-based multi-gene knockout screening system and new toolkits for extensible assembly of barcoded high-order combinatorial guide RNA libraries *en masse.* We apply this system for systematically identifying not only pairwise but also three-way synergistic therapeutic target combinations, and successfully validated double and triple combination regimens for suppression on cancer cell growth and protection against Parkinson's disease-associated toxicity. This system overcomes the practical challenges to experiment on a large number of high-order genetic and drug combinations and is applicable for uncovering the rare synergistic interactions between druggable targets.

Systematically characterizing genetic interactions among multiple (i.e. more than two)elements. One application of this system is to enable high-throughput screening of disease alleviating genetic combinations to identify two-way and even three-way synergistic drug combinations as potential treatment regimens. Drug combination effect is difficult to predict due to unanticipated synergy or antagonism and is not simply the sum of the effects brought by each drug. Discovering effective drug combinations for diseases has been a major challenge because of the technical difficulties in systematically screening a vast number of possible combinations. For example, microplate arrays are coupled to robotics systems to screen large panels of drug combinations. However, as the number of experiments grows exponentially with the number of drugs and the order of combinatorial complexity being studied, such approach requiring a large amount of drugs can become prohibitively expensive. Thus, despite the promise of combination therapies to enhance treatment efficacy for various diseases, only a limited number of effective combinations, especially those comprising more than two drugs, have been discovered so far.

Described herein but not forming part of the invention is a system for multiplexed genome editing or a two or three-way combinatorial CRISPR screening comprising: a lentiviral vector comprising human U6 (hU6) promoter, mouse U6 (mU6) promoter and human H1 (hH1) promoter expressing an array of three or more barcoded guide RNAs ("gRNAs") oligo pairs, the promoters having a 3' end comprising modified hU6, mU6 and hH1 promoter sequences for paired annealing of the barcoded gRNAs oligo pairs.

Described herein but not forming part of the invention is a system for multiplexed genome editing or a two or three-way combinatorial CRISPR screening comprising: a lentiviral vector comprising human U6 (hU6) promoter, mouse U6 (mU6) promoter and human H1 (hH1) promoter expressing an array of three or more barcoded guide RNAs ("gRNAs") oligo pairs, the hU6 promoter having an unmodified promoter sequence at 3' end and the mU6 and hH1 promoters having modified promoter sequences at 3' end for paired annealing of the barcoded gRNAs oligo pairs.

In one embodiment, the paired annealing of the barcoded gRNAs oligo pairs form RNA scaffolds.

In one embodiment, the combinatorial gRNA library is assembled by CombiGEM-CRISPR v2.0.

In one embodiment, the lentiviral vector transfects human cells and the barcoded gRNAs are delivered to the human cells.

In one embodiment, the system further comprising quantitation of barcoded gRNAs using next-generation sequencing at a time point post transfection.

In one embodiment, the three-way combinatorial CRISPR screen is a high-throughput screen.

In one embodiment, the gRNAs form a RNA scaffold sequence comprising the same 3' end of the modified hU6, mU6 and hH1 promoter sequences as the combinatorial gRNA libraries.

In one embodiment, the gRNAs form a RNA scaffold sequence comprising the same 3' end of the hU6, mU6 and hH1 promoter sequences as the combinatorial gRNA libraries.

Described herein but not forming part of the invention is a method to screen for at least a three-way drug target combination; said method comprises: (i) providing a gRNA library targeting druggable genes of HGSOC wherein each gene comprises an array of 3 gRNAs; (ii) transfecting human cells; and (iii) quantifying barcoded gRNAs using next-generation sequencing.

Described herein but not forming part of the invention is a system to screen for at least a three-way drug target combination comprising: (i) providing a lentiviral three-way combinatorial gRNA expressing construct that express gRNAs; (ii) transfecting human cells with fluorescence reporter gene; and (iii) measuring percentage of cell population positive for fluorescence at a time period post-transfection.

In one embodiment, the fluorescence is measured using a flow cytometry and wherein the fluorescence is GFP, RFP and BFP fluorescence.

In one embodiment, the gRNAs target an exonic regions of green (GFP), red(RFP), and blue (BFP) fluorescent protein reporter genes.

In one embodiment, the human cells are ovarian cancer cells.

In one embodiment, the ovarian cancer cells are high-grade serous ovarian cancer ("HGSOC") cells.

In one embodiment, the ovarian cancer cells are OVCAR8-ADR and OVCAR8-ADR-Cas9. Described herein but not forming part of the invention is a method to screen for at least a three-way drug target combination comprising the steps of: (i) providing a lentiviral three-way combinatorial gRNA expressing construct that express gRNAs; (ii) transfecting human cells with fluorescence reporter gene; and (iii) measuring percentage of cell population positive for fluorescence at a time period post-transfection.

In one embodiment, the method further comprising validation of the three-way drug target combination by matching a drug to the drug target.

In one embodiment, the three-way drug target combinations provides a three-drug regimen for a disease.

In one embodiment, the fluorescence is GFP, RFP or BFP.

In one embodiment, the at least a three-way drug target combination are synergistic combinations.

In one embodiment, the disease is cancer or Parkinson's disease.

Described herein but not forming part of the invention is a method to treat HGSOC comprising administering drugs that targets PARP1, DNMT1, CDK2, FKBP1A or a combination thereof.

In one embodiment, the drug comprises Olaparib (OLA), azacitdine (AZA), seliciclib (SEL), sirolimus (SIR), or a combination thereof.

In one embodiment, the drug comprises OLA and AZA.

Provided herein is a system for CRISPR-based multi-gene knockout screening comprising a barcoded gRNA expression cassette comprising: (i) a first promoter operatively linked to a first gRNA; (ii) a second promoter operatively linked to a second gRNA; (iii) a third promoter operatively linked to a third gRNA; and (iv) three barcoded gRNA sequencing region, wherein the gRNA expression cassette is in a single vector.

In one embodiment, the promoters are human U6, mouse U6, and Human H1 promoters and the three barcoded gRNA are modified gRNA scaffold variants.

In one embodiment, the promoters comprises a modified 3' end sequence which are complementary to the modified gRNA scaffold variants, said 3' end sequence anneals to the modified gRNA scaffold variants.

In one embodiment, the system further comprising: (i) pooled digestion and ligation of the annealed 3' end sequence and the gRNA scaffold variants to form an assembly of pooled barcoded combinatorial gRNA library.

In one embodiment, the expression cassettes knockout target GFP gene in OVACR8-ADR-Cas9 cells.

In one embodiment, the gRNA scaffold variants comprises: (i) higher on-target activity than wild-type scaffold; (ii) low off-target activities; and (iii) high on-to-off target activity.

Provided herein is a system for CRISPR-based multi-gene knockout screening comprising a barcoded gRNA expression cassette comprising v3.11, v.3.12 or v.3.13.

### 4. BRIEF SUMMARY OF THE DRAWINGS

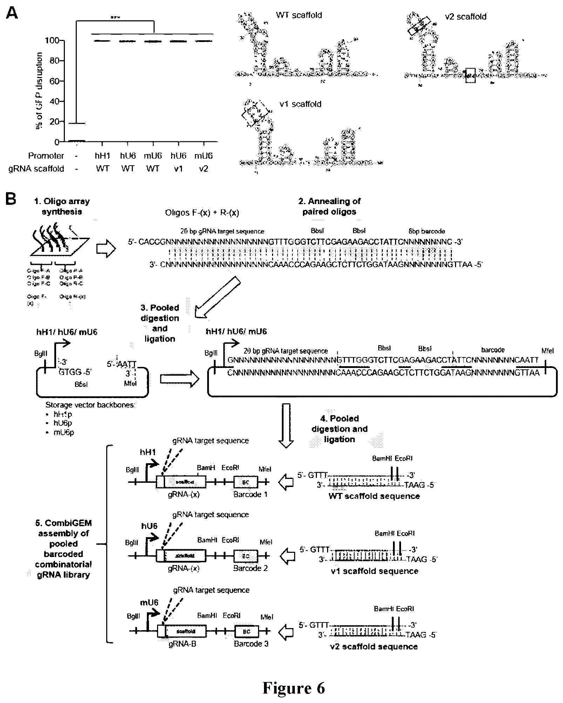

**Figure 1****. Evaluation of the extensibility of existing methods and possible toolkits for assembling three-way gRNA combinations for screening.**
**Figure 2****. Overview of CombiGEM-CRISPR v2.0**
   The workflow starts with the synthesis of barcoded gRNA oligo pairs, which are annealed and cloned into gRNA expression vectors in pooled format. Only one set of oligos is needed for building the libraries of higher-order gRNA combinations for multiplexed CRISPR screens, as the 3' end of promoters are sequence-adapted to the sticky ends of the annealed oligos. Barcoded combinatorial gRNA library is assembled multiplicatively using one-pot reactions as described in Figure 6, and delivered into human cells by lentiviruses. Barcoded representations within cell pools are quantified using next-generation sequencing. Then, validation is done by using matching drugs. Pre-assembled libraries can also be flexibly extended to higher-order ones, or a more focused library can be constructed using a subset of the same oligos, for a secondary screen.

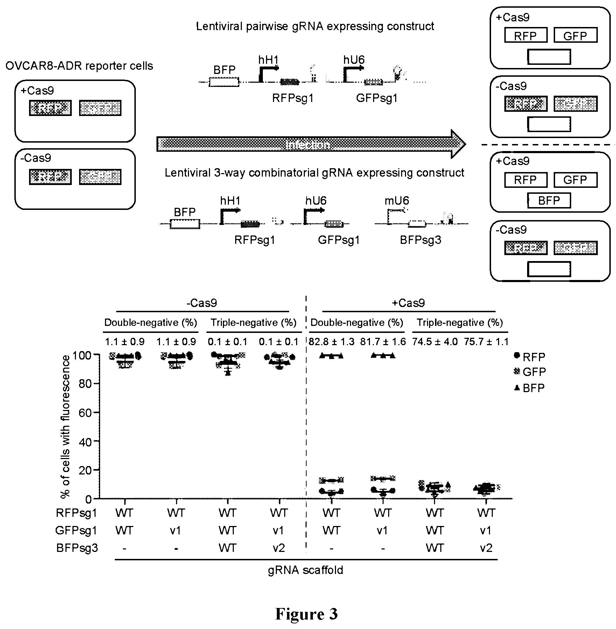

**Figure 3****. Functional disruption of multiple genes using a CRISPR-Cas9-based multi-gene knockout system**
   Lentiviral delivery of combinatorial gRNA expression constructs efficiently disrupt multiple target genes. Flow cytometry was used to measure the percentage of cell populations positive for GFP, RFP, and BFP fluorescence at day 11-14 post-infection in OVCAR8-ADR and OVCAR8-ADR-Cas9 cells. Data are mean ± SD, n = 3 biological replicates.

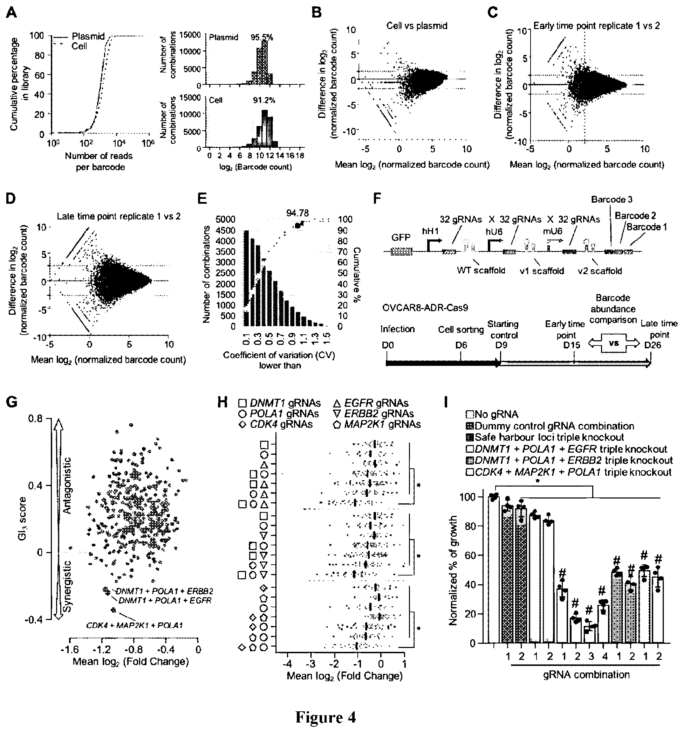

**Figure 4****. A CRISPR-based triple-gene knockout screen identifies synergistic three-way combinations that inhibit ovarian cancer cell growth**
   (A) Distributions of barcode reads in the plasmid and infected OVCAR8-ADR-Cas9 cell pools. A high-coverage three-way combinatorial gRNA library (99.8% of all expected gRNA combinations; 32,705 out of 32,768) was obtained in both the plasmid and cell pools. Most barcoded gRNA combinations were detected within a 5-fold range from the mean barcode reads per combination (highlighted by the shaded areas).
   (B) High correlation between barcode representations (normalized barcode counts) within the plasmid pool and infected cell pool indicates efficient lentiviral delivery of the three-way combinatorial gRNA library into cells. The horizontal dotted lines in the Bland-Altman plots indicate the 95% limits of agreement.
   (C-D) High reproducibility for barcode representations between two biological replicates in cells cultured for 15-day (C), and 26-day (D) post-infection with the three-way combinatorial gRNA library. The horizontal dotted lines in the Bland-Altman plots indicate the 95% limits of agreement. The vertical dash line indicates the threshold of 100 raw barcode counts.
   (E) The coefficient of variation (CV; defined as SD/mean of the fold changes of normalized barcode counts for 26-day versus 15-day cultured cells) was determined for the two biological replicates. Over 94.8% of pairwise gRNA combinations had a CV of < 1 in the screen.
   (F) OVCAR8-ADR-Cas9 cells infected with the barcoded three-way combinatorial gRNA library were cultured for 15 and 26 days. Barcode representations within the cell pools were quantified using Illumina HiSeq. The barcoded library vector uses hH1-gRNA-WT scaffold, hU6-gRNA-v1 scaffold, and mU6-gRNA-v2 scaffold in the first, second, and third expression cassettes, respectively.
   (G) A plot of screen data showing the abundance changes of each barcoded gRNA combinations at day 26 versus day 15 post-infection (in mean log₂ (Fold Change); x-axis) and their genetic interaction (GI₃) score (y-axis). Hit combinations, *DNMT1* + *POLA1* + *EGFR*, *DNMT1* + *POLA1* + *ERBB2*, *and CDK4* + *MAP2K1* +*POLA1*, are highlighted in red. Data were collected from two biological replicates.
   (H) Comparisons of the mean log₂ (Fold Change) of three-way gRNA hit combinations with their constituent single and pairwise gRNA combinations (see STAR Methods for details). Statistical significance was analysed by one-way ANOVA with Dunnett's post hoc test. * indicates P < 0.05.
   (I) Viability, determined by MTT assay, of OVCAR8-ADR-Cas9 cells infected with the indicated triple-gene knockouts and controls. Data shown are mean ± SD from biological replicates (n = 4). Statistical significance was analysed by one-way ANOVA with Tukey's post hoc test. * indicates P < 0.05; ^{#}P < 0.05 indicates the comparisons with the safe harbour loci triple knockouts.

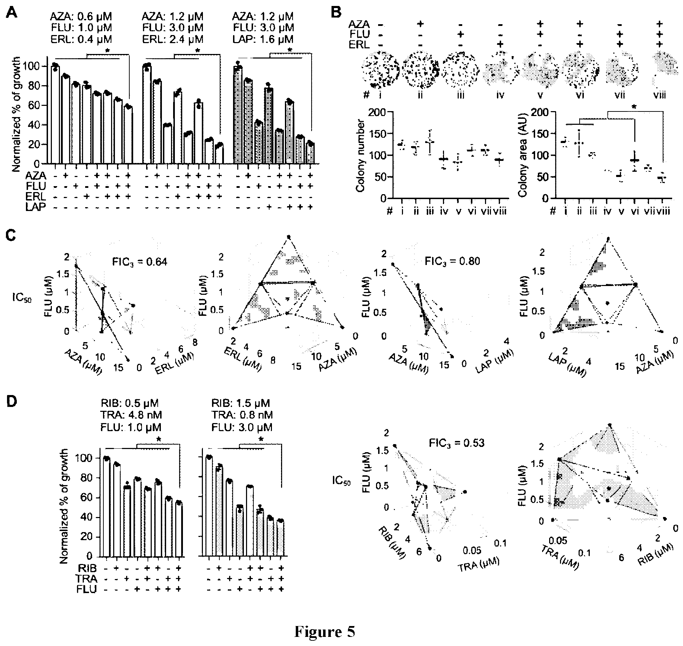

**Figure 5****. Validation of screen hits with matching three-drug regimens**
   (A) Viability, determined by MTT assay, of OVCAR8-ADR cells treated with AZA-, FLU- and/or ERL. Data shown are mean ± SD from biological replicates (n = 3). Statistical significance was analysed by one-way ANOVA with Dunnett's post hoc test, * indicates P < 0.05.
   (B) Colony formation assay of AZA-, FLU- and/or ERL- treated OVCAR8-ADR cells. The colony numbers and areas were quantified. Data shown are mean ± SD from biological replicates (n = 3). Statistical significance was analysed by one-way ANOVA with Dunnett's post hoc test, * indicates P < 0.05.
   (C) Surface plots depict the drug synergy of AZA + FLU + ERL (orange) and AZA + FLU + LAP (cyan). Circles on the transparent triangular plane indicate the expected IC₃₀ for each two-drug combination, and the expected IC₅₀ for triple-drug treatment is located at the center of this triangle. Gray dots are the observed IC₅₀s for single- and double- drug treatments. Red dots are the observed IC₅₀s for the triple-drug treatments. Concave or convex colored planes indicate synergistic or antagonistic drug interaction, respectively. FIC₃ is the fractional inhibitory concentration. Views from two angles are displayed (left and right panels).
   (D) Viability, determined by MTT assay (left panels), of OVCAR8-ADR cells treated with RIB-, TRA- and/or FLU. Data shown are mean ± SD from biological replicates (n = 3). Statistical significance was analysed by one-way ANOVA with Dunnett's post hoc test, * indicates P < 0.05. Surface plots (right panels) depict the drug synergy of RIB + TRA + FLU, as presented in (C).
**Figure 6****. Strategy for assembling the barcoded gRNA library pools**
   (A) gRNA expression cassettes with different promoters and scaffold variants generate efficient knockout of the target *GFP* gene in OVCAR8-ADR-Cas9 cells. GFPsg1 was used. Flow cytometry was performed to measure the percentage of cell populations positive for GFP fluorescence. Data are mean ± SD, n = 3 biological replicates. Statistical significance was analysed by one-way ANOVA with Tukey's post hoc test. Modifications in the gRNA scaffolds v1 and v2 are shown.
   (B) Library assembly procedures. Oligo pairs (Oligo-F and Oligo-R, respectively) were synthesized and annealed to create double-stranded inserts with 20 bp gRNA target sequences, two BbsI sites, 8 bp barcodes, and 5' overhangs at their ends. The inserts were mixed in equal molar ratio and cloned into three BbsI- and Mfe- digested storage vectors, which contain the hH1, hU6, or mU6 promoters, with one-pot ligation reactions via their compatible ends to create the pooled storage vector libraries. The 3' end of the promoter sequences of hH1 and mU6 were modified such that the same pool of gRNA oligos can be used for building the libraries. Subsequent single-pot ligation reactions were performed with the BbsI-digested pooled storage vector libraries and an insert containing the gRNA scaffold sequence (WT, v1, or v2), BamHI and EcoRI sites, and 5' overhangs at their ends to assemble the barcoded gRNA library pools.

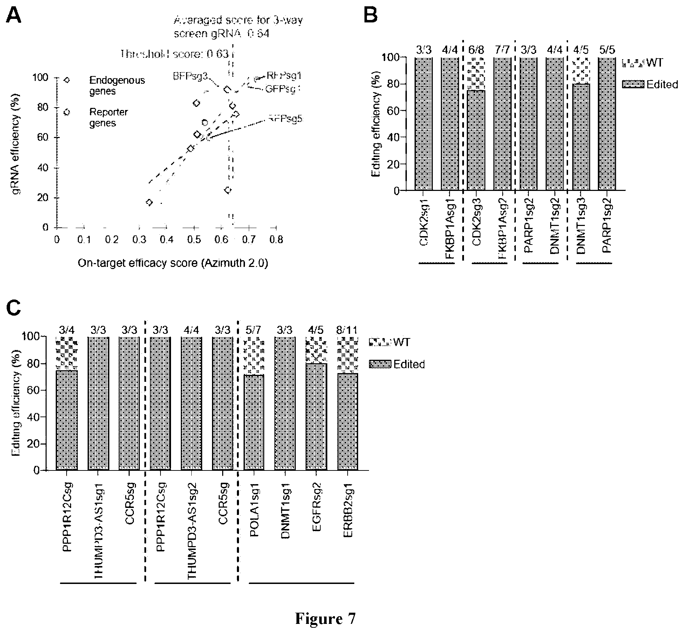

**Figure 7****. Activity of gRNAs at targeted genes in OVCAR8-ADR-Cas9 cells**
   (A) The efficiency of each gRNA was plotted against its on-target efficacy score predicted by Azimuth 2.0. Efficiency of gRNAs targeting the fluorescent reporter genes were determined using flow cytometry, and data for endogenous genes were obtained from our published data (Wong et al., 2016a).
   (B-C) Sequencing of targeted allele for cells infected with gRNA expression construct. OVCAR8-ADR-Cas9 cells were infected with lentiviruses encoding the indicated pairwise (B) and three-way combinatorial (C) gRNA expression constructs. The targeted alleles were amplified from the genomic DNA by PCR and cloned into storage vector for Sanger sequencing. The editing efficiency at each targeted locus and the number of individual clones being sequenced are shown.

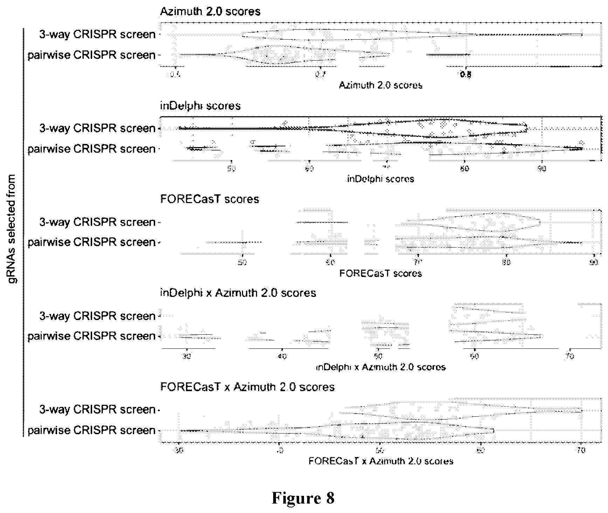

**Figure 8****. Prediction of on-target and frameshift scores for gRNAs used in the screening libraries**
   The gRNAs selected for the three-way combinatorial CRISPR screen showed a higher average on-target score and a higher average frameshift scores predicted by inDelphi and FORECasT than those used in the pairwise CRISPR screen. The combined effect brought by both the on-target and frameshift predictions was also evaluated.

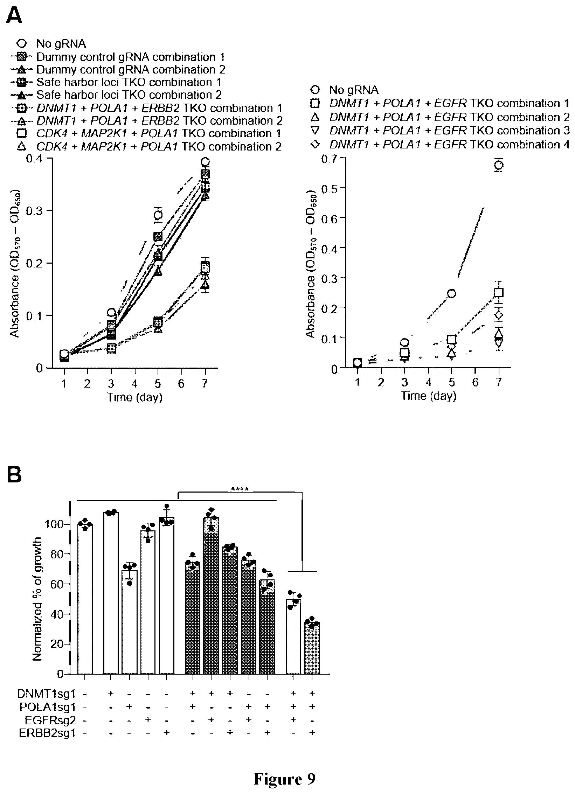

**Figure 9****. Growth inhibition induced by simultaneous targeting *DNMT1* + *POLA1* + *EGFR*/*ERBB2* and *CDK4* + *MAP2K1* + *POLA1* genes, but not safe harbour loci**
   (A-B) OVCAR8-ADR-Cas9 cells were infected with triple-gRNA combinations that target the indicated genes (A), and the respective single and/or double-gRNA combinations (B). The three safe harbour loci being targeted were *PPP1R12C*, *THUMPD3-AS1*, and *CCR5.* The gRNAs used in each combination are listed in Table S5. Cell viability was determined by MTT assay. Data are mean ± SD from biological replicates (n = 4).

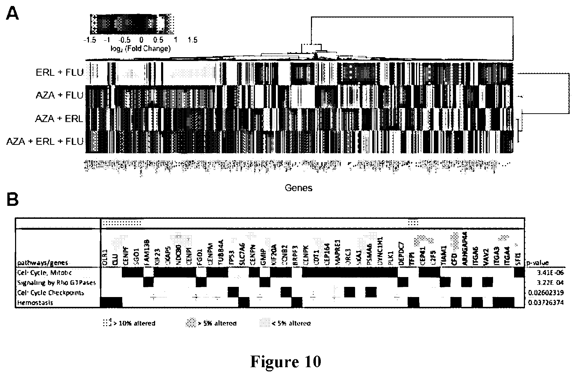

**Figure 10****. RNA-seq analysis of OVCAR8-ADR cells treated with drug combinations**
   (A) Heatmaps showing the log₂ fold changes of the expression of 3,834 genes that were identified with an FDR < 0.05 as differentially expressed in OVCAR8-ADR cells treated with the three-drug regimen (AZA + FLU + ERL), when compared to the untreated control. The log₂ fold changes of the expression of those genes after treating the cells with the corresponding drug pairs are included. Hierarchical clustering of genes and samples was performed based on the Pearson's correlation.
   (B) Matrix showing the mapped pathways for the differentially expressed genes, which were at least 20% up- (orange) or down- (blue) regulated in OVCAR8-ADR cells treated with the three-drug regimen (AZA + FLU + ERL) when compared to the untreated control and also had a fold change of > 10%, > 5%, or < 5% in cells treated with the three-drug regimen over all of those treated with the corresponding two-drug regimens. The bar on top of the x-axis indicates the level of fold change detected by different shades of green. Each darken spot indicates that a gene exists in a particular pathway.

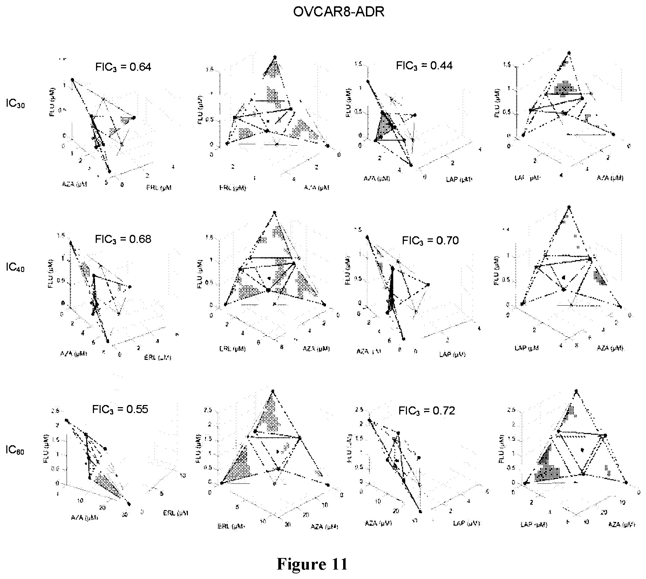

**Figure 11****. Synergistic drug interactions of AZA-FLU-ERL/LAP in OVCAR-ADR cells assessed at different inhibitory concentrations**
   Surface plots depicting the drug interactions of AZA + FLU + ERL (orange) and AZA + FLU + LAP (cyan) and the fractional inhibitory concentration (FIC₃) scores are shown. Panel of IC₅₀ is shown in Figure 5C.

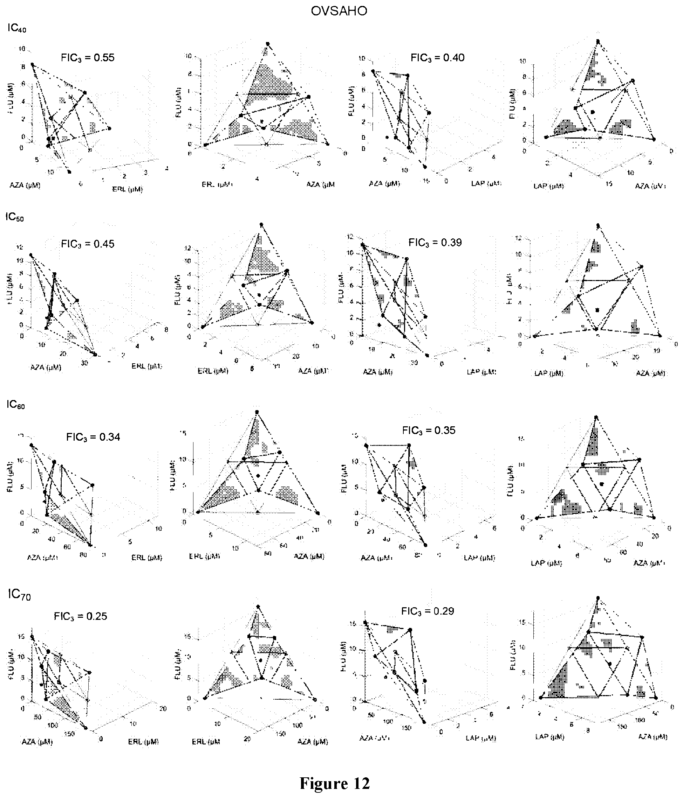

**Figure 12****. Synergistic drug interactions of AZA-FLU-ERL/LAP in OVSAHO cells assessed at different inhibitory concentrations**
   Surface plots depicting the drug interactions of AZA + FLU + ERL (orange) and AZA + FLU + LAP (cyan) and the fractional inhibitory concentration (FIC₃) scores are shown.

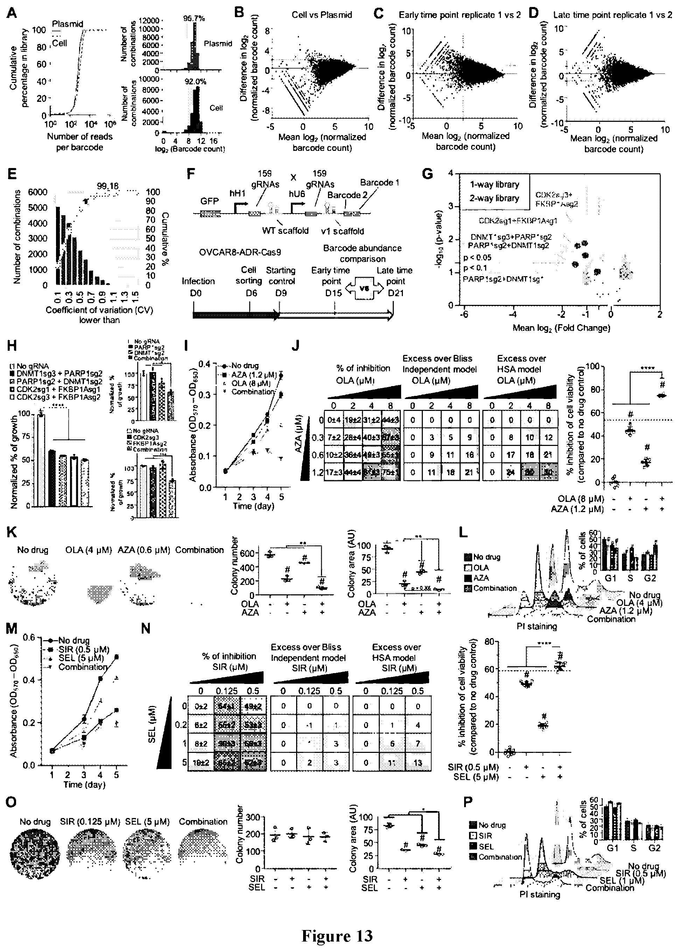

**Figure 13****. A CRISPR-based dual-gene knockout screen identifies drug pairs that inhibit ovarian cancer cell growth**
   (A) Distributions of barcode reads in the plasmid and 9-day post-infected OVCAR8-ADR-Cas9 cell pools. 99.7% (25,201 out of 25,281) and 99.0% (25,027 out of 25,281) of all expected gRNA combinations was obtained in the plasmid and cell pools, respectively. Most barcoded gRNA combinations were detected within a 5-fold range from the mean barcode reads per combination (highlighted by the shaded areas).
   (B) High correlation between barcode representations (normalized barcode counts) within the plasmid pool and infected cell pool indicates efficient lentiviral delivery of the pairwise gRNA library into cells. The horizontal dotted lines in the Bland-Altman plots indicate the 95% limits of agreement.
   (C-D) High reproducibility for barcode representations between two biological replicates in cells cultured for 15-day (C), and 21-day (D) post-infection with the pairwise gRNA library. The horizontal dotted lines in the Bland-Altman plots indicate the 95% limits of agreement. The vertical dash line indicates the threshold of 100 raw barcode counts.
   (E) The coefficient of variation (CV; defined as SD/mean of the fold changes of normalized barcode counts for 21-day versus 15-day cultured cells) was determined for the two biological replicates. Over 99.2% of pairwise gRNA combinations had a CV of < 1 in the screen.
   (F) OVCAR8-ADR-Cas9 cells infected with the barcoded pairwise gRNA library were cultured for 15 and 21 days. Barcode representations within the cell pools were quantified using Illumina HiSeq. The barcoded library vector uses hH1-gRNA-WT scaffold and hU6-gRNA-v1 scaffold in the first and second expression cassettes, respectively.
   (G) A volcano plot for comparing the abundance changes of each barcoded gRNA combinations at day 21 versus day 15 post-infection. Hit combinations, *DNMT1* + *PARP1* and *FKBP1A* + *CDK2*, are highlighted in blue and red, respectively. Data were collected from two biological replicates.
   (H-J, M-N) Cell viability, determined by MTT assay, of the indicated gRNA-infected OVCAR8-ADR-Cas9 cells (H) and drug-treated OVCAR8-ADR cells (I, J, M, N). In (J and N), percentage of growth inhibition was calculated by comparing each drug-treated group to the untreated control. Drug synergy was measured by the Bliss Independent model and the HSA model. The growth-inhibitory effects brought by treatment with 8 µM of OLA and 1.2 µM of AZA (J), and 5 µM SEL and 0.5 µM SIR (N), were plotted as examples to illustrate, and the dash lines indicate the expected drug combination effects based on the Bliss Independent model. Data shown are mean ± SD from biological replicates (n = 3 in H; n = 6 in I and J; n = 8 in M and N).
   (K, O) Colony formation assay of OLA- and/or AZA- (K), and SEL- and/or SIR- (O), treated OVCAR8-ADR cells. The colony numbers and areas were quantified. Data shown are mean ± SD from biological replicates (n = 3).
   (L, P) Cell cycle analysis of OLA- and/or AZA- (K), and SEL- and/or SIR- (O), treated OVCAR8-ADR cells. The percentage of cells in each cell cycle phase was quantified. Data shown are mean ± SD from biological replicates (n = 3).
   Statistical significance in H-P was analysed by one-way ANOVA with Tukey's post hoc test. *P < 0.05, **P < 0.01, and ****P < 0.0001; ^{#}P < 0.05 in J-L and N-O indicates the comparisons with the untreated control.

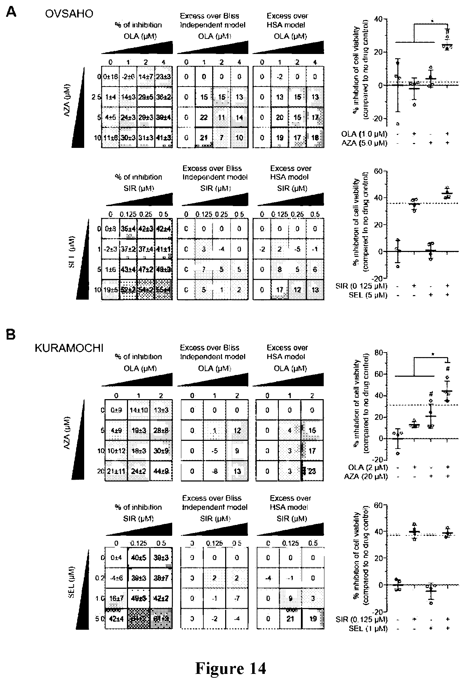

**Figure 14****. Growth-inhibitory effects brought by AZA and OLA, and SEL and SIR, on OVSAHO and KURAMOCHI cells**
   (A-B) Growth inhibition brought by the indicated drug treatments to OVSAHO (A) and KURAMOCHI (B) cells were measured by MTT assay, and was calculated by comparing each drug treatment to the untreated control. Drug synergy was measured by Bliss Independent model and HSA model. The dash line in the plots in the right panels indicates the expected drug combination effects based on the Bliss Independent model. Data are mean ± SD from biological replicates (n = 4). Statistical significance was analysed by one-way ANOVA with Tukey's post hoc test. *P < 0.05; ^{#}P < 0.05 indicates the comparisons with the untreated control.

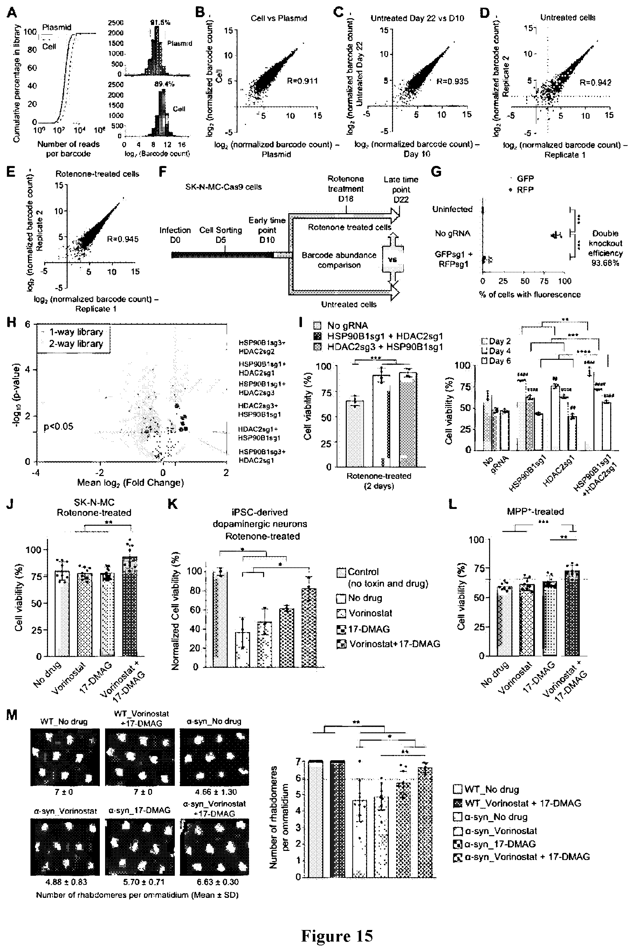

**Figure 15****. A CRISPR-based dual-gene knockout screen identifies a drug combination that enhances protection against Parkinson's disease toxicity**
   (A) Distributions of barcode reads in the plasmid and infected SK-N-MC-Cas9 cell pools. 99.1% (7,499 out of 7,569) and 98.7% (7,467 out of 7,569) of all expected gRNA combinations was obtained in the plasmid and cell pools, respectively. Most barcoded gRNA combinations were detected within a 5-fold range from the mean barcode reads per combination (highlighted by the shaded areas).
   (B) High correlation between barcode representations (normalized barcode counts) within the plasmid pool and infected cell pool indicates efficient lentiviral delivery of the pairwise gRNA library into cells.
   (C) High correlation between barcode representations within cell pools cultured for 10 and 22 days indicates the CRISPR-mediated gene knockouts did not result in severe cell death.
   (D-E) High reproducibility for barcode representations between two biological replicates in untreated (D) and rotenone-treated (E) cells. The dash lines in (D) indicate the threshold of 20 raw barcode counts. R is the Pearson correlation coefficient.
   (F) SK-N-MC-Cas9 cells infected with the barcoded pairwise gRNA library were either treated with rotenone or remained untreated. Barcode representations within the cell pools were quantified using Illumina HiSeq.
   (G) Lentiviral delivery of dual-gRNA expression constructs efficiently disrupt multiple target genes in SK-N-MC-Cas9 cells. Flow cytometry was used to measure the percentage of cell populations positive for GFP and RFP fluorescence at day 6 post-infection. Data are mean ± SD, n = 3 biological replicates.
   (H) A volcano plot for comparing the abundance changes of each barcoded gRNA combinations in rotenone-treated versus untreated cell pools. Hit combination, *HSP90B1* + *HDAC2,* is highlighted in red. Data were collected from two biological replicates.
   (I) Cell viability, determined by MTT assay, of the indicated sgRNA-infected SK-N-MC-Cas9 cells in the presence of rotenone. Data shown are mean ± SD (n = 4) from biological replicates, and data of left and right panels were obtained from the same experiments.
   (J-K) Cell viability of the indicated drug-treated SK-N-MC cells (J) and iPSC-derived dopaminergic neurons (K) in the presence of rotenone, determined by MTT assay and DAPI uptake assay, respectively. Data shown in are mean ± SD (n = 9 in (J); n = 3 in (K)) from biological replicates.
   (L) Cell viability, determined by MTT assay, of the indicated drug-treated SK-N-MC cells in the presence of MPP⁺. Data shown are mean ± SD (n = 8) from biological replicates.
   (M) Quantification of the number of rhabdomeres per ommatidium in wild-type and alpha-synuclein-expressing flies that were fed with the indicated drug(s). Combination of 17-DMAG and vorinostat restored the number of rhabdomeres per ommatidium in alpha-synuclein-expressing *Drosophila* eyes. Representative images showing the rhabdomeres of wild-type and alpha-synuclein-expressing flies that were fed with the indicated drug(s). Data are mean ± SD from at least 100 ommatidia of 5-10 flies.
   Statistical significance in (I-M) was analysed by one-way ANOVA with Tukey's post hoc test. *P < 0.05, **P < 0.01 and ****P < 0.0001 represent significant differences between the indicated samples. In (I), ^{#}P < 0.05, ^{##}P < 0.01, ^{###}P < 0.001, and ^{####}P < 0.0001 indicates the comparisons with the respective no gRNA controls. Dash line indicates the expected drug combination effects based on the Bliss Independent model.

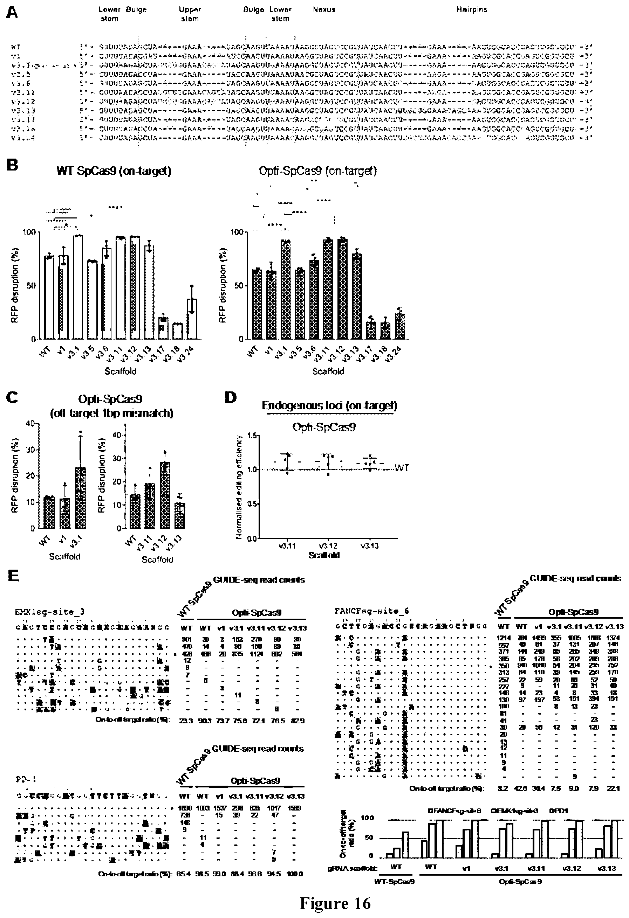

**Figure 16****. Engineered gRNA scaffold variants exhibit improved on-target and low off-target activities.**
   (A) Sequence of gRNA scaffold sequences used.
   (B-C) OVCAR8-ADR cells harboring reporter constructs with on-target (B) and off-target (C) sites were infected with lentiviruses encoding wildtype or Opti-SpCas9. The editing efficiency of the gRNA scaffold variants was measured as the percentage of cells with depleted RFP fluorescence.
   (D) Assessment of gRNA scaffold variants for efficient on-target editing with gRNAs targeting endogenous loci. The percentage of sites with indels was measured using a T7 endonuclease I (T7E1) assay. The ratio of the on-target activity of gRNA scaffold variants to the activity of scaffold was determined, and the median and interquartile range for the normalized percentage of indel formation are shown for the 5 loci tested. Each locus was measured three times.
   (E) GUIDE-seq genome-wide specificity profiles for the panel of gRNA scaffold variants paired with the indicated gRNAs. Mismatched positions in off-target sites are colored, and GUIDE-seq read counts were used as a measure of the cleavage efficiency at a given site.

### 5. DETAILED DESCRIPTION

Combinatorial drug therapy targeting multiple pathways can limit the development of drug resistant phenotype in cancer cells since it is harder for the cells to activate multiple compensatory survival mechanisms.

This disclosure relates to systems and compositions that enables highly efficient, multiplexed genome editing and CRISPR screening. The engineered guide RNA scaffolds and promoters developed in this work enable three-way combinatorial CRISPR screens to be carried out based on a prior assembly method called CombiGEM-CRISPR, and can be used to improve genome editing efficiency in routine experiments and applications. High-throughput CRISPR screens from prior arts have only been able to study paired interactions between guide RNAs, and not those of greater complexity (such as interactions between three or more guide RNAs). The systematic screening of complex genetic interactions is enabled by the generation of engineered scaffolds and promoter sequences that can minimize possible lentiviral vector recombination due to long homologous sequences and permit use of the same pool of guide RNA oligos for building high-quality combinatorial guide RNA libraries.

Technically, the guide RNA scaffold sequences are engineered to minimize possible lentiviral vector recombination due to long homologous sequences, and the 3' end of the promoter sequences of hH1 and mU6 are modified such that the same pool of gRNA oligos can be used for building the combinatorial guide RNA libraries. The engineered guide RNA scaffold and modified promoter sequences show higher or comparable activity for driving guide RNA expression when compared to their wild-type counterparts.

### 5.1 Design of CombiGEM-CRISPR v2.0

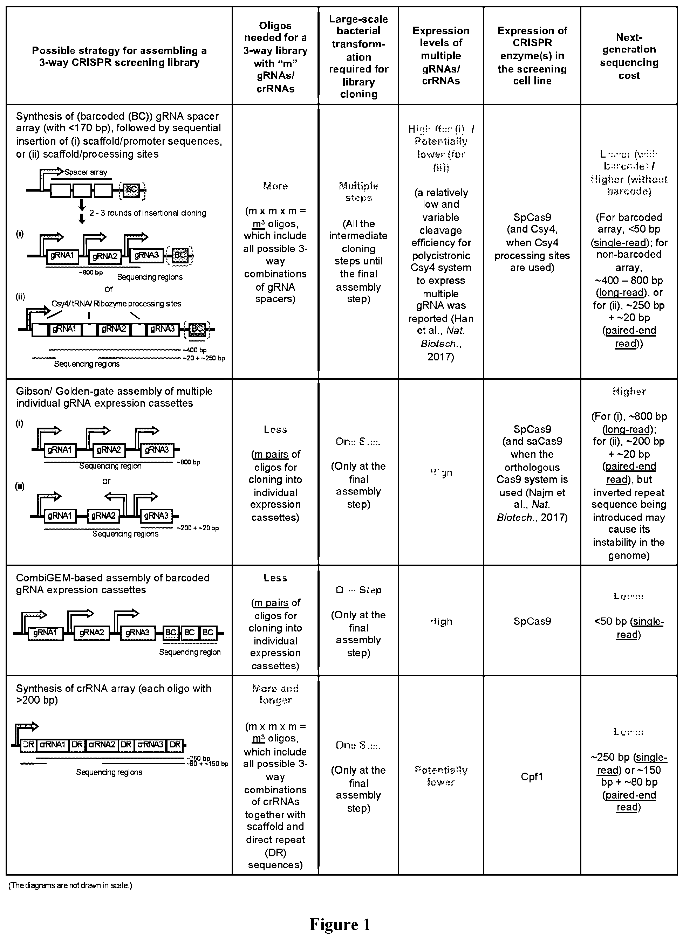

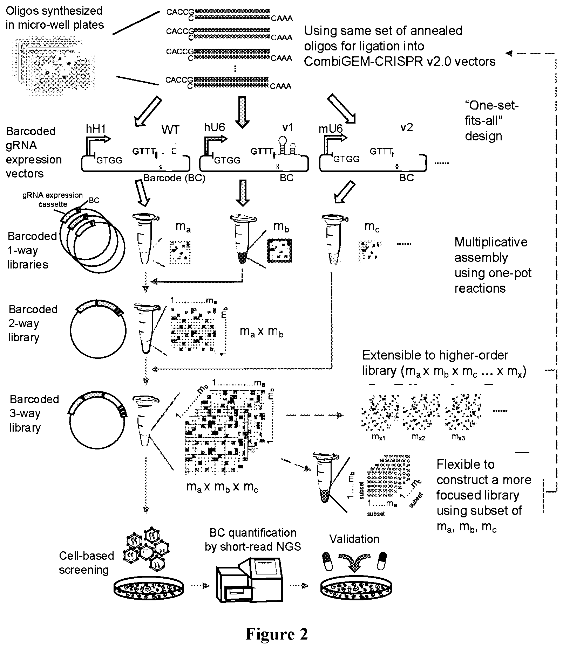

CombiGEM-CRISPR v2.0 toolkits include add-on designs on library vectors that enable only a single, reusable set of oligos to be synthesized for performing high-order combinatorial CRISPR screens. We and others have shown that CRISPR screens can be carried out via targeting two genes simultaneously using dual guide RNA (gRNA) expression cassettes (Chow et al., 2019; Du et al., 2017; Han et al., 2017; Najm et al., 2018; Shen et al., 2017; Wong et al., 2016a). Here we evaluated the extensibility of existing methods and other possible toolkits for assembling a three-way combinatorial gRNA library for screening (Figure 1). Considering using an oligo synthesis-based approach to build a library with mₐ x m_{b} x m_{c} combinations, the same number of gRNA protospacers-containing oligos (i.e., mₐ x m_{b} x m_{c}) has to be first designed and synthesized to include all combinations and followed by sequential insertions of the promoter and scaffold sequences. The drawbacks are that (1) all the intermediate cloning steps require large-scale bacterial transformations for maintaining a high library coverage, which is technically more demanding than CombiGEM-CRISPR v2.0 method that only requires a large-scale transformation at the final assembly step, and (2) the rigid cloning framework does not permit post-assembly insertion of additional gRNAs for building higher-order libraries. The inflexible workflow also limits the reusability of oligos for building a more compact library for secondary screens. For example, if a pairwise gRNA library was constructed for identifying the core effectors to be included in a three-way gRNA library, building of the higher-order library requires new set of oligos to be synthesized for a complete re-assembly of libraries. Alternative library assembly strategies are through Gibson- (Gibson et al., 2009) or Golden-gate- (Engler et al., 2008) based methods, which require designing overlapping regions or complementary overhang of adjacent gRNA expression units for fusing multiple parts together. However, these methods also do not allow additional parts to be introduced to pre-existing barcoded libraries. Our CombiGEM-CRISPR v2.0 strategy provides a truly scalable solution that enables multiplicative assembly of additional barcoded gRNA expression units for extending from n to n+1 combinatorial CRISPR libraries (Figure 2). Libraries can be also tailored and re-assembled from selected gRNAs by choosing from the suite of gRNAs stored in vectors for another screen or a secondary screen for higher resolution. In addition, the creation of barcodes that are unique for each assembled combination allows their rapid characterization to be performed via short-sequencing reads, which reduces the sequencing cost and potential errors generated via long reads. We therefore decided to take this approach to build three-way combinatorial gRNA libraries for screening applications.

Since previous CombiGEM toolkits were not directly adaptable for assembling three-way and even higher-order combinatorial gRNA libraries, here we further created a "one-set-fits-all" design such that building a n-way combinatorial CRISPR screening library of m guide RNAs using multiple gRNA expression cassettes always requires only m (instead of n x m) pairs of oligos to be synthesized. To ensure expression of three gRNAs in single cells, we assembled together the multiple gRNA expression cassettes in a single vector. Multiple promoters (including human U6, mouse U6, and human H1) (Adamson et al., 2016; Ma et al., 2014; Vidigal and Ventura, 2015) and modified gRNA scaffolds (Adamson et al., 2016; Briner et al., 2014; Dang et al., 2015; Grevet et al., 2018) were used in the expression cassettes (Figure 6), which minimizes possible lentiviral vector recombination due to long homologous sequences. However, the use of multiple promoters requires multiple sets of oligos, and/or additional PCR and restriction enzyme digestion reactions, for building the different expression cassettes for library assembly. To allow researchers synthesizing and annealing only a single set of oligos as parts to build all possible higher-order combinations of gRNAs for multiplexed CRISPR screens, we performed sequence adaptation by modifying the 3' end sequence of promoters to those that are complementary to the sticky ends of the annealed oligos, and these promoters expressed gRNAs and generated efficient gene knockouts (Figure 6). These new standardized vector parts are useful for the flexible assembly of high-order combinatorial gRNA libraries and extensible combinatorial CRISPR screens.

In some embodiments, the promoters have sequences as shown below.
>Wild type Human U6 promoter: 
AGAGGGCCTATTTCCCATGATTCCTTCATATTTGCATATACGATACAAGGCTGTTAG
AGAGATAATTAGAATTAATTTGACTGTAAACACAAAGATATTAGTACAAAATACGT
GACGTAGAAAGTAATAATTTCTTGGGTAGTTTGCAGTTTTAAAATTATGTTTTAAAA
TGGACTATCATATGCTTACCGTAACTTGAAAGTATTTCGATTTCTTGGCTTTATATAT
CTTGTGGAAAGGACGAAACACCG
>Modified Mouse U6 promoter: 
GATCCGACGCCGCCATCTCTAGGCCCGCGCCGGCCCCCTCGCACAGACTTGTGGGAG
AAGCTCGGCTACTCCCCTGCCCCGGTTAATTTGCATATAATATTTCCTAGTAACTATA
GAGGCTTAATGTGCGATAAAAGACAGATAATCTGTTCTTTTTAATACTAGCTACATT
TTACATGATAGGCTTGGATTTCTATAAGAGATACAAATACTAAATTATTATTTTAAA
AAACAGCACAAAAGGAAACTCACCCTAACTGTAAAGTAATTGTGTGTTTTGAGACT
ATAAATATCCCTTGGAGAAAAGCCTTCACCG
>Wild type Mouse U6 promoter (GeneBank: X06980.1) 
GATCCGACGCCGCCATCTCTAGGCCCGCGCCGGCCCCCTCGCACAGACTTGTGGGAG
AAGCTCGGCTACTCCCCTGCCCCGGTTAATTTGCATATAATATTTCCTAGTAACTATA
GAGGCTTAATGTGCGATAAAAGACAGATAATCTGTTCTTTTTAATACTAGCTACATT
TTACATGATAGGCTTGGATTTCTATAAGAGATACAAATACTAAATTATTATTTTAAA
AAACAGCACAAAAGGAAACTCACCCTAACTGTAAAGTAATTGTGTGTTTTGAGACT
ATAAATATCCCTTGGAGAAAAGCCTT
> Modified Human H1 promoter: 
TTCGAACGCTGACGTCATCAACCCGCTCCAAGGAATCGCGGCCCAGTGTCACTAGG
CGGGAACACCCAGCGCGTGCGCCCTGGCAGGAAGATGGCTGTGAGGGACAGGG
GAGTGGCGCCCTGCAATATTTGCATGTCGCTATGTGTTCTGGGAAATCACCATAAAC
GTGAAATGTCTTTGGATTTGGGAATCTTATAAGTTCTGTATGAGACCACTCTTCACC
G
>Wild type Human H1 promoter (GeneBank: X16612.1)

TTCGAACGCTGACGTCATCAACCCGCTCCAAGGAATCGCGGCCCAGTGTCACTAGGCGGAACA
CCCAGCGCGCGTGCGCCCTGGCAGGAAGATGGCTGTGAGGGACAGGGGAGTGGCGCCCTGCAATA
TTTGCATGTCGCTATGTGTTCTGGGAAATCACCATAAACGTGAAATGTCTTTGGATTTGGGAATC
TTATAAGTTCTGTATGAGACCACTCTT

### 5.2 Establishment of a CRISPR-Cas9-based multi-gene knockout system

We constructed a lentiviral combinatorial gRNA expression vector containing multiple gRNA expression cassettes to efficiently and simultaneously knock out three target genes (Figure 3), and evaluated its functionality in human cells (OVCAR8-ADR) using gRNAs targeting the exonic regions of green (GFP), red (RFP), and blue (BFP) fluorescent protein reporter genes. Lentiviruses carrying a BFP reporter and the combinatorial gRNA units were generated to infect both the OVCAR8-ADR and OVCAR8-ADR-Cas9 cells stably expressing RFP and GFP reporters. Flow cytometry analysis was performed to confirm the effective generation of double- and triple-knockout of the reporter proteins (> 81% and > 74%, respectively) (Figure 3). Similar knockout efficiencies were observed when wildtype (WT) or modified gRNA scaffolds were used. Our results indicate that the lentiviral vector can be used to deliver gRNAs to generate multi-gene knockouts, and this vector design was used for the combinatorial CRISPR-Cas9 screens in this study.

### 5.3 Three-way combinatorial CRISPR-Cas9 screen for synergistic anti-cancer genetic combinations

Combinatorial drug therapy targeting multiple pathways can limit the development of drug resistant phenotype in cancer cells since it is harder for the cells to activate multiple compensatory survival mechanisms (Bozic et al., 2013). We performed high-throughput studies to search for effective therapeutic combinations against high-grade serous ovarian cancer (HGSOC), the most prevalent subtype that contributes to two-third of all ovarian cancer deaths (Bowtell, 2010). With the CRISPR-based multi-gene knockout system described above, we applied CombiGEM-CRISPR v2.0 to assemble a high-coverage (99.8%) three-way combinatorial gRNA library (with 32 x 32 x 32 gRNAs = 32,768 total combinations) (Figure 4A-E). This library included 15 druggable protein-encoding genes (with 2 gRNAs per gene) that are commonly targeted in anti-cancer therapies and whose expressions were reported in OVCAR8-ADR, an established cell model of HGSOC (Baratta et al., 2015), and also other ovarian cancer cells based on the NCI-60 proteome database (Gholami et al., 2013) to demonstrate the feasibility of our approach (Table S2 and S3). To generate combinatorial gene knockouts at a high rate, we selected gRNAs with predicted high on-target (and low off-target) activities based on the Azimuth 2.0 model (Doench et al., 2016). We compared the on-target efficacy score and the indel generation efficiency of gRNAs, and observed that the on-target efficacy score largely predicts the efficiency of gRNAs in OVCAR8-ADR cells (Figure 7A). We chose gRNAs that have on-target scores > 0.64 based on the Azimuth 2.0 model, and most of them also have high frameshift generation rates analysed under the newly developed inDelphi and FORECasT models (Figure 8) (Allen et al., 2018; Shen et al., 2018). Two non-targeting control gRNAs from the GeCKOv2 library (Shalem et al., 2014) that do not have on-target loci in the human genome were also included in the library as references.

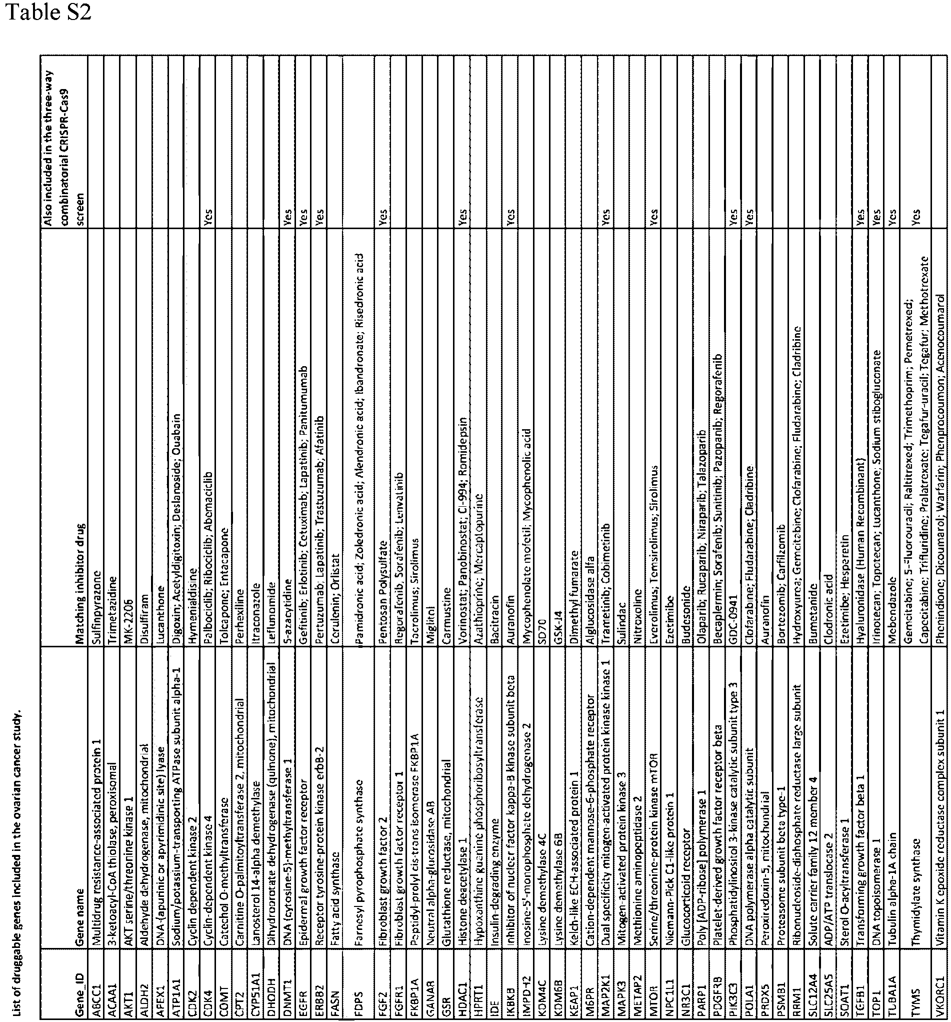

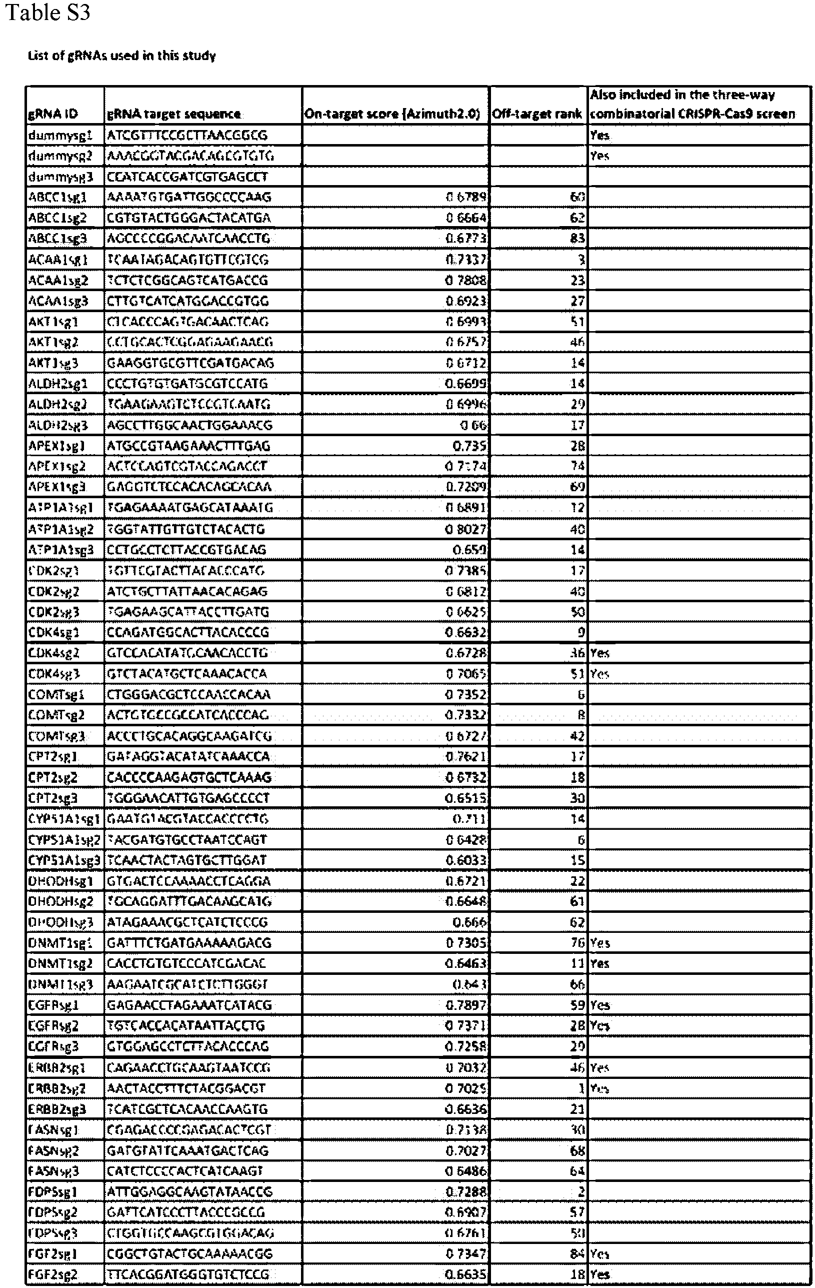

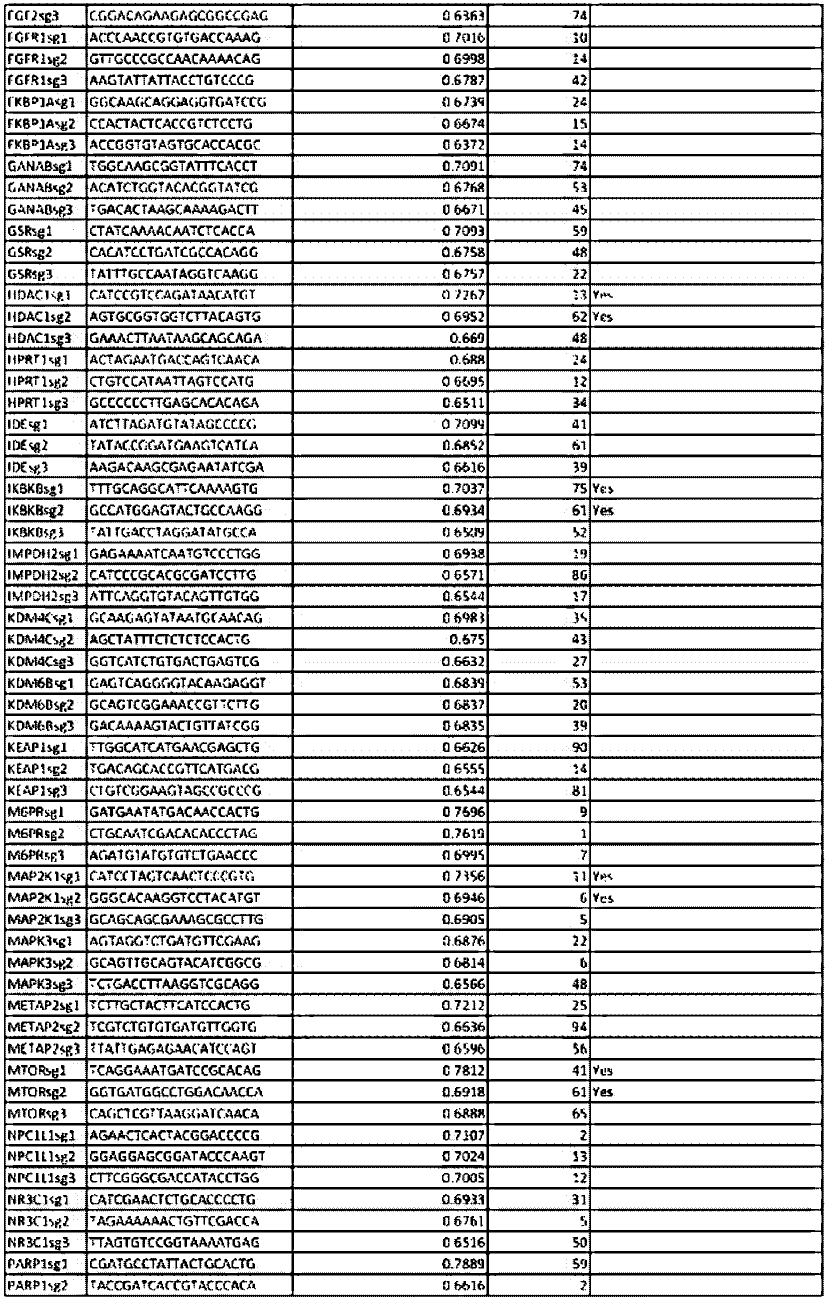

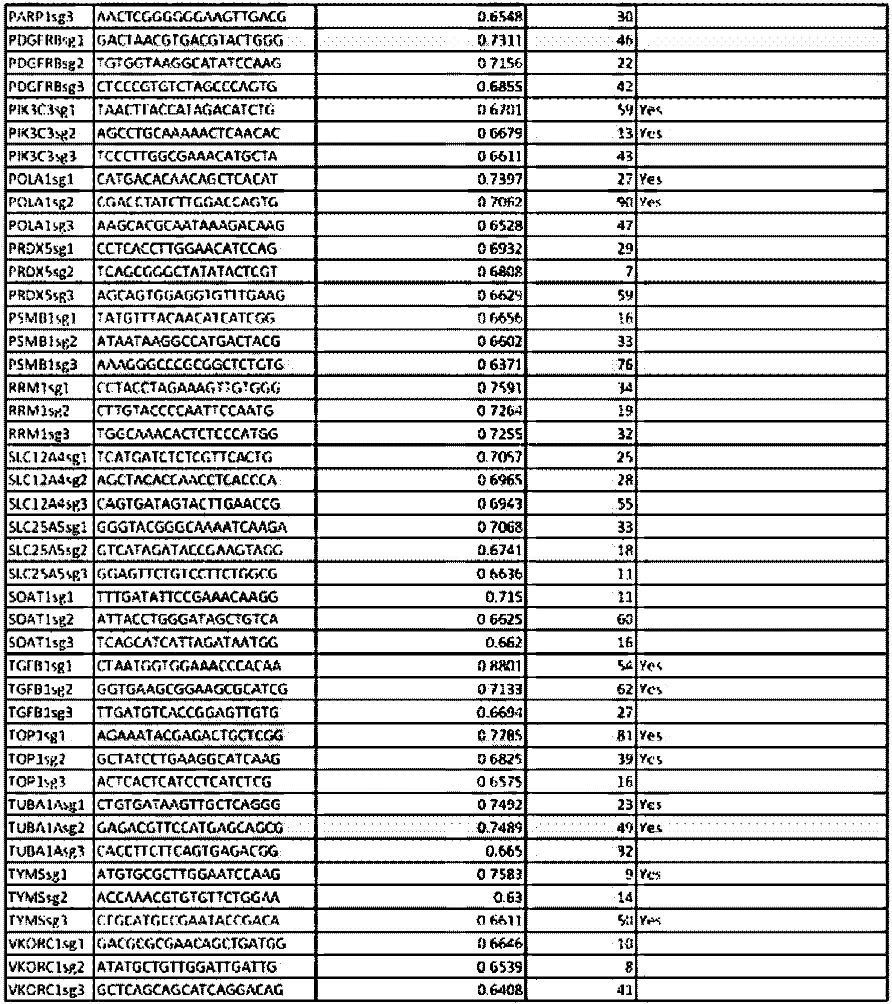

We then conducted a pooled screen to isolate three-way gRNA combinations that modulate OVCAR8-ADR growth (Figure 4F). Barcode abundances between day 15 and day 26 groups were compared to yield log₂ values as a measure of cell growth. Based on the genetic screen data, 3 (out of 455) three-way gene combinations had a mean log₂ ratios of < -1 (based on data obtained from two biological replicates with at least 50% fewer barcode counts in day 26- versus day 15- cultured cells) and a GI₃ score of < -0.2 (Figure 4G and Table S4). These three-way combinations (targeting *DNMT1 + POLA1 + EGFR* or *ERBB2,* as well as *CDK4 + MAP2K1 + POLA1*) also showed significantly different growth-modulatory effects from their respective singles and pairwise combinations (Figure 4H and Table S4). Because they showed strong growth inhibitory effects and strong synergistic genetic interactions, the three combinations were selected for further characterization. Their growth inhibition effects (reduced by 51.4 to 88.4% for the six tested gRNA combinations for *DNMT1 + POLA1 + EGFR* or *ERBB2*, and reduced by 50.5% and 54.9% for the two tested gRNA combinations for *CDK4 + MAP2K1+ POLA1*) were further confirmed by individual non-pooled assays and were not false positives that are caused by excessive DNA double stranded breaks generated by CRISPR-Cas9 (Aguirre et al., 2016; Munoz et al., 2016) because simultaneous targeting of three safe harbour loci did not result in strong growth arrest (Figures 4I, 7C and 9). In addition, the DNA copy number of DNMT1, POLA1, EGFR, ERBB2, CDK4, and MAP2K1 loci are not largely amplified in OVCAR8-ADR's genome based on the analysis using CellMiner (Reinhold et al., 2012).

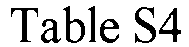

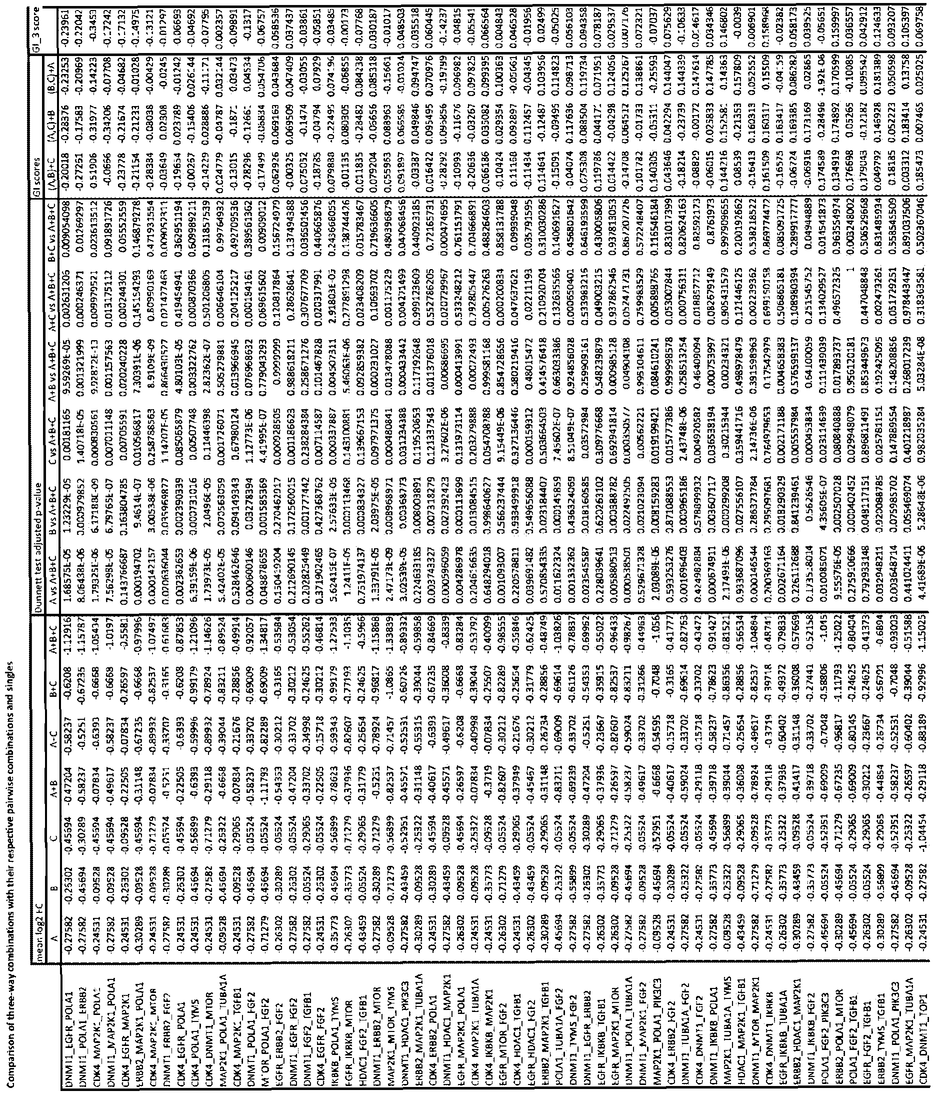

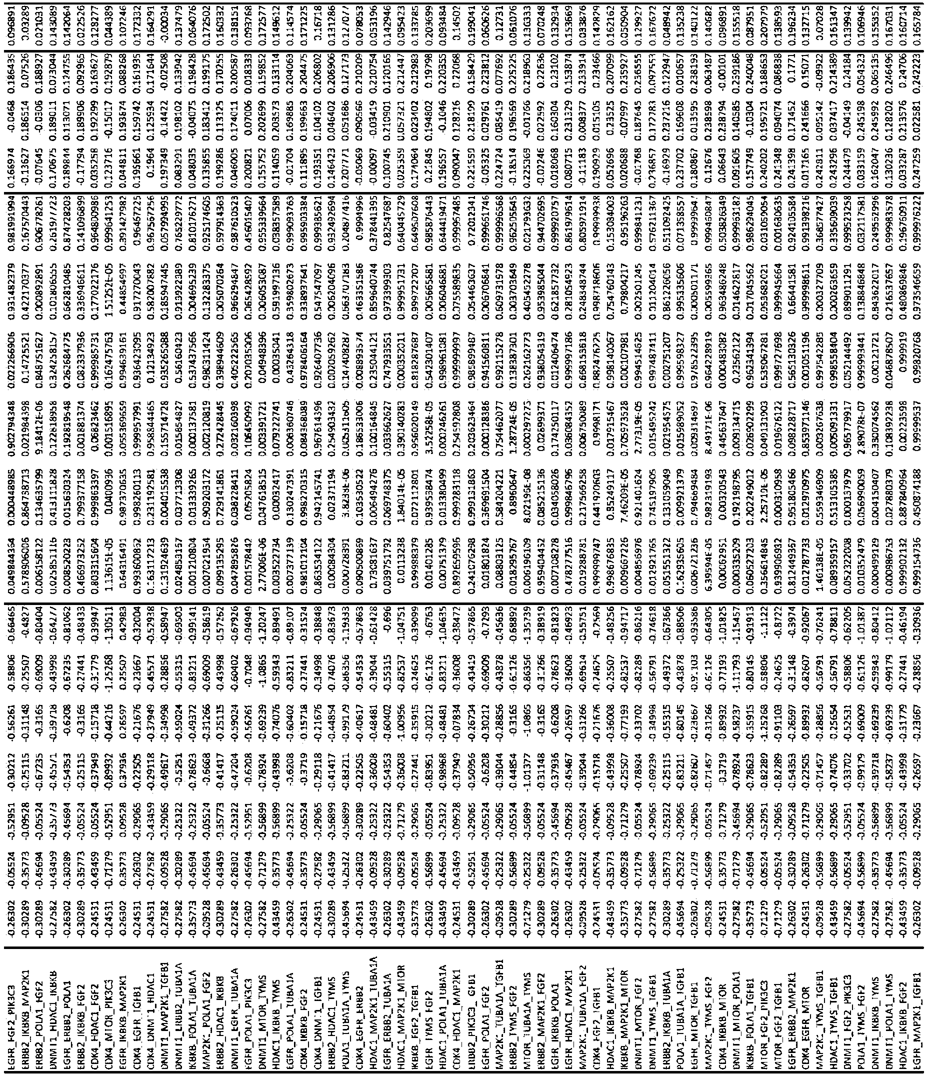

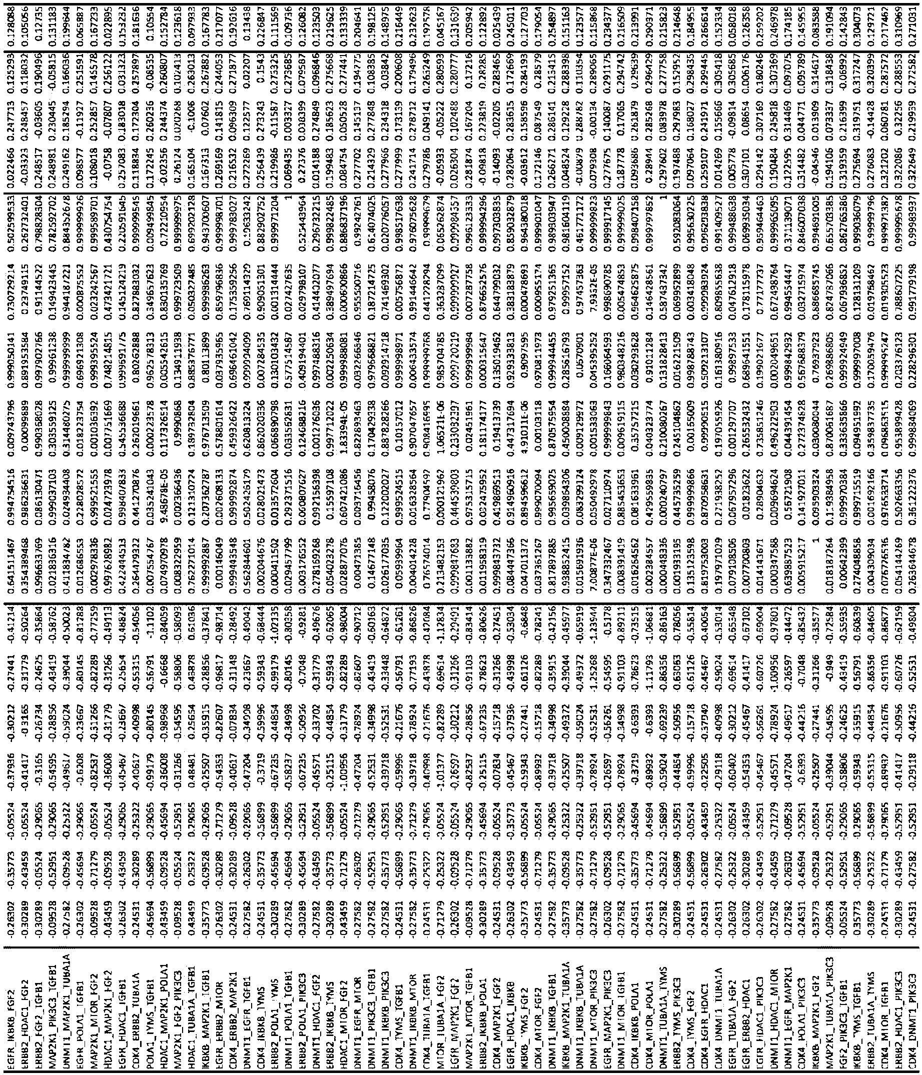

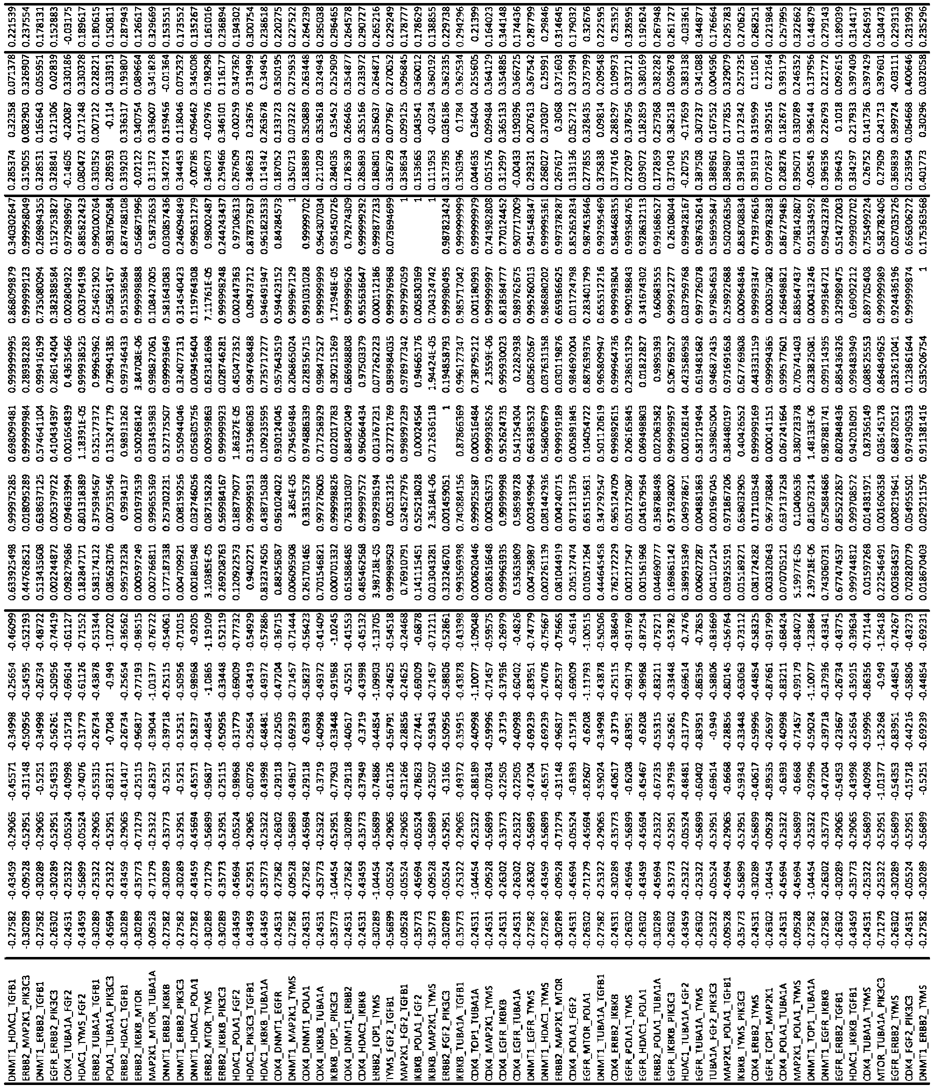

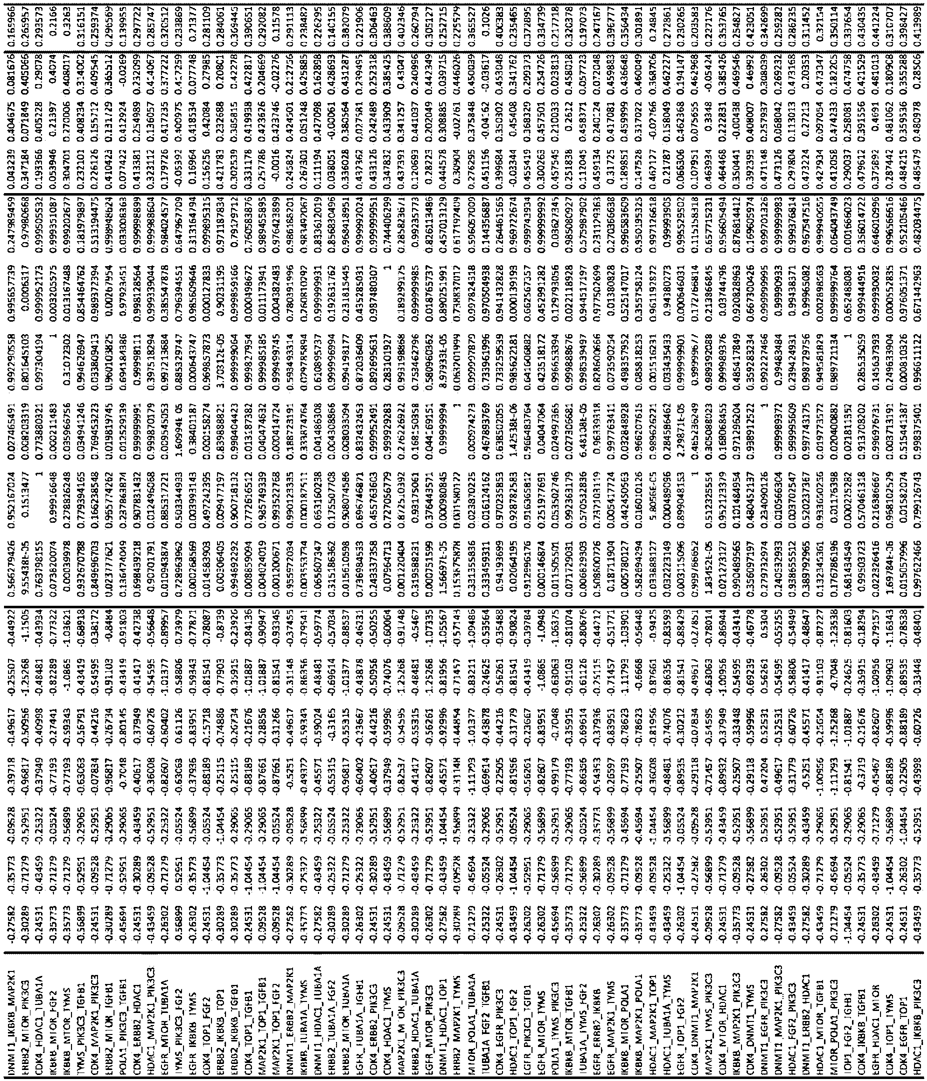

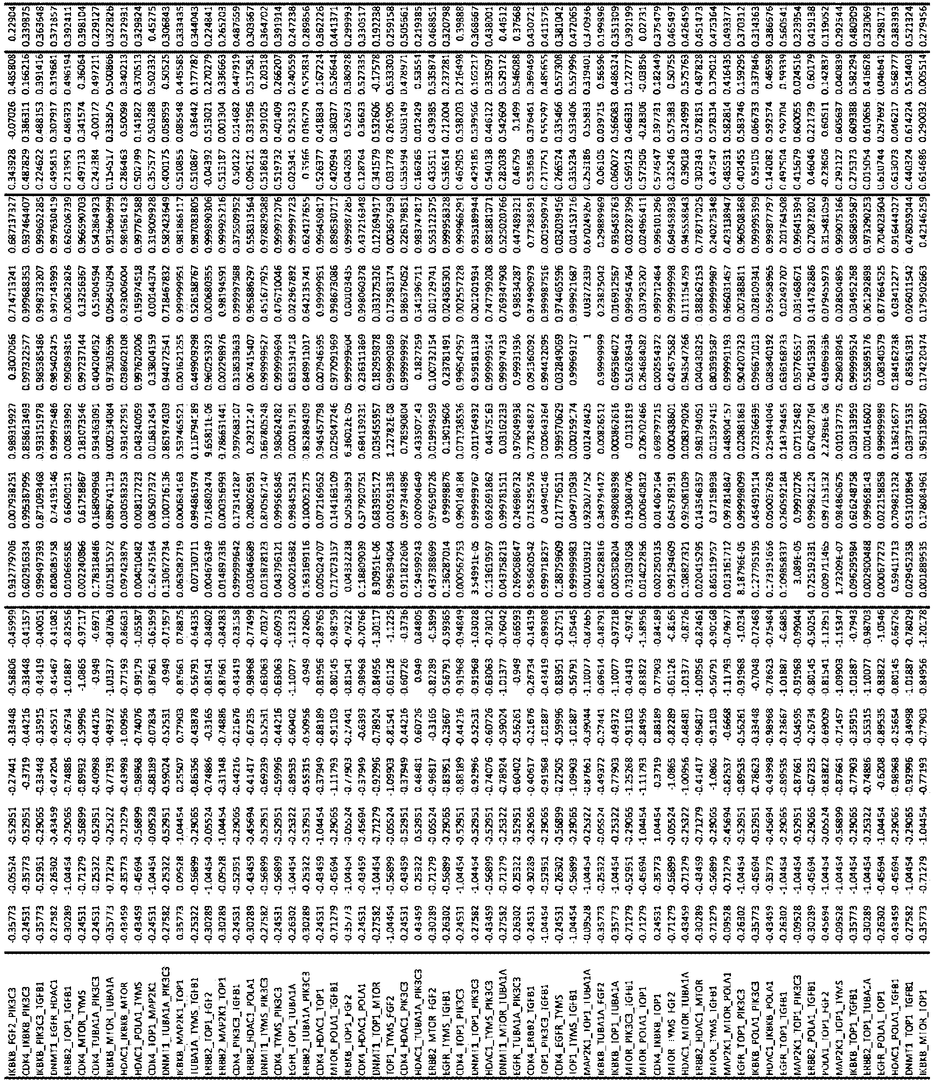

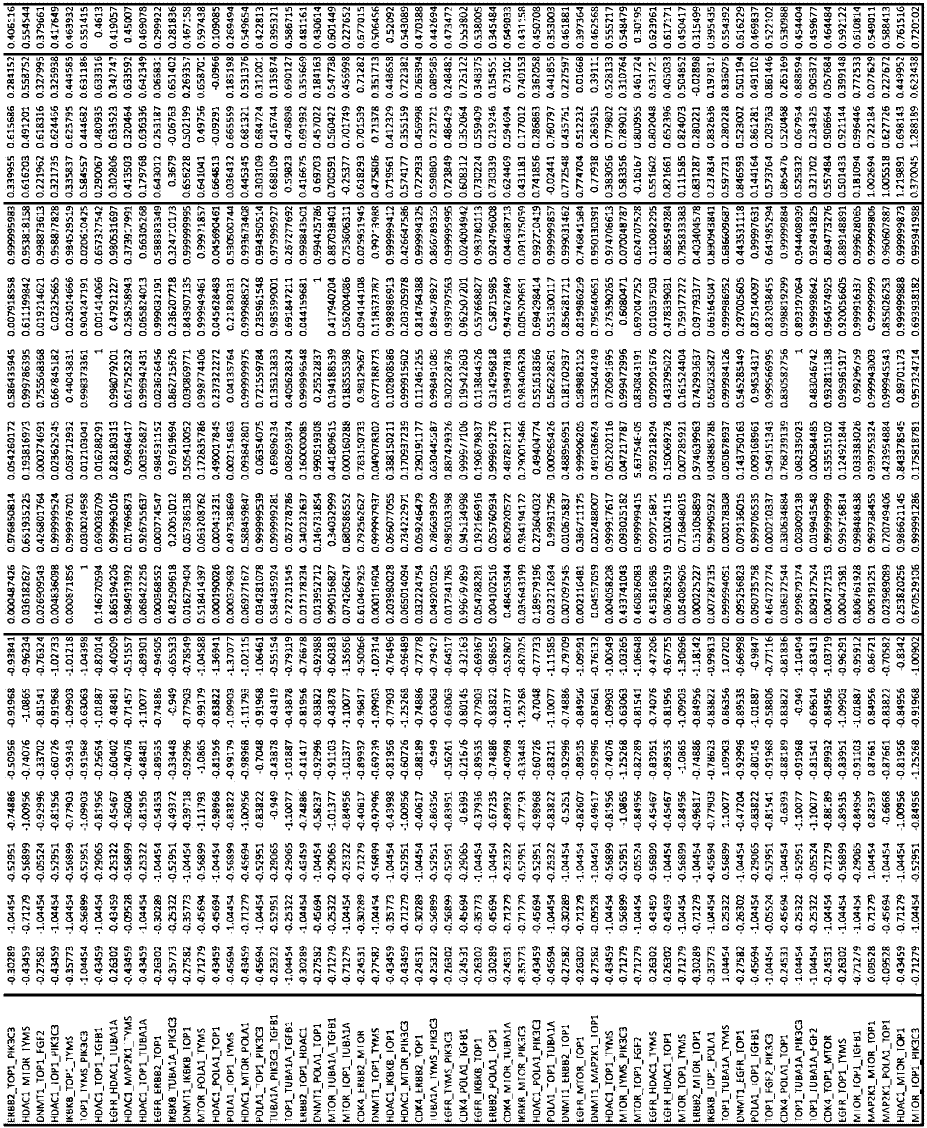

### 5.4 Validation of screen hits with matching drug combinations

Azacitidine (AZA), fludarabine (FLU), and erlotinib (ERL) were used to target DNMT1, POLA1, and EGFR, respectively. Lapatinib (LAP) was used to inhibit ERBB2, while it also acts on EGFR that belongs to the same Erb protein family. The three-drug treatment of AZA, FLU, and ERL/LAP showed significantly stronger growth-inhibitory effects than the single- and double-drug treatments (Figure 5A-B), and also resulted in a different set of perturbed genes including those involved in cell cycle regulation when comparing cells treated with the three-drug regimen to all of the respective two-drug combinations (Figure 10). Synergy among the three drugs suppressing ovarian cancer cell growth was confirmed using the DiaMOND scoring method (Figure 5C) (Cokol et al., 2017). The fractional inhibitory concentration (FIC) scores for AZA + FLU + ERL and AZA + FLU + LAP were 0.64 and 0.8, respectively, in OVCAR8-ADR cells. By comparing the drug dose required for achieving half maximal inhibiting concentration (IC₅₀) upon single-, double- and triple- drug treatments, we found that the triple-drug formulation of AZA + FLU + ERL demand ~4.5-fold less of each component (and ~3.7-fold less for AZA + FLU + LAP). Similar results were observed when measuring other inhibitory concentrations (IC₃₀, IC₄₀, and IC₆₀; Figure 11). The synergy among the three drugs (AZA + FLU + ERL or LAP) was also detected in OVSAHO cells (Figure 12). We also confirmed the synergy among the three matching drugs (Ribociclib (RIB) + Trametinib (TRA) + FLU) for our third screen hit (i.e., CDK4 + MAP2K1 + POLA1) in suppressing ovarian cancer cell growth (Figure 5D). These results indicate that our platform enables high-throughput screening and identification of synergistic three-way therapeutic combinations.

### 5.5 CRISPR-Cas9 screen for drug pairs against ovarian cancer

Via one-pot reactions using CombiGEM-CRISPR v2.0 (Figure 6B), we built a paired gRNA library targeting 52 druggable genes (3 gRNAs per gene; Tables S2 and S3), whose expressions were shown in OVCAR8-ADR, and also other ovarian cancer cells based on the NCI-60 proteome database (Gholami et al., 2013). We selected gRNAs with on-target scores scored > 0.63 (with predicted efficiency of > ~80%), except one with a score of 0.60. Three control gRNAs from the GeCKOv2 library (Shalem et al., 2014) that do not have on-target loci in the human genome were included as references. The pairwise gRNA library pool (with 159 x 159 gRNAs = 25,281 total combinations) was then delivered into OVCAR8-ADR cells via lentiviruses. Using our established experimental pipeline (Wong et al., 2015; Wong et al., 2016), we performed Illumina HiSeq to confirm the high coverage (> 99.0%) of the pairwise library and high correlation of barcode representation between the plasmid and infected cell pools (Figure 13A-E). Using similar time windows used in our previous study, barcode abundances between day 15 and day 21 groups were compared to yield log2 values as a measure of cell growth (Figure 13F). Based on a selection criteria that required a mean log2 ratios of < -1 (based on data obtained from two biological replicates with at least 50% fewer barcode counts in day 21- versus day 15-cultured cells) and multiple gRNAs targeting the same gene pair being detected with at least P < 0.1, two combinations (PARP1 + DNMT1 and CDK2 + FKBP1 A highlighted in blue and red, respectively, in Figure 13G) were defined as top screen hits. The growth inhibition brought by these two dual-gene knockouts were validated using individual non-pooled assays, and were not resulted from the knockout of either gene (Figure 13H; 7B).

We then evaluated the growth inhibition effects brought by these two hit combinations by treating OVCAR8-ADR cells with drug pairs. Olaparib (OLA), azacitdine (AZA), seliciclib (SEL), and sirolimus (SIR) were used as the drugs to target PARP1, DNMT1, CDK2, and FKBP1A, respectively. These drug molecules have been reported to have potent effects on their targets (McClue et al., 2002; Muvarak et al., 2016; Sabers et al., 1995; Wishart et al., 2006; Yang et al., 2017). Our results indicated that OLA and AZA act synergistically to suppress the growth of OVCAR8-ADR cells (Figures 13I-K) and induce G2 cell-cycle arrest (Figures 13L), while combined treatment with SEL and SIR exerted an additive effect that renders its growth (Figures 13M-P). Similar growth inhibition effects were observed when these drug combinations were treated to OVSAHO and KURAMOCHI (Figure 14), two other characterized cell models of HGSOC (Coscia et al., 2016; Domcke et al., 2013). Our results also corroborate with the observation that co-administration of PARP1 inhibitor talazoparib and DNMT1 inhibitor guadecitabine synergistically suppressed tumor growth in PEO1 and PEO4 cells, as well as in an OVCAR4 xenograft model (Pulliam et al., 2018), further suggesting PARP1 + DNMT1 inhibitor combination as an effective therapeutic option for ovarian cancer.

### 5.6 CRISPR-Cas9 screen for drug pairs against Parkinson's disease toxicity

Our screening approach can also be applied for searching effective therapeutic combinations that enhance protection against other disease phenotypes, such as Parkinson's disease (PD)-associated toxicity. We assembled another high-coverage (99.1%) pairwise gRNA library targeting 28 druggable genes, whose ablations or matching drug inhibitors were reported to suppress neuronal toxicity (Figure 15A-E; Table S9). The library was delivered into SK-N-MC-Cas9 cells via lentiviruses to generate dual gene knockouts, and the cells were then treated with rotenone to induce PD-associated toxicity (Figure 15F-G). Barcode abundances between the rotenone-treated and untreated groups were compared to identify enriched gRNA combinations that protect the cells from rotenone-induced toxicity. Based on a selection criteria that required a mean log2 fold-change of > 0.378 (based on data obtained from two biological replicates with at least 30% more barcode counts in rotenone-treated versus untreated cells) and multiple gRNAs targeting the same gene pair being detected with P < 0.05, our genetic screen identified HSP90B1 + HDAC2 as the top hit (with six gRNA combinations identified, having an average of 51.6% increase in barcode counts) that enhances cell survival upon rotenone treatment (Figure 15H; Table S8). The protective effect brought by the simultaneous knockout of HSP90B1 + HDAC2 was validated in non-pooled assays, which was greater than that from the knockout of either gene (Figure 15I). We further confirmed the protective effect of the matching drugs (17-(Dimethylaminoethylamino)-17-demethoxygeldanamycin (17-DMAG) + vorinostat) for this identified combination. Combined drug treatment enhanced cell survival against rotenone-induced toxicity when compared to single-drug treatments in both SK-N-MC cells (Figure 15J) and iPSC-derived dopaminergic neurons (Figure 15K). In addition, we observed this drug combination reduced toxicity induced by MPP+ treatment in cultured cells (Figure 15L) and alpha-synuclein expression in transgenic flies (Figure 15M), two other well-characterized models of PD. Our results demonstrate the versatility of our platform for screening therapeutic combinations against different disease phenotypes, including those for alleviating neurodegenerative disease phenotypes.

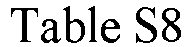

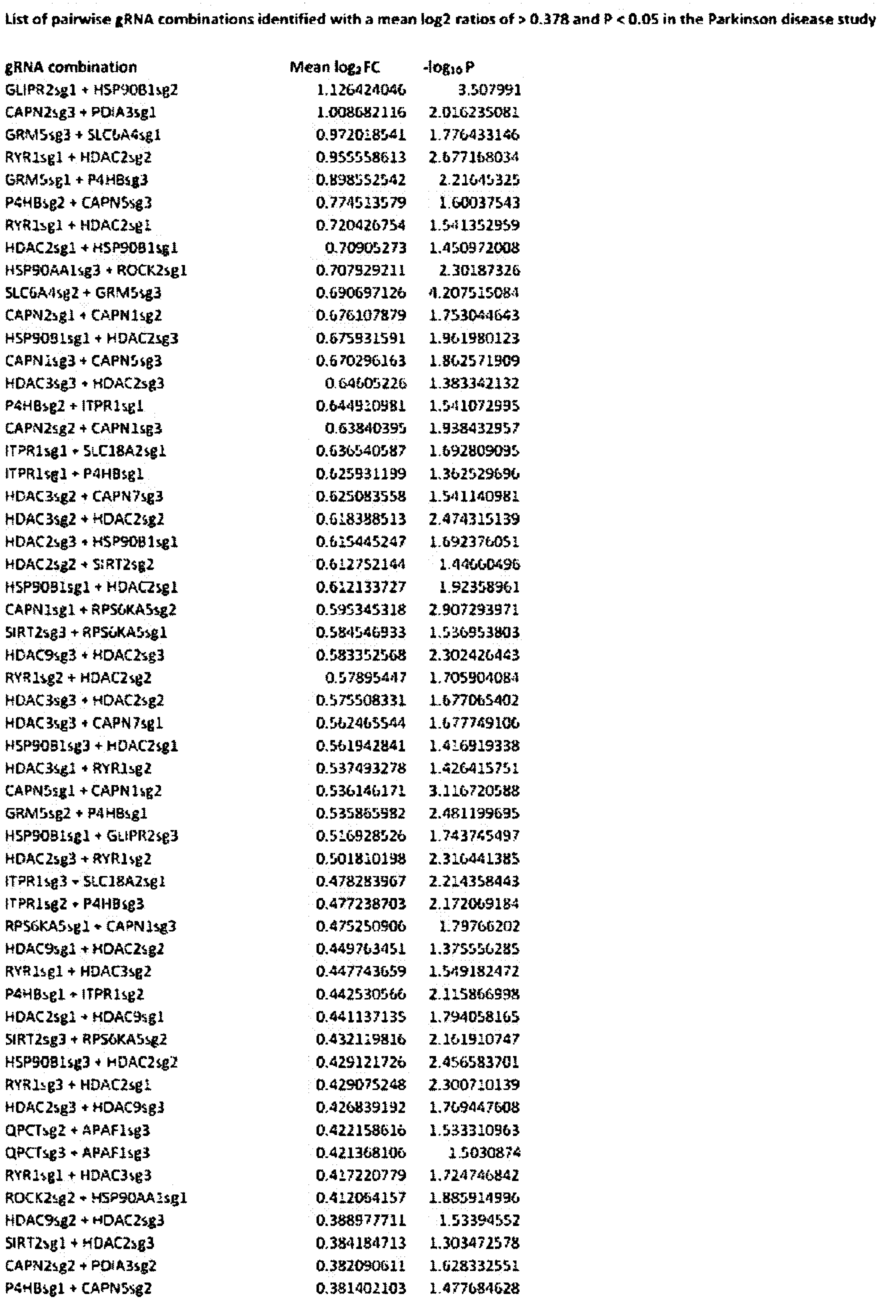

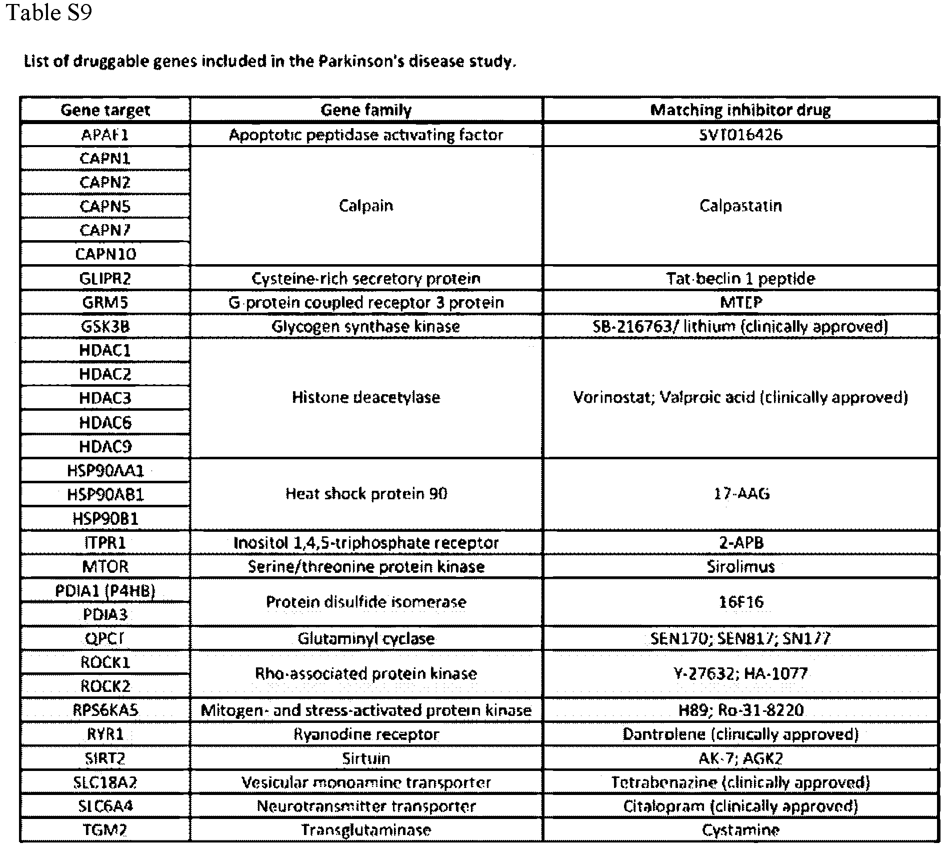

### 5.7 Discussion

In summary, we have established a CRISPR-based multi-gene knockout screening platform to address the unmet need for rapid identification of effective three-way therapeutic combinations. Via pairing drug mechanisms of action to specific genes helps accelerating the identification of effective combinations for directing secondary screens and narrows a vast number of possible combinations down to few top-performing hits for further testing. We have demonstrated that systematic characterization of three-way combinations using CRISPR-based screening discovers the rare ones with synergistic interactions as most of them showed buffering interactions and were able to validate all three screen hits with strong growth inhibition effects and three-way interactions. Our CombiGEM-CRISPR v2.0 platform has broad utility as it can also be used for identifying new two- drug regimens that inhibit cancer cell growth (Figures 13-14) and enhance protection against other disease phenotypes, such as Parkinson's disease (PD)-associated toxicity (Figure 15), as well as extended to analyse interactions among > 3 genetic components by using additional engineered promoters and scaffolds including our newly engineered v3.11, v.3.12, and v3.13 scaffolds (See Figure 16) for multiplexed CRISPR-based editing (Reis et al., 2019).

As described in Figure 16, engineered gRNA scaffold variants exhibit improved on-target and low off-target activities. A, Sequence of gRNA scaffold sequences used. B, C, OVCAR8-ADR cells harboring reporter constructs with on-target (B) and off-target (C) sites were infected with lentiviruses encoding wildtype or Opti-SpCas9. The editing efficiency of the gRNA scaffold variants was measured as the percentage of cells with depleted RFP fluorescence. D, Assessment of gRNA scaffold variants for efficient on-target editing with gRNAs targeting endogenous loci. The percentage of sites with indels was measured using a T7 endonuclease I (T7E1) assay. The ratio of the on-target activity of gRNA scaffold variants to the activity of scaffold was determined, and the median and interquartile range for the normalized percentage of indel formation are shown for the 5 loci tested. Each locus was measured three times. E, GUIDE-seq genome-wide specificity profiles for the panel of gRNA scaffold variants paired with the indicated gRNAs. Mismatched positions in off-target sites are colored, and GUIDE-seq read counts were used as a measure of the cleavage efficiency at a given site.

This platform is also versatile to be used together with dCas9-based CRISPR interference systems (Qi et al., 2013) to partially lower the target gene expressions for mimicking drug inhibitor effects. This platform could be coupled with other technologies like single-cell RNA-seq to explore different cell signatures and contribute to the generation of druggable gene interaction network using existing knowledge (Adamson et al., 2016; Bassik et al., 2013; Chow et al., 2019; Du et al., 2017; Han et al., 2017; Shen et al., 2017). The platform presented in this study is easy-to-implement and will be valuable for perturbing the multi-layer genetic networks for understanding complex biological systems and designing new combination therapies.

### 6. EXAMPLES

### Experimental model and subject details

### Cell culture and generation of cell lines

HEK293T (female) and SK-N-MC (female) cells were obtained from American Type Culture Collection (ATCC). OVCAR8-ADR (female) cells were a gift from T. Ochiya (Japanese National Cancer Center Research Institute, Japan)(Honma et al., 2008). The identity of the OVCAR8-ADR cells was confirmed by a cell line authentication test (Genetica DNA Laboratories). KURAMOCHI (female) and OVSAHO (female) cells were obtained from Japanese Collection of Research Bioresources (JCRB) Cell Bank. iPSC-derived dopaminergic neurons were obtained from TGD Life Company Limited. OVCAR8-ADR-Cas9 and SK-N-MC-Cas9 cells were generated by transducing pAWp30 (Addgene, 73857) into the OVCAR8-ADR and SK-N-MC cells, respectively, followed by selection using zeocin (Life Technologies) for stable Cas9-integrated cells. The *Streptococcus pyogenes* Cas9 was used in this study. OVCAR8-ADR reporter cells that stably express RFP and GFP were generated by transducing the cells with pAWp9, followed by sorting based on GFP and RFP signals. The reporter cells were then infected with pAWp30 to stably integrate Cas9 after zeocin selection. HEK 293T and S-N-MC cells were cultured in DMEM supplemented with 10% FBS and 1 X antibiotic-antimycotic (Life Technologies) at 37°C with 5% CO₂. KURAMOCHI, OVSAHO, and OVCAR8-ADR cells were cultured in RPMI 1640 supplemented with 10% FBS and 1 X antibiotic-antimycotic at 37°C with 5% CO₂. Cells were checked for mycoplasma contamination every three or four months and were never tested positive.

### Methods details

### Plasmid construction

The vectors used in this study (Table S5) were generated by standard molecular cloning strategies, including PCR, oligo annealing, restriction enzyme digestion, ligation, and Gibson assembly. Custom oligonucleotides were purchased from Genewiz. Vectors were transformed into *E. coli* strain *DH5α* competent cells and selected with ampicillin (100 µg/ml, USB) or carbenicillin (50 µg/ml, Teknova). DNA was extracted and purified by Plasmid Mini (Takara and Tiangen) or Midi preparation (Qiagen) kits. Sequences of the vectors were verified with Sanger sequencing.

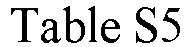

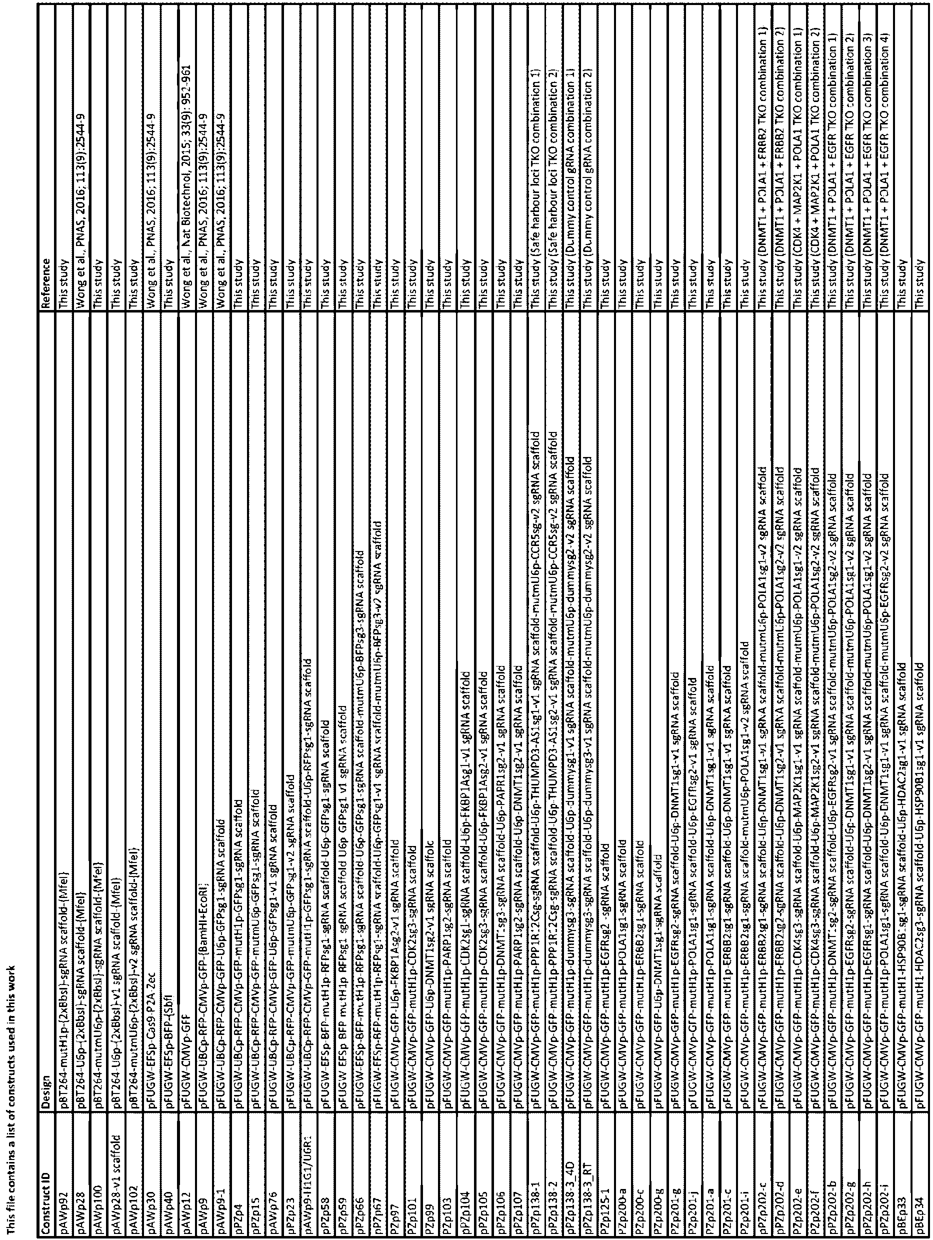

To construct storage vectors with mouse U6 (mU6)- and human H1 (hH1) promoter-gRNA WT scaffold sequences, the promoter sequences were amplified from mouse and human genomic DNAs, respectively, and cloned into the vector backbone of pAWp28 (Addgene, 73850). pAWp28 is the storage vector with human U6 (hU6) promoter-gRNA WT scaffold sequence. Storage vectors with hU6-gRNA v1 scaffold and mU6-gRNA v2 scaffold were created by PCR-based mutagenesis. To drive gRNA expression to target the gene of interest, oligo pairs with gRNA target sequences were synthesized, annealed, and cloned into BbsI-digested storage vectors by T4 DNA ligase (New England Biolabs). To generate lentiviral vectors for expression of single gRNA targeting *GFP*, *RFP*, and *BFP* gene, the gRNA expression cassettes were released from the storage vectors by digestion with BglII and EcoRI enzymes (Thermo Fisher Scientific) and cloned into pAWp9 vector (Addgene, 73851) using ligation via the compatible stick ends generated by digestion of the vector with BamHI and EcoRI enzymes (Thermo Fisher Scientific). To build lentiviral vectors for expression of multiple gRNAs targeting the fluorescent proteins, the second gRNA expression cassette with hU6-gRNA-v1 (or WT) scaffold was released from the storage vector by digestion with BglII and EcoRI enzymes, and ligated into the BamHI- and EcoRI-digested storage vector containing the first gRNA expression cassette with hH1-gRNA-WT scaffold. Similarly, the third gRNA expression cassette with mU6-gRNA-v2 (or WT) scaffold was released from the storage vector by digestion and ligated into the storage vector harbouring the first and second gRNA expression cassettes. Lentiviral vectors were then generated by amplifying the pairwise or three-way gRNA expression cassettes from the storage vector by PCR, and cloned into the Sbfl-digested pFUGW vector backbone (pAWp40) by Gibson assembly.

### Guide RNA library design and assembly

The gRNAs used in this study were designed based on GPP sgRNA Designer (Table S3). For the pairwise gRNA libraries, three gRNAs were selected per target gene based on the following criteria: 1) on-target efficacy scores are > 0.6; 2) off-target ranks are < 100; and 3) target sites are within 5-65% of the protein-coding sequence. gRNA sequences containing BamHI, EcoRI, BglII, and MfeI digestion sites were excluded to avoid incompatibility with CombiGEM. For the three-wise combinatorial gRNA library, two gRNAs were selected per target gene using the same criteria, except that their on-target efficacy scores are all > 0.64. inDelphi and FORECasT were applied to predict the frameshift rate of gRNA. The gRNA sequences were inputted into BLAST to extract the 70-nucleotide context sequences of the gRNAs. The PAM sequence index were located in the 70-nt sequences and were inputted alongside with the context sequence into inDelphi and FORECasT, which were downloaded from GitHub. The K562 cell line was the prediction model used in inDelphi, and the output frameshift scores are extracted from the "Frameshift frequency" option. The output summary file from FORECasT was inputted into a Python code calculating the predicted frameshift frequency by summing up the percentage of the target frameshift categories that are not multiples of three then dividing the sum by 10.

To assemble the gRNA libraries (Figure 6B), oligo pairs with the gRNA target sequence, two BbsI restriction digestion sites, and a unique 8-bp barcode were annealed, pooled at an equal molar ratio, and cloned into storage vector backbones containing the hH1, hU6, and mU6 promoter sequences (pAWp92, AWp28, and pAWp100, respectively). The gRNA scaffold sequences (WT, v1, and v2) were then inserted into the vectors to create three pooled libraries of barcoded single gRNA expression cassette. The combinatorial gRNA libraries were assembled using the CombiGEM-CRISPR method (Wong et al., 2016a). The first pool of inserts was released from the storage vector containing the hH1-gRNA-WT scaffold by digestion with BglII and MfeI enzymes and ligated into the BamHI- and EcoRI- digested pAWp12 (Addgene, 72732) to generate the barcoded single gRNA library in a lentiviral vector. Then, the second pool of inserts was released from the storage vector containing the hU6-gRNA-v1 scaffold by digestion and ligated into the lentiviral vector containing the first gRNA expression cassette to generate the barcoded pairwise gRNA library. Similarly, the third pool of inserts was released from the storage vector containing the mU6-gRNA-v2 scaffold and inserted into the lentiviral vector containing the two gRNA expression cassettes to generate the barcoded three-way combinatorial gRNA library. The three-way combinatorial gRNA library was delivered into OVCAR8-ADR cells using lentiviruses, and Sanger sequencing analysis was performed on genomic DNA extracted from single cell-derived clones and confirmed the majority of assembled barcoded gRNA constructs (7 out of 8 colonies) harbored the expected gRNA target sequences. To construct the individual combinatorial gRNA vectors used in the validation experiments, the same assembly strategy was used, except that the annealed oligo pairs were not pooled.

### Lentiviral vector generation and transduction

The second-generation lentiviral vector system was used in this study. HEK293T cells were transfected by FuGene HD transfection reagent (Promega) according to manufacturer's instructions in 6-well plate, with 0.5 µg of pCMV-VSV-G, 1µg of pCMV-dR8.2-dvpr, and 0.5 µg of the respective lentiviral vector per well. Lentivirus-containing supernatants were collected at 48 and 72 hrs post-transfection, which are then combined and filtered by 0.45 µm polyethersulfone membrane (Pall). For routine transduction, we applied 300 µL of the filtered supernatant to one well of 12-well plate in the presence of 8 µg/ml polybrene (Sigma), with cell confluence at about 30%. For library transduction, Cas9-expressing cells were seeded onto 150-mm culture dishes at confluence about 50% with the cell number roughly equals 400-fold representation of the library size, and were transduced by the viruses at a multiplicity of infection (MOI) of ~0.3, to ensure most cells were infected with just one virion.

### Flow cytometry, cell cycle analysis, and cell sorting

To prepare samples for flow cytometry, cells were trypsinized and resuspended in FACS buffer (PBS with 2% FBS). BD LSR Fortessa analyser (Becton Dickinson) was used to detect the signal of TurboRFP, EGFP, and mTagBFP by 561 nm yellow-green laser (610/20 nm), 488 nm blue laser (530/30 nm), and 405 nm violet laser (450/50 nm), respectively. For cell cycle analysis, cells were fixed by ice-cold 70% ethanol at 4°C for 1 hr, and then rehydrated by replacing the ethanol with PBS for 15 min at room temperature. To remove RNAs, RNase A (10 mg/ml) was added to the cells and incubated at 37°C for 15 min. Genomic DNA contents were stained by propidium iodide (PI; Invitrogen) for 1 hr at room temperature in dark. Signal was detected by 561 nm yellow-green laser (586/15 nm) using a BD LSR Fortessa analyser. FlowJo software (v10.5.3, Becton Dickinson) was used for data analysis. For cell sorting, samples were prepared similarly as for FACS analysis, except that FACS buffer was supplemented with 2 X antibiotic-antimycotic. BD Influx cell sorter (Becton Dickinson) equipped with 100-µm nozzle (24 psi with a frequency of 39.2 kHz) was used. GFP-positive cells were detected by 488 nm blue laser (530/40 nm) and sorted using 1.0 Drop Pure mode. For cells being infected with the screening libraries, the 1-2% cells that had the strongest GFP signals were not collected to minimize the chance of acquiring cells that were infected with more than a single virion. At least 100-fold more cells than the library size were collected.

### Sample preparation for barcode reading

For library-transduced cell pool, genomic DNA was extracted from cells with DNeasy Blood and Tissue kit (Qiagen) and quantified by Quant-iT PicoGreen dsDNA Assay kit (Life Technologies). To extract the 298-bp barcode-containing fragments, 0.5 ng of library plasmid DNA and 800 ng of genomic DNA per 50 µl of PCR reaction were used for PCR amplification using Kapa HiFi Hotstart Ready-mix (Kapa Biosystems). The forward and reverse primers used were 5'-GGATCCGCAACGGAATTC-3' and 5'-GGTTGCGTCAGCAAACACAG-3'. The PCR amplification was kept at the exponential phase to minimize PCR bias. To ensure sufficient library coverage amplified from the genomic DNA, 20 and 10 PCR reactions were performed for the pairwise libraries used in studying ovarian cancer and Parkinson's disease, respectively, and 30 PCR reactions were performed for three-way combinatorial library. Illumina adapters and sequencing indices were then added to the amplicons by performing PCR using Kapa HiFi Hotstart Ready-mix. The forward and reverse primers used were 5'-CAAGCAGAAGACGGCATACGAGATGTGACTGGAGTTCAGACGTGTGCTCTTCCGAT CTGGTTGCGTCAGCAAACACAG-3' and 5'-AATGATACGGCGACCACCGAGATCTACACTCTTTCCCTACACGACGCTCTTCCGATC TNNNNNNNN(N₁₋₄)NNNNNNNN₁₋₄) indicates the 1 to 4 nucleotides added to increase the diversity of the sequencing library. The final amplicons were purified by two rounds of size selection using a 1:0.5 and 1:0.95 ratio of Agencourt AMPure XP beads (Beckman Coulter Genomics). Quantity and quality of samples were measured by real-time PCR using Kapa SYBR Fast qPCR Master Mix (Kapa Biosystems) with primer pair 5'-AATGATACGGCGACCACCGA-3' and 5'-CAAGCAGAAGACGGCATACGA-3', and analysed using a high-sensitivity DNA chip (Agilent) on an Agilent 2100 Bioanalyzer.

### Barcode sequencing data analysis

Barcode reads were processed from the sequencing data and normalized to count per million reads for comparison among samples. The normalized barcode counts for each gRNA combination in the cell pools were compared to the ones for dummy control gRNA combination within each sample to generate a log₂-transformed fold change. To improve data reliability, combinations that had a raw barcode read of < 100 in the early time point samples from the ovarian cancer studies were excluded (Figures 4C and 13C). Combinations that had a raw barcode read of < 20 in the untreated samples from the Parkinson's disease study were filtered out as there were fewer total reads (Figure 15D). Also, only combinations that contain gRNAs targeting different genes, and had a coefficient of variation (CV) of < 1 are included in the analysis (Figures 4E and 13E). Overall, 97.3% (22,921 out of 23,556 combinations) and 79.1% (21,172 out of 26,782 combinations) of the pairwise and three-way combinations are included, respectively, in the ovarian cancer study. 96.7% (6,878 out of 7,108 combinations) of the pairwise combinations are included in the Parkinson's disease study. In the ovarian cancer studies, gRNA combinations with log₂ fold-change of < -1 are listed in Tables S6 and S7. In the Parkinson's disease study, gRNA combinations with log₂ fold-change > 0.378 (~30% increase) and P < 0.05 are listed in Table S8. To identify synergistic three-way combinations, the gRNA combinations targeting three non-redundant genes with a coefficient of variance < 1 were grouped into the total of 455 unique three-gene targeting combinations. The R package DescTools was used to perform Dunnett's test, where the three-way combinations served as the control and being compared to the two-gene and single-gene targeting combinations (6 comparisons in total). To measure genetic interactions, a scoring system similar to one we previously described was applied (Wong et al., 2015). Genetic interaction (GI) scores were calculated by subtracting the expected fold change of the triple-gene knockout, which is estimated by the sum of respective double- and single- gene knockouts' fold changes, by the observed fold change of the triple-gene knockout, where a negative GI score indicates genetic synergy in this study. GI scores calculated for a given three-way combination represent the interaction between the third gene with the remaining two genes in combination. The GI₃ score for a three-way combination was calculated based on the geometric mean of the three GI scores. The potentially synergistic gene combinations were selected through the adjusted P-value threshold of < 0.05 in all 6 comparisons from the Dunnett's test, a mean log₂ fold change of < -1, and GI₃ scores of < -0.2 (also with GI scores of < -0.14 in all 3 possible permutations ("A,B" + "C", "A,C" + "B", "B,C" + "A") for the same three-way combination).

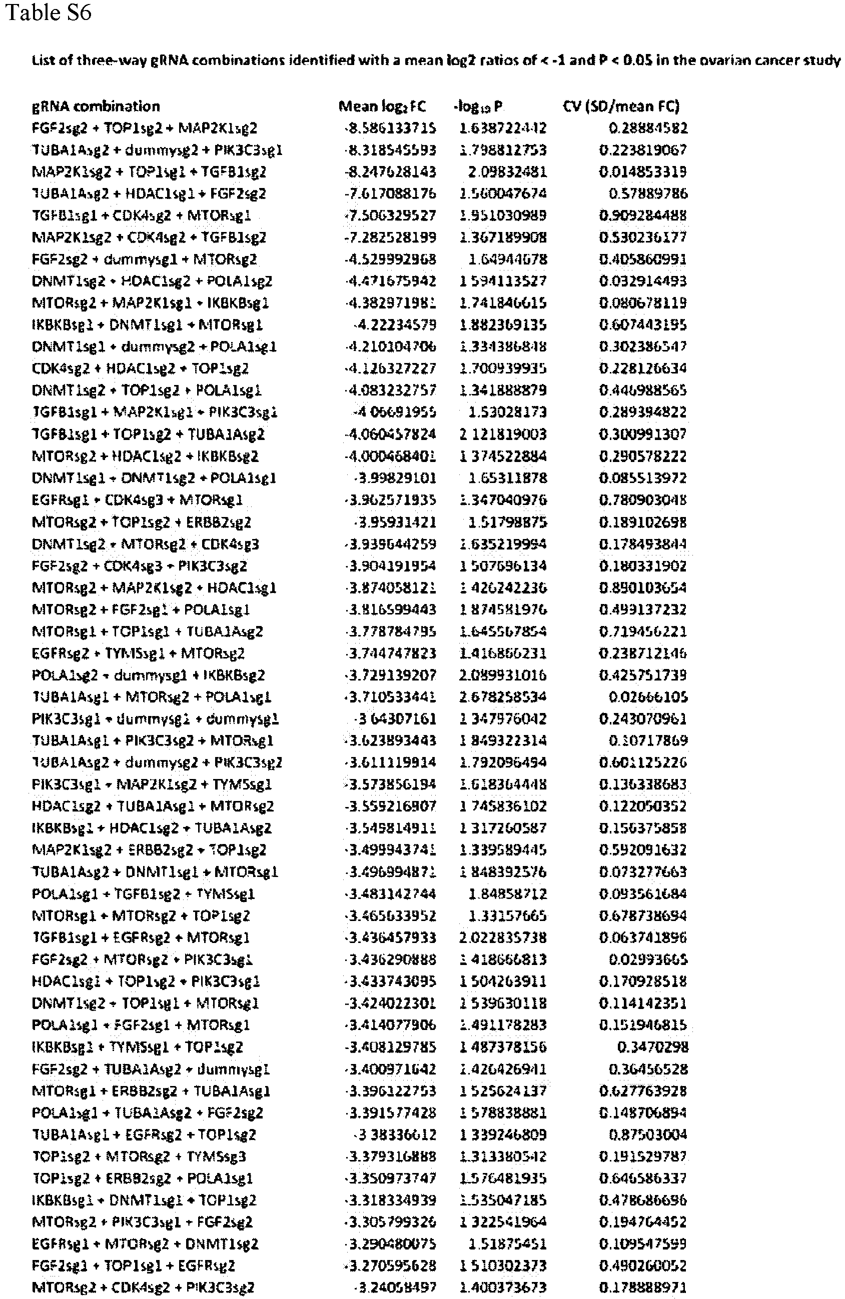

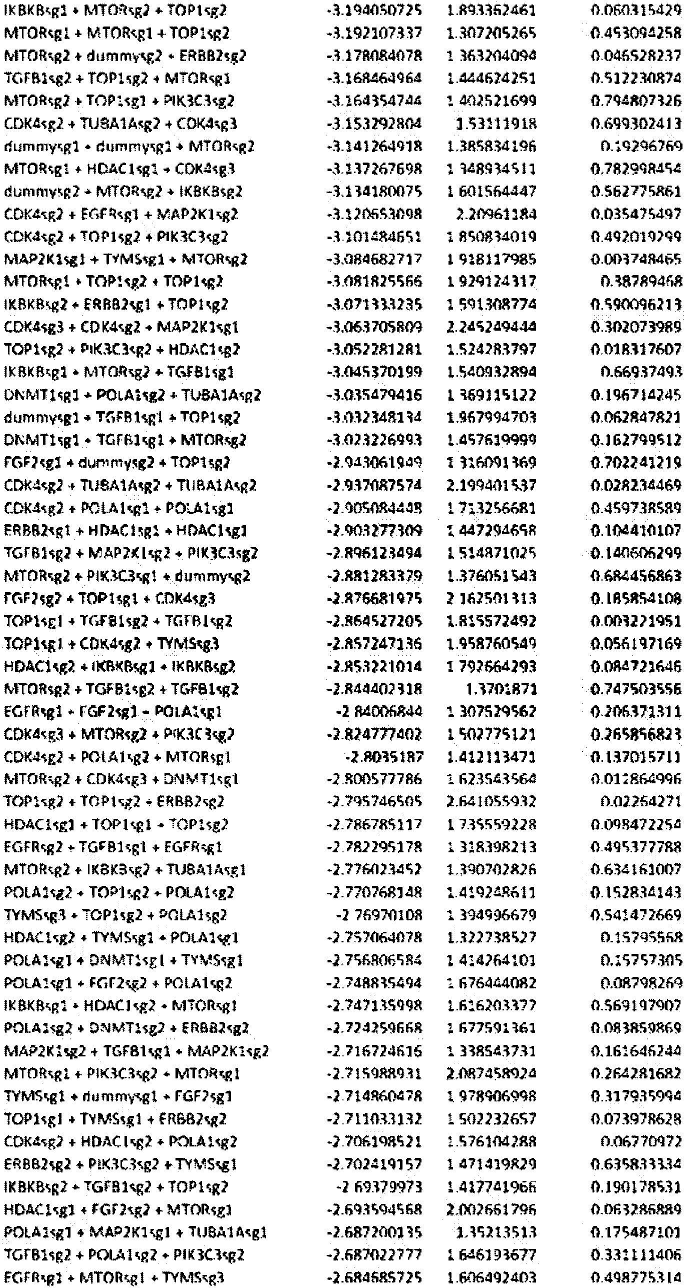

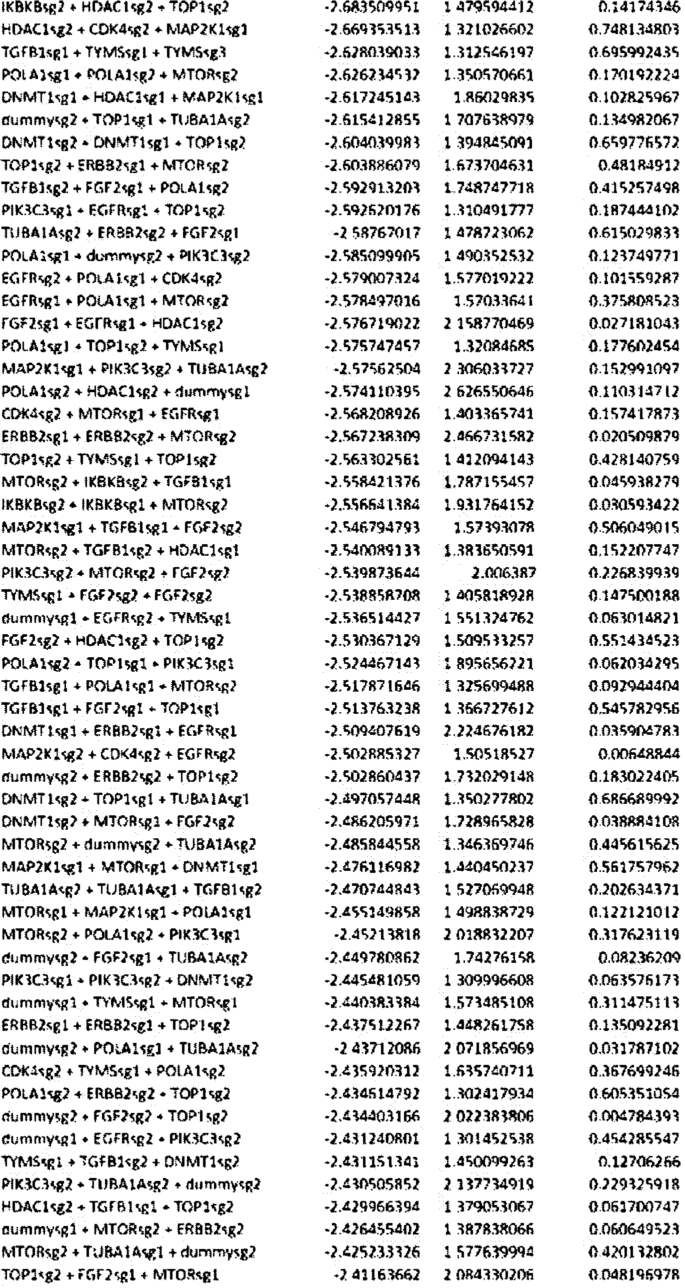

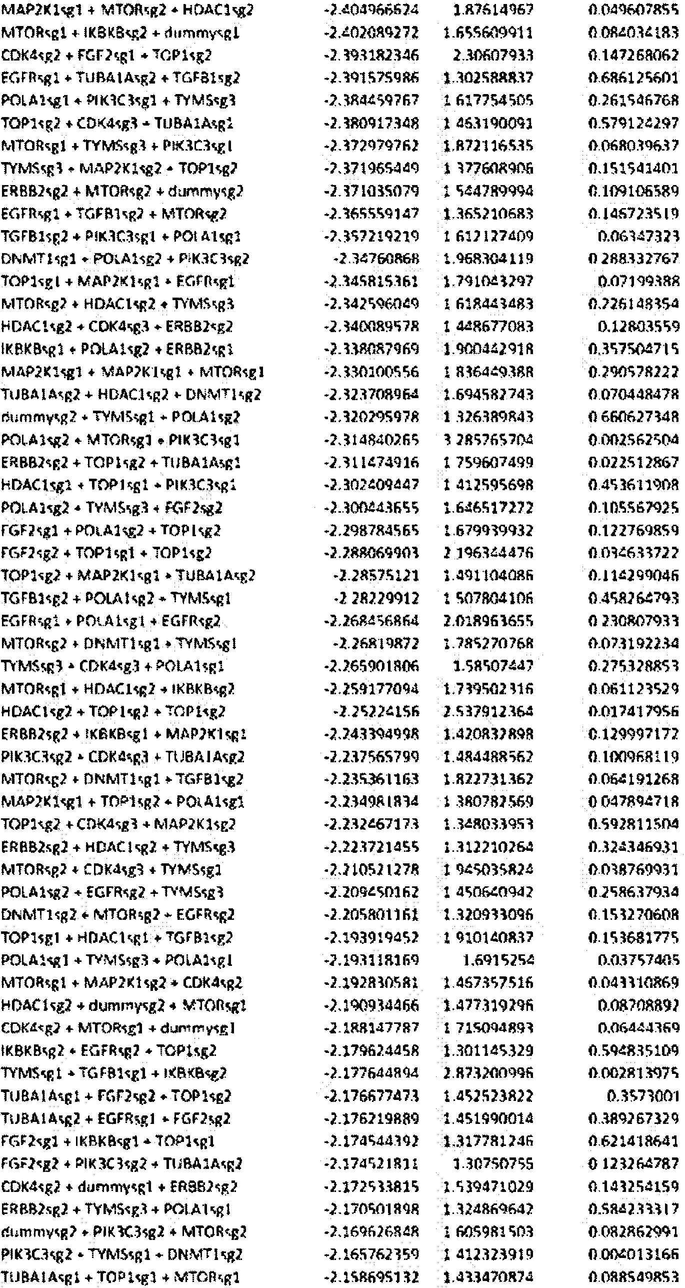

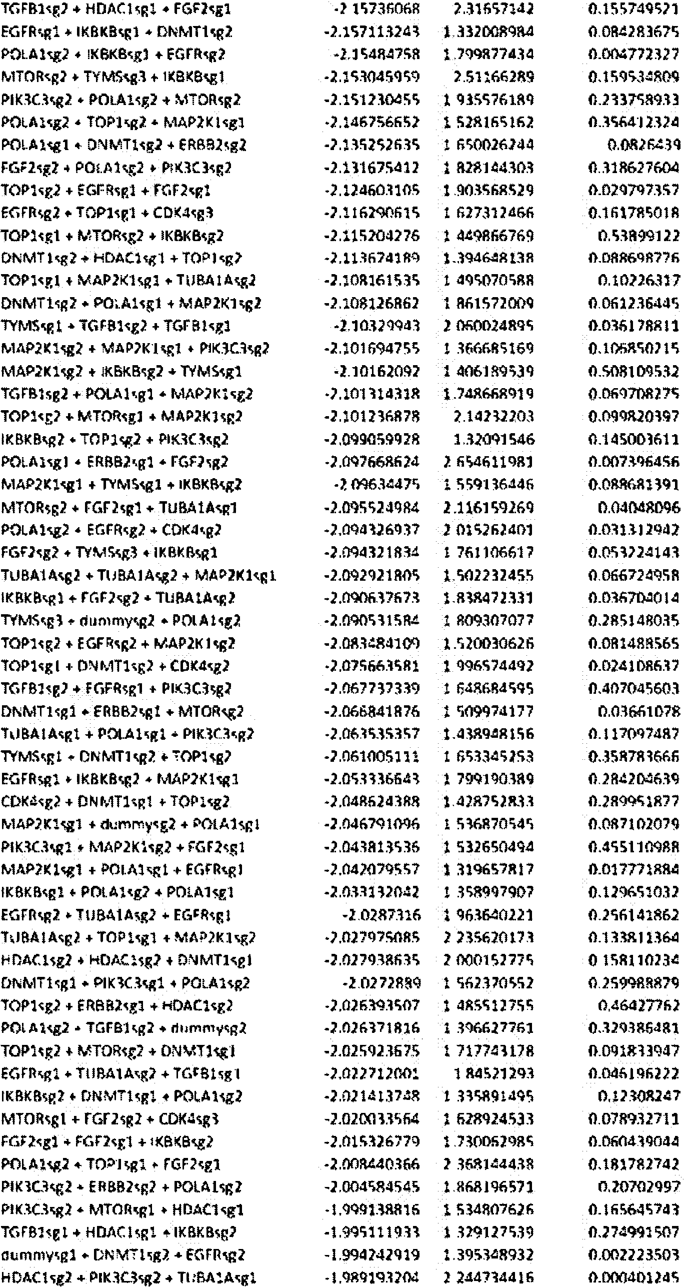

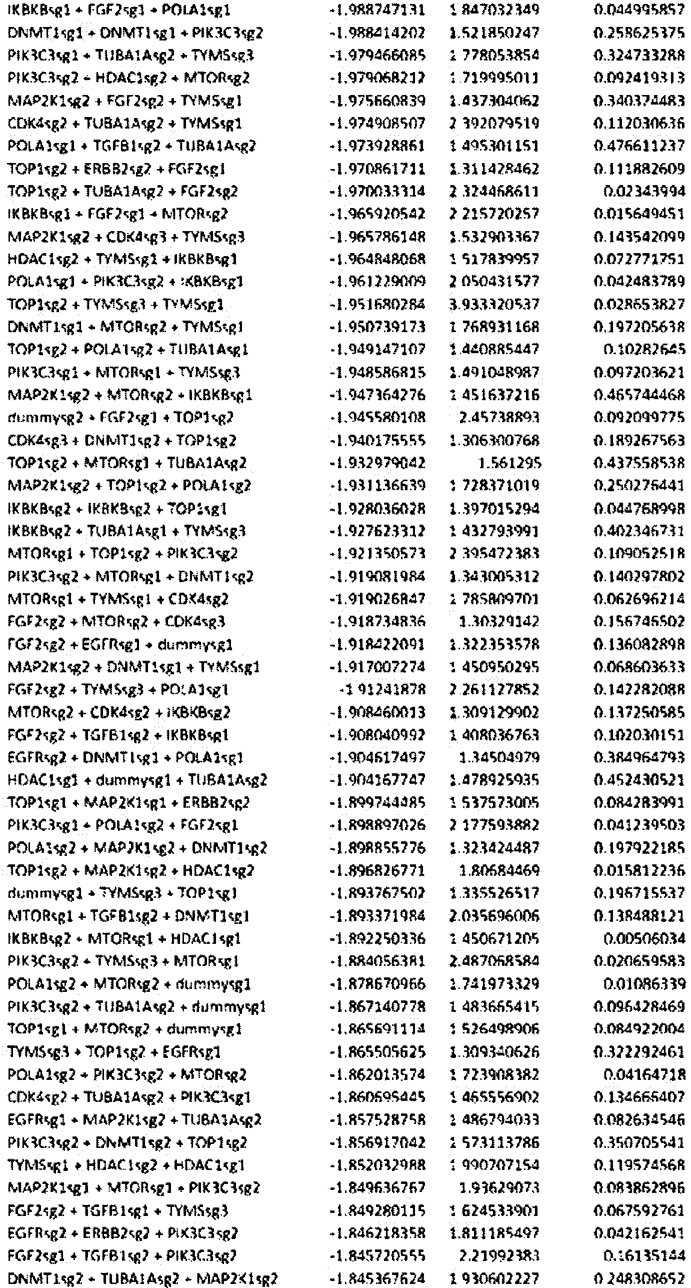

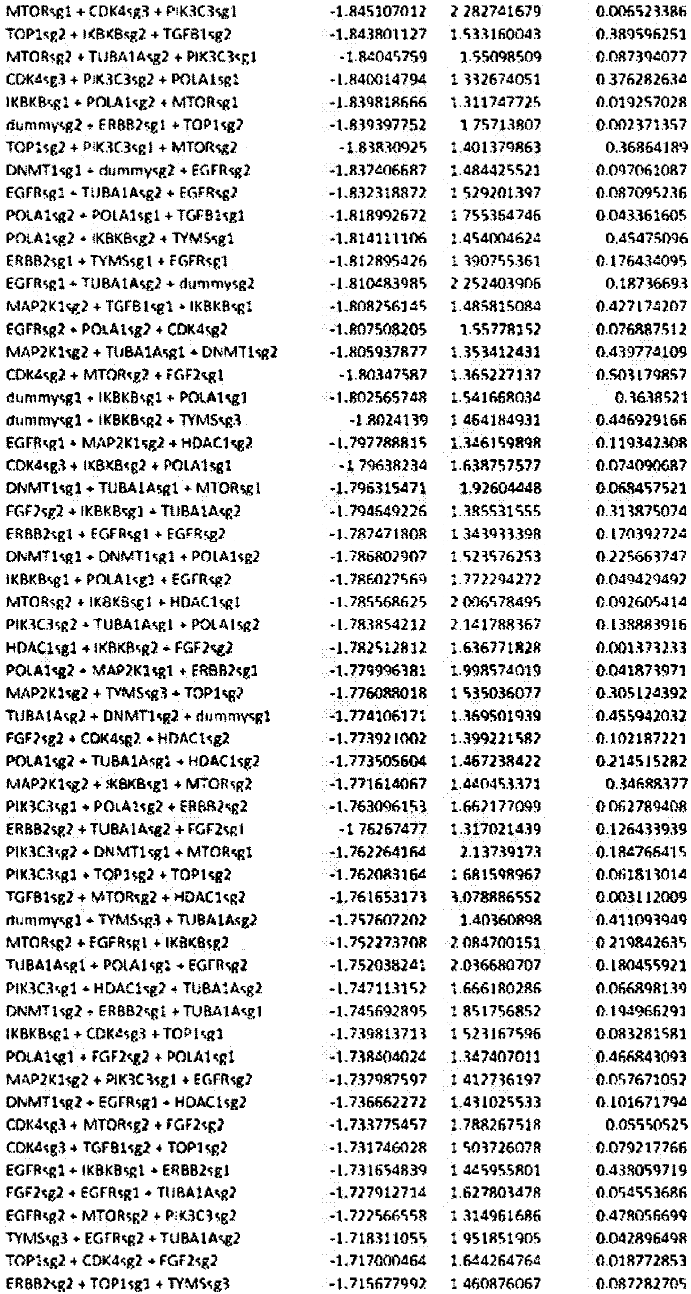

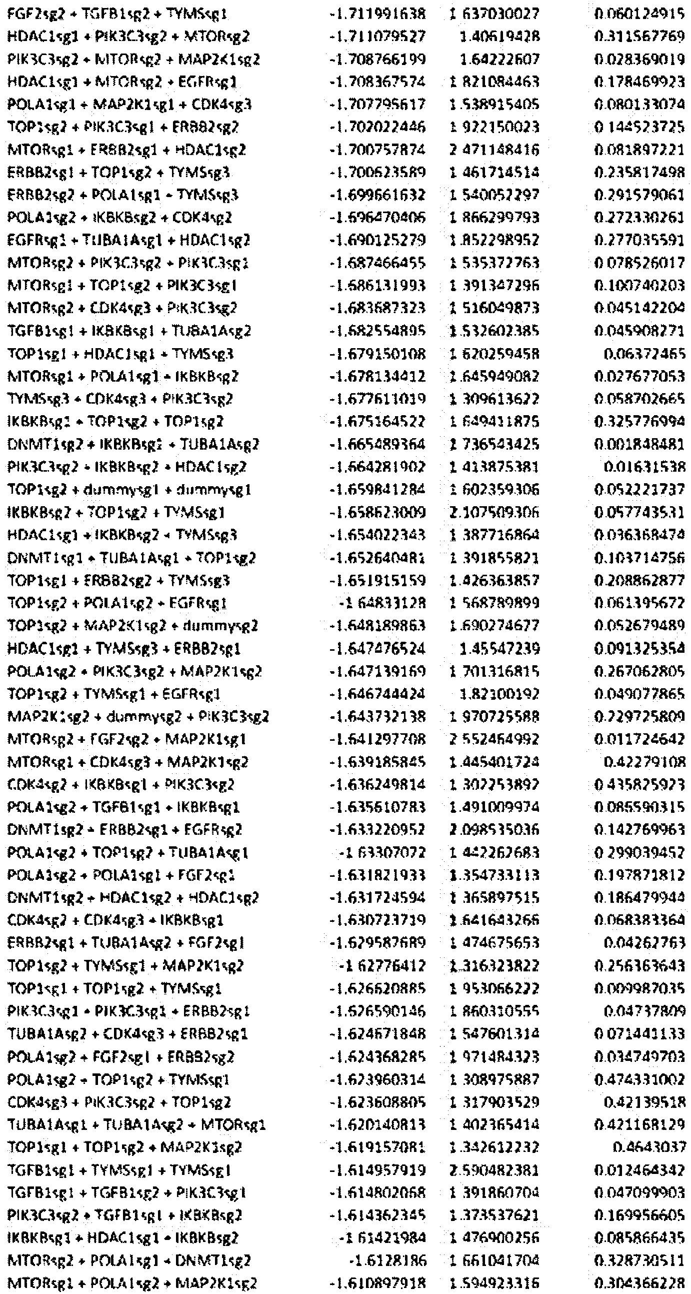

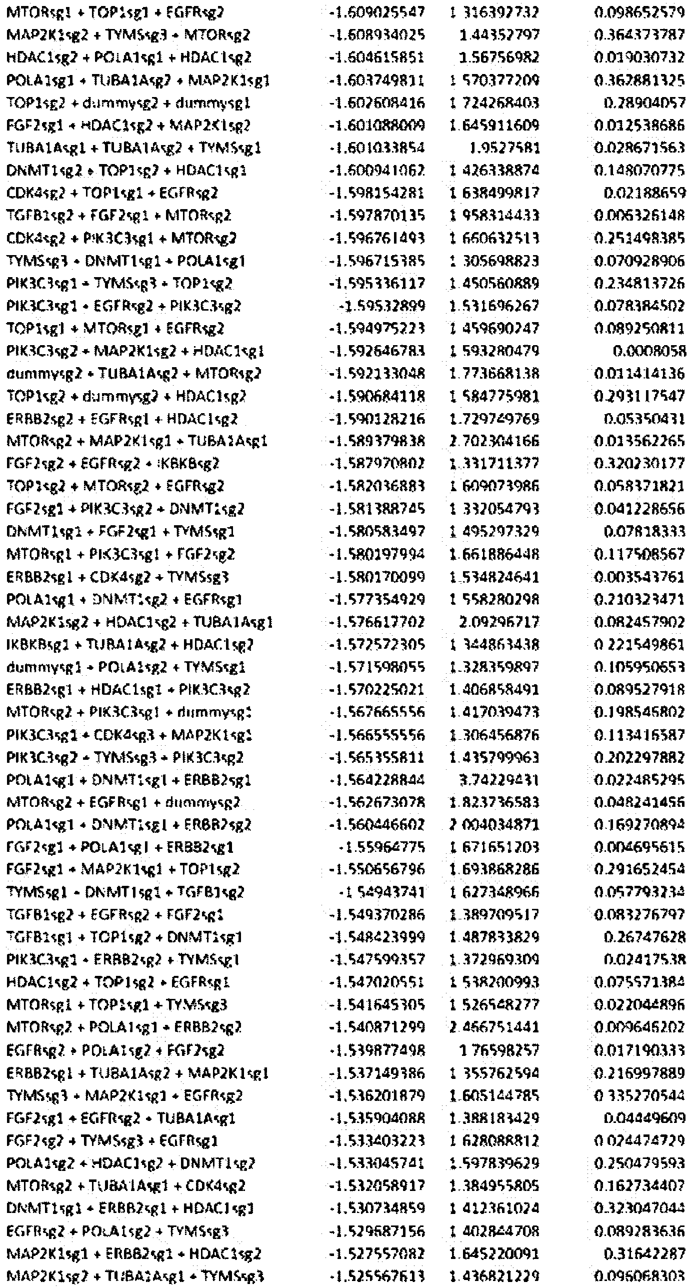

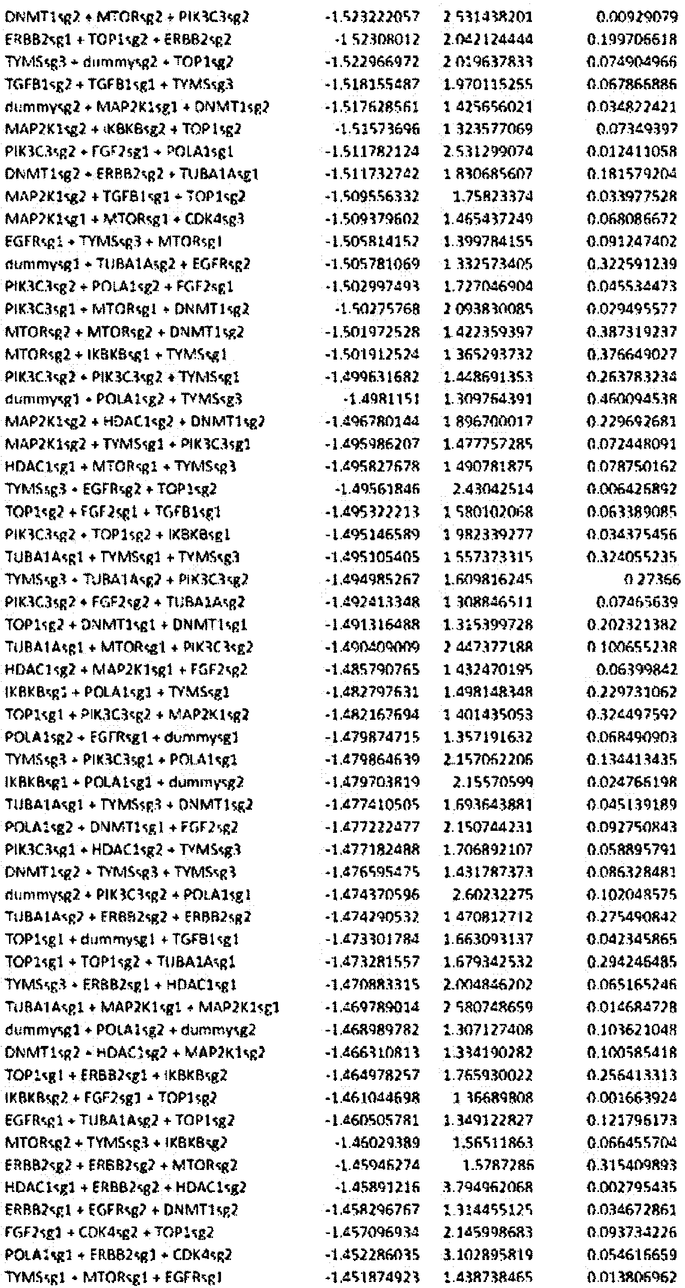

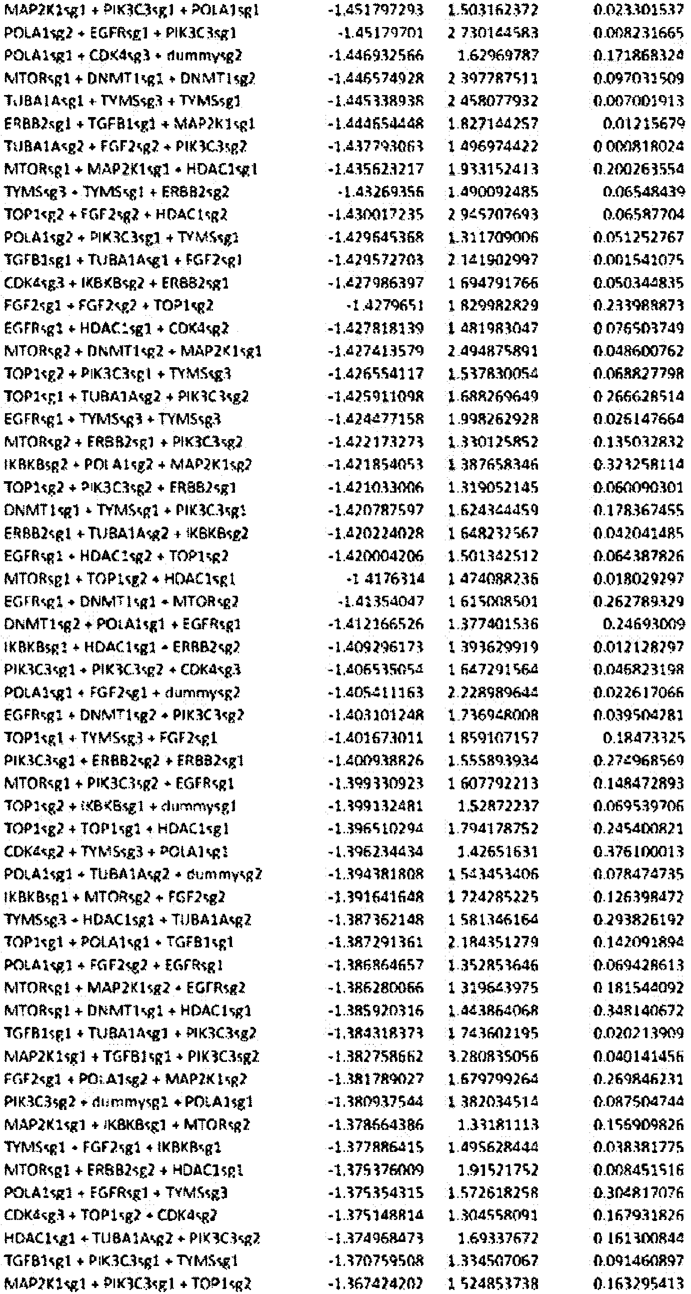

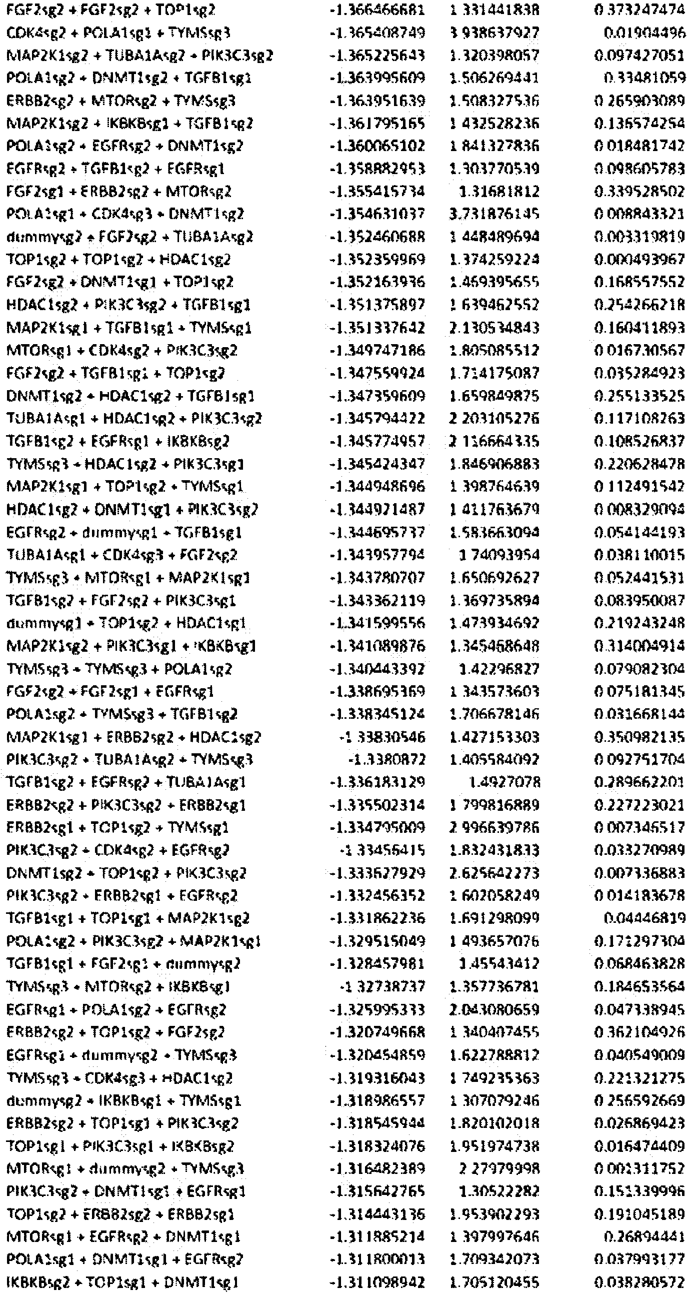

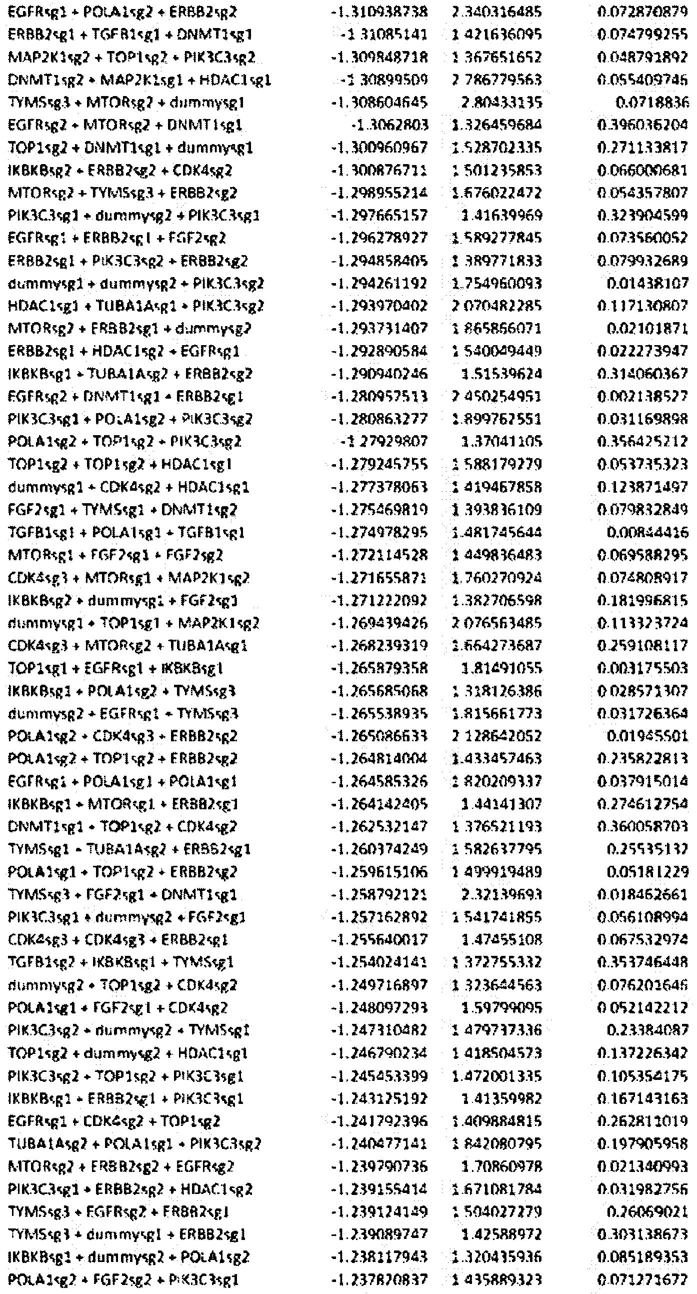

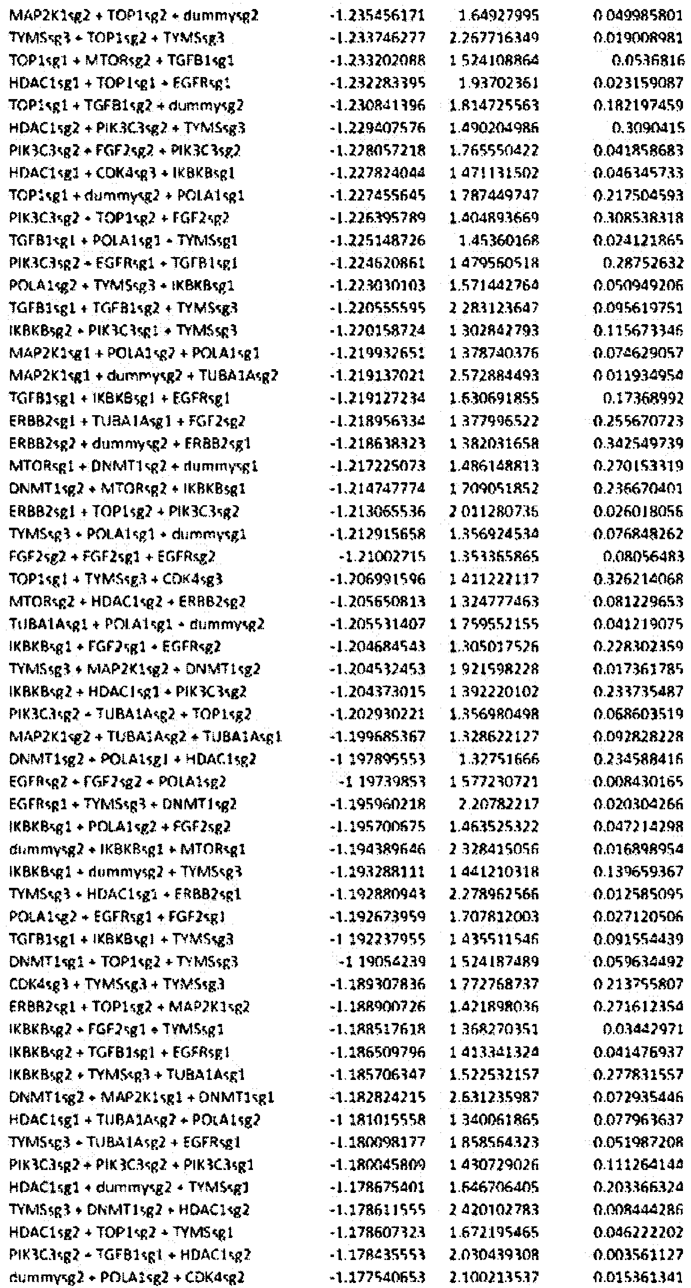

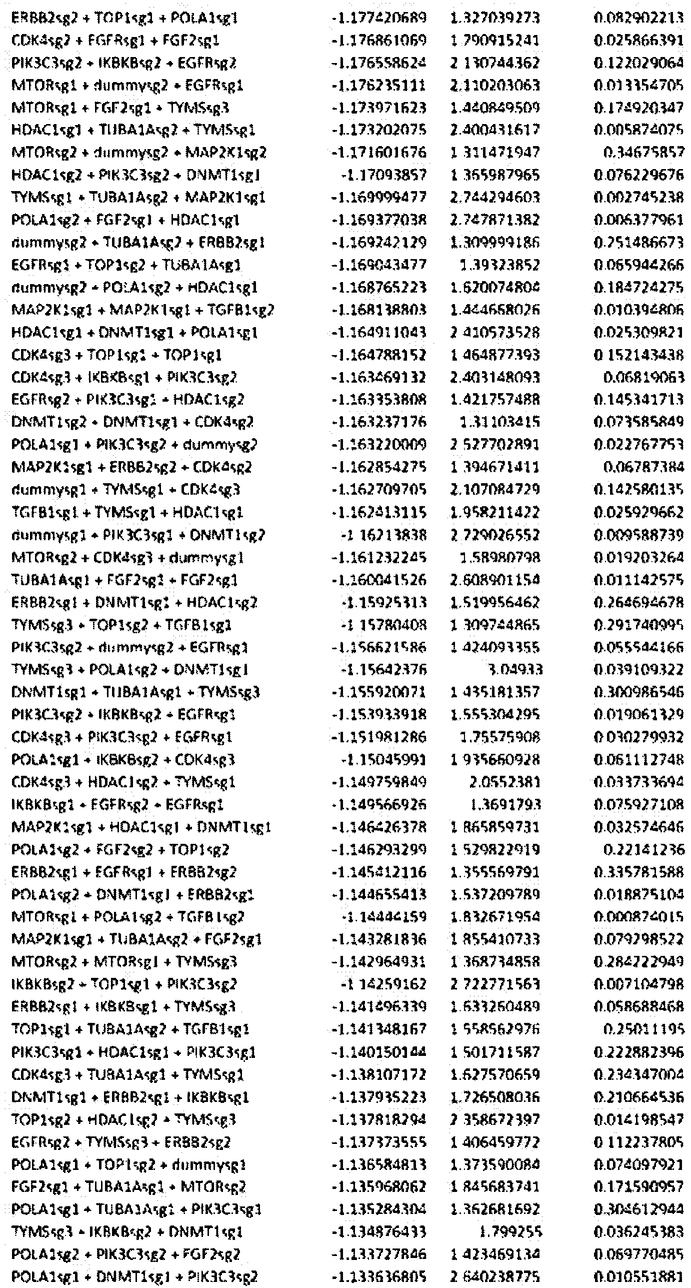

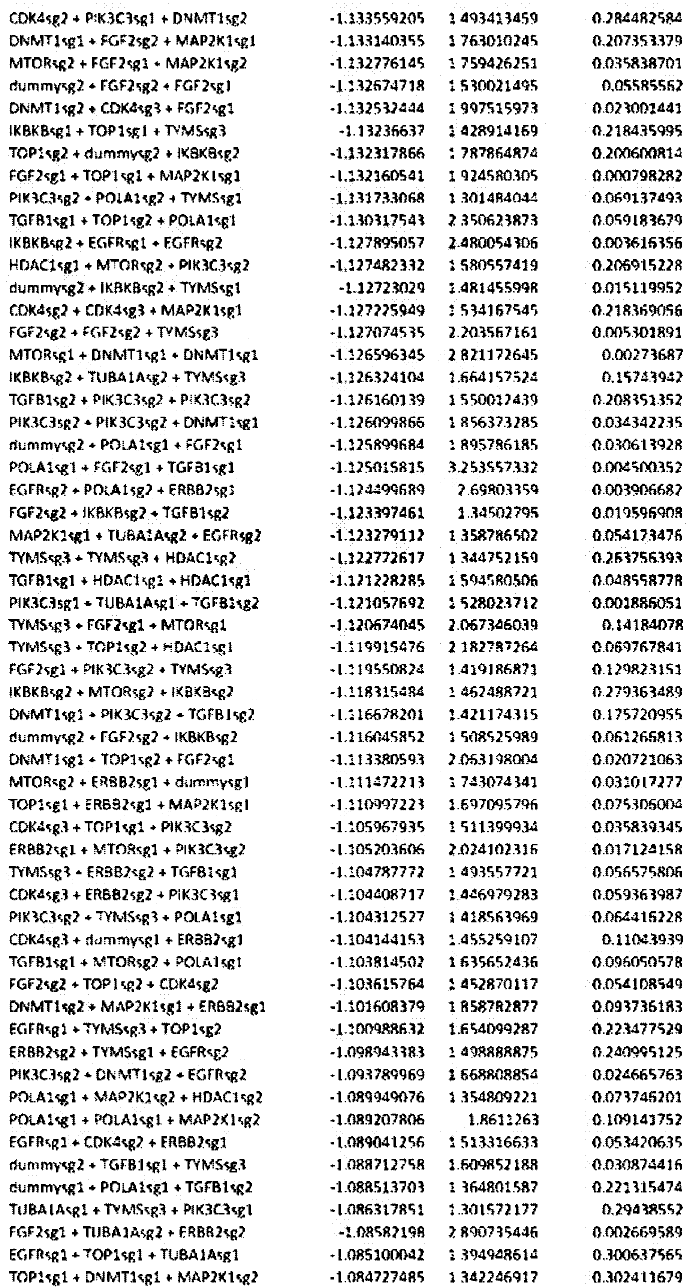

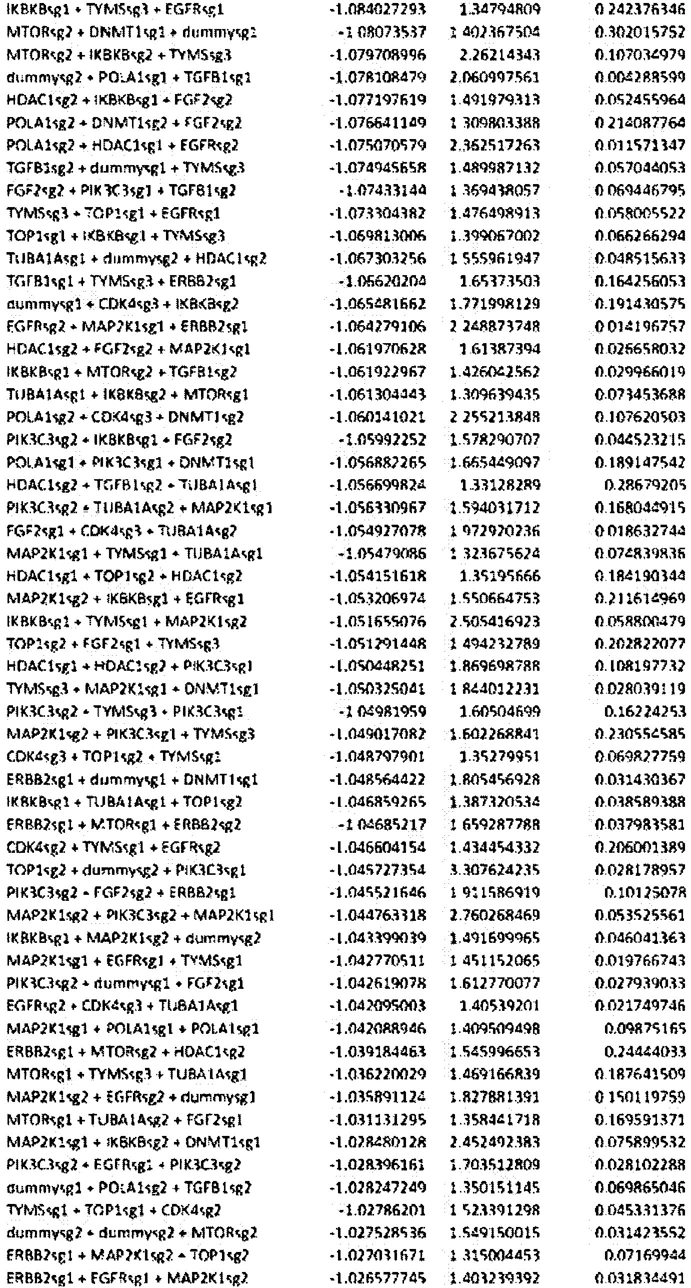

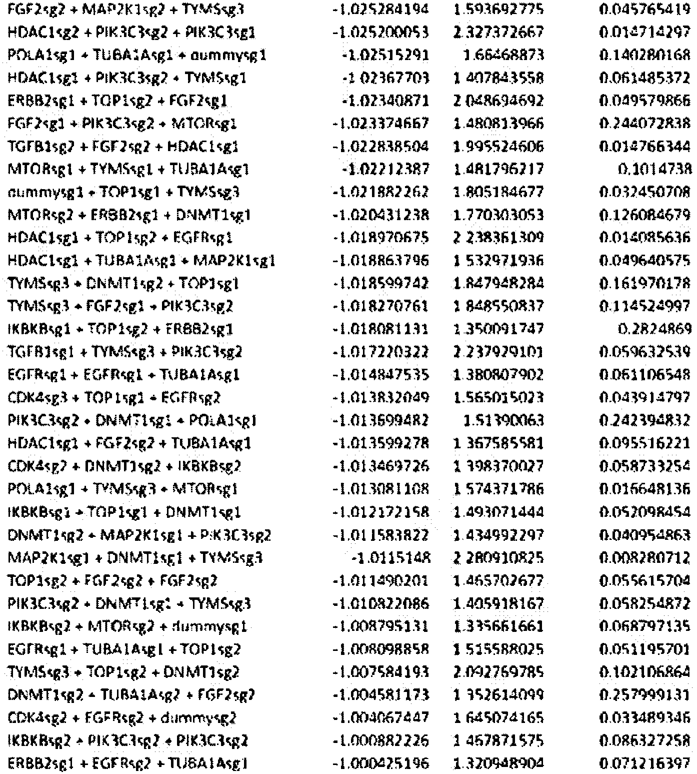

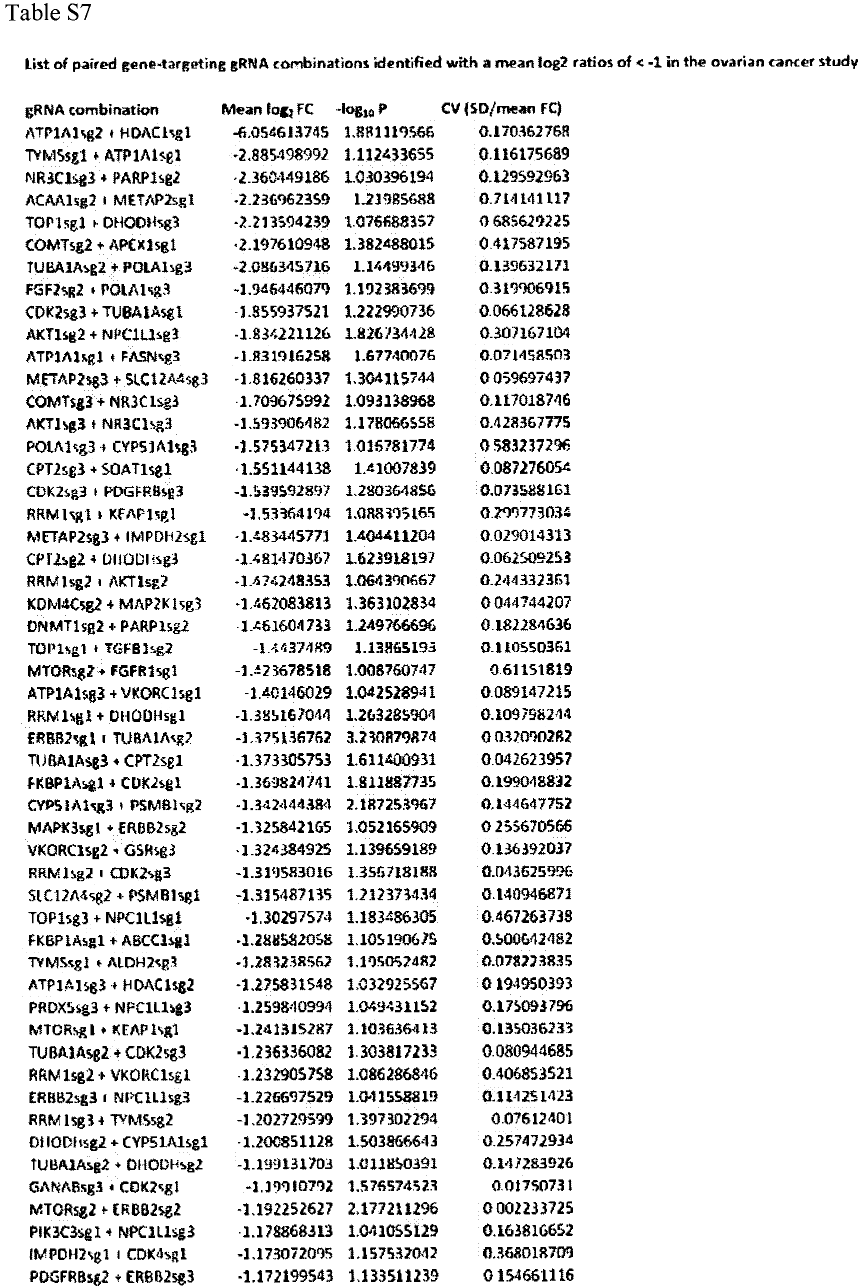

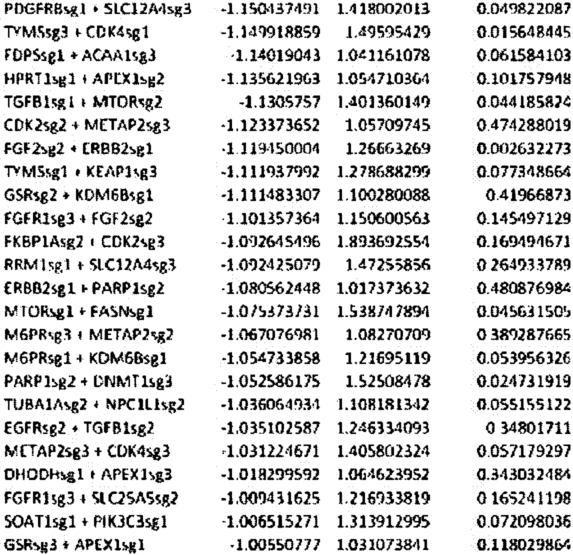

### Cell viability assay and drug interaction analysis

1,500 OVCAR8-ADR cells were seeded onto one well of a 96-well plate one day prior to drug treatment. 4,800 SK-N-MC cells were seeded onto one well of a 96-well plate and were pretreated with the drug(s) for 72 hours, followed by adding rotenone (Abcam, ab 143145) or MPP⁺ (Abcam, ab144783) to induce toxicity. Drugs were applied at indicated doses. Azacitidine (A-5959), olaparib (0-9201), sirolimus (R-5000), seliciclib (R-1234), lapatinib (L-4899), erlotinib (E4007), and vorinostat (V-8477) were purchased from LC Laboratories. Fludarabine (#14128) was purchased from Cayman Chemical Company. 17-DMAG (A2213) was purchased from ApexBio. 3-(4,5-dimethylthiazol-2-yl)-2,5-diphenyl tetrazolium bromide (MTT) assay was performed, as described previously (Wong et al., 2015), to assess cell growth at different time points. Briefly, medium in the cell growing wells were replaced by 100 ul of 1X MTT solution in RPMI 1640 without phenol red and incubated at 37°C with 5% CO₂ for 3 hrs. Then 100 µl solubilization buffer (10% Triton X-100, 0.1N HCl in isopropanol) are applied to each well to dissolve the blue formazan crystals. The absorbance was measured at 570 nm and 650 nm by VARIOSKAN FLASH microplate reader (Thermo Scientific). Bliss independent (Bliss, 1939) and HSA (Borisy et al., 2003) models were adopted for evaluating interactions between drug pairs, and DiaMOND model (Cokol et al., 2017; Cokol-Cakmak et al., 2018) was used for measuring three-way drug interactions. The excess over Bliss independent model was calculated as, g12 - (g1 + g2 - g1 x g2 / 100), where g indicates the percentage of growth inhibition, the number indicates the drug component; the excess over HSA model was calculated by subtracting the highest growth inhibition effect of single agent from that of the combination; the DiaMOND model was used for calculating the frictional inhibitory concentration (FIC₃), which equals (o1 + o2 + o3)/((e1 + e2 + e3)/3), where o indicates the observed concentration of each component in the combination, and e indicates the expected concentration of individual drugs at certain inhibitory level, which is determined by drug response curves. To generate the drug response curves, the three drugs were combined in a fixed ratio of 1:1:1 of their respective IC₅₀ and scaled proportionally. If FIC₃ is < 1, the interaction is synergistic; if FIC₃ is = 1, the interaction is additive; and if FIC₃ is > 1, the interaction is antagonistic.

### Colony formation assay

1,000 OVCAR8-ADR cells were seeded onto one well of a 6-well plate one day prior to drug treatment at indicated doses. Colonies were fixed by ice-cold methanol at -20°C for 30 min and stained by crystal violet. Colony number and area were determined by ImageJ software.

### Pseudopupil assay

Wild-type and transgenic *Drosophila* strains carrying *gmr-GAL4* and *UAS-α-syn(Auluck et al.*, *2002)* were used. *gmr-GAL4* female flies were crossed with *w¹¹¹⁸* (control) or *UAS-α-syn* male flies, and raised at 21.5°C on cornmeal medium supplemented with drug(s) or vehicle control. Drug treatment was performed by adding vorinostat (LC Laboratories, V-8477) and/or 17-DMAG (InvivoGen, ant-dgl-25) into 2 ml of the medium at final concentrations of 0.5 µM and 96 µg/ml, respectively. After eclosion, progeny were transferred into a new vial with medium supplemented with fresh drug(s), and the drug(s) were changed every 3-4 days prior to the assay. Eyes of 7- to 11-day-old flies were examined under a light microscope (Olympus CX31) with a 60X oil objective as described previously(Wong et al., 2008). At least 100 ommatidia from 5-10 flies were examined and the number of rhabdomeres were recorded.

### RNA-seq

Total RNAs were isolated from drug-treated OVCAR8-ADR cells by MiniBEST universal RNA extraction kit (Takara). RNA samples were quantified and analysed using Qubit assay and high-sensitivity DNA chip (Agilent) on an Agilent 2100 Bioanalyzer, respectively. RNA-seq experiments were performed at the Centre for Genomic Sciences (LKS Faculty of Medicine, The University of Hong Kong). The Illumina adaptors of the paired-end raw sequence reads were trimmed by Trimmomatic 0.39. The STAR aligner version 2.7 was used to align the sequence reads to the human genome, where the genome index was built using the primary assembly of Gencode's version 30 release of the human genome. The raw count reads were extracted using the R package Rsubread. R packages EdgeR, limma, and HTSFilter were used for differential expression analysis comparing each of the pairwise and three-way drug combinations with the untreated samples. An FDR < 0.05 filter was applied for the three-way combination versus untreated samples while an FDR < 1 filter was applied for each of the two-way combinations versus untreated samples. The combinations and the genes were clustered by complete-linkage clustering, where the distance is defined as 1-Pearson correlation. The genes that were at least 20% up- or down- regulated in cells treated with the three-drug regimen when compared to the untreated control were inputted into DAVID web tools for pathway analysis, and the Reactome pathway database was used. The pathway mapping used a P = 0.05 threshold.

### Quantification and statistical analysis

Data analyses were performed using GraphPad Prism 7 software (GraphPad Software). Data expressed are mean ± SD, biological replicates are specified for each experiment in figure legends. Statistical comparisons between two groups were carried out by Student t-test, whereas one-way ANOVA followed by Tukey's or Dunnett's post hoc tests were used for comparisons of groups more than two.

### Data and Code Availability

All sequencing data generated or analysed during this study are available.

### Key Resources Table

| REAGENT or RESOURCE | \| SOURCE | IDENTIFIER |
|---|---|---|
| Chemicals, Peptides, and Recombinant Proteins | | |
| Zeocin | Life Technologies | Cat#R25001 |
| FuGene HD transfection reagent | Promega | Cat#E2312 |
| Polybrene | Sigma | Cat#TR-1003-G |
| Propidium iodide | Invitrogen | Cat#BMS500PI |
| Rotenone | Abcam | Cat#ab143145 |
| MPP⁺ | Abcam | Cat#ab144783 |
| Azacitidine | LC Laboratories | Cat#A-5959 |
| Olaparib | LC Laboratories | Cat#O-9201 |
| Sirolimus | LC Laboratories | Cat#R-5000 |
| Seliciclib | LC Laboratories | Cat#R-1234 |
| Lapatinib | LC Laboratories | Cat#L-4899 |
| Erlotinib | LC Laboratories | Cat#E4007 |
| Vorinostat | LC Laboratories | Cat#V-8477 |
| Fludarabine | Cayman Chemical Company | Cat#14128 |
| 17-DMAG | InvivoGen | Cat#ant-dgl-25 |
| Critical Commercial Assays | | |
| Quant-iT PicoGreen dsDNA Assay kit | Life Technologies | Cat#P11496 |
| Kapa HiFi Hotstart Ready-mix | Kapa Biosystems | Cat#KK2602 |
| Agencourt AMPure XP beads | Beckman Coulter Genomics | Cat#A63881 |
| Kapa SYBR Fast qPCR Master Mix | Kapa Biosystems | Cat#KK4600 |
| Experimental Models: Cell Lines | | |
| HEK293T (female) | ATCC | CRL-3216 |
| SK-N-MC (female) | ATCC | HTB-10 |
| OVCAR8-ADR (female) | Honma et al., 2008 | N/A |
| KURAMOCHI (female) | JCRB | JCRB0098 |
| OVSAHO (female) | JCRB | JCRB1046 |
| Experimental Models: Organisms/Strains | | |
| *GMR-GAL4* (*Drosophila* strain) | FlyBase | FBtp0018010 |
| *UAS-α-synuclein* (*Drosophila* strain) | FlyBase | FBtp0012468 |
| Oligonucleotides | | |
| All gRNAs used are listed in Table S3 | This paper | N/A |
| Recombinant DNA | | |
| All plasmid constructs are listed in Table S5 | This paper | N/A |
| Software and Algorithms | | |
| inDelphi | Shen et al., 2018 | http://indelphi.giffordl ab.mit.edu/ |
| FORECasT | Allen et al., 2019 | https://github.com/felic ityallen/SelfT arget |
| FlowJo v10.5.3 | Becton Dickinson | N/A |
| R package DescTools | Signorell et al., 2019 | https://CRAN.R-project.org/package=D escTools |
| ImageJ | Schneider et al., 2012 | https://imagej.nih.gov/i j/ |
| R package Rsubread | Liao et al., 2019 | 10.18129/B9.bioc.Rsu bread |
| R packages EdgeR | Robinson et al, 2010 | 10.18129/B9.bioc.edge R |
| limma | Ritchie et al., 2015 | 10.18129/B9.bioc.lim ma |
| HTSFilter | Rau et al., 2013 | 10.18129/B9.bioc.HTS Filter |
| DAVID web tools | Huang et al., 2009 | https://david.ncifcrf.go v/ |
| Reactome pathway database | Fabregat et al., 2018 | https://reactome.org/ |
| GraphPad Prism 7 | GraphPad Software | N/A |

### References

Adamson, B., Norman, T.M., Jost, M., Cho, M.Y., Nunez, J.K., Chen, Y., Villalta, J.E., Gilbert, L.A., Horlbeck, M.A., Hein, M.Y., et al. (2016). A Multiplexed Single-Cell CRISPR Screening Platform Enables Systematic Dissection of the Unfolded Protein Response. Cell 167, 1867-1882 e1821.
Aguirre, A.J., Meyers, R.M., Weir, B.A., Vazquez, F., Zhang, C.Z., Ben-David, U., Cook, A., Ha, G., Harrington, W.F., Doshi, M.B., et al. (2016). Genomic Copy Number Dictates a Gene-Independent Cell Response to CRISPR/Cas9 Targeting. Cancer Discov 6, 914-929.
Al-Lazikani, B., Banerji, U., and Workman, P. (2012). Combinatorial drug therapy for cancer in the post-genomic era. Nat Biotechnol 30, 679-692.
Allen, F., Crepaldi, L., Alsinet, C., Strong, A.J., Kleshchevnikov, V., De Angeli, P., Palenikova, P., Khodak, A., Kiselev, V., Kosicki, M., et al. (2018). Predicting the mutations generated by repair of Cas9-induced double-strand breaks. Nat Biotechnol.
Auluck, P.K., Chan, H.Y., Trojanowski, J.Q., Lee, V.M., and Bonini, N.M. (2002). Chaperone suppression of alpha-synuclein toxicity in a Drosophila model for Parkinson's disease. Science 295, 865-868.
Baratta, M.G., Schinzel, A.C., Zwang, Y., Bandopadhayay, P., Bowman-Colin, C., Kutt, J., Curtis, J., Piao, H., Wong, L.C., Kung, A.L., et al. (2015). An in-tumor genetic screen reveals that the BET bromodomain protein, BRD4, is a potential therapeutic target in ovarian carcinoma. Proc Natl Acad Sci U S A 112, 232-237.
Bassik, M.C., Kampmann, M., Lebbink, R.J., Wang, S., Hein, M.Y., Poser, I., Weibezahn, J., Horlbeck, M.A., Chen, S., Mann, M., et al. (2013). A systematic mammalian genetic interaction map reveals pathways underlying ricin susceptibility. Cell 152, 909-922.
Bliss, C. (1939). The toxicity of poisons applied jointly. Annals of applied biology 26, 585-615.
Borisy, A.A., Elliott, P.J., Hurst, N.W., Lee, M.S., Lehar, J., Price, E.R., Serbedzija, G., Zimmermann, G.R., Foley, M.A., Stockwell, B.R., et al. (2003). Systematic discovery of multicomponent therapeutics. Proc Natl Acad Sci U S A 100, 7977-7982.
Bowtell, D.D. (2010). The genesis and evolution of high-grade serous ovarian cancer. Nat Rev Cancer 10, 803-808.
Bozic, I., Reiter, J.G., Allen, B., Antal, T., Chatterjee, K., Shah, P., Moon, Y.S., Yaqubie, A., Kelly, N., Le, D.T., et al. (2013). Evolutionary dynamics of cancer in response to targeted combination therapy. Elife 2, e00747.
Briner, A.E., Donohoue, P.D., Gomaa, A.A., Selle, K., Slorach, E.M., Nye, C.H., Haurwitz, R.E., Beisel, C.L., May, A.P., and Barrangou, R. (2014). Guide RNA functional modules direct Cas9 activity and orthogonality. Mol Cell 56, 333-339.
Chow, R.D., Wang, G., Ye, L., Codina, A., Kim, H.R., Shen, L., Dong, M.B., Errami, Y., and Chen, S. (2019). In vivo profiling of metastatic double knockouts through CRISPR-Cpfl screens. Nat Methods 16, 405-408.
Cokol, M., Kuru, N., Bicak, E., Larkins-Ford, J., and Aldridge, B.B. (2017). Efficient measurement and factorization of high-order drug interactions in Mycobacterium tuberculosis. Sci Adv 3, e1701881.
Cokol-Cakmak, M., Bakan, F., Cetiner, S., and Cokol, M. (2018). Diagonal Method to Measure Synergy Among Any Number of Drugs. J Vis Exp.
Dang, Y., Jia, G., Choi, J., Ma, H., Anaya, E., Ye, C., Shankar, P., and Wu, H. (2015). Optimizing sgRNA structure to improve CRISPR-Cas9 knockout efficiency. Genome Biol 16, 280.
Doench, J.G. (2018). Am I ready for CRISPR? A user's guide to genetic screens. Nat Rev Genet 19, 67-80.
Doench, J.G., Fusi, N., Sullender, M., Hegde, M., Vaimberg, E.W., Donovan, K.F., Smith, I., Tothova, Z., Wilen, C., Orchard, R., et al. (2016). Optimized sgRNA design to maximize activity and minimize off-target effects of CRISPR-Cas9. Nat Biotechnol 34, 184-191.
Du, D., Roguev, A., Gordon, D.E., Chen, M., Chen, S.H., Shales, M., Shen, J.P., Ideker, T., Mali, P., Qi, L.S., et al. (2017). Genetic interaction mapping in mammalian cells using CRISPR interference. Nat Methods 14, 577-580.
Engler, C., Kandzia, R., and Marillonnet, S. (2008). A one pot, one step, precision cloning method with high throughput capability. PLoS One 3, e3647.
Gholami, A.M., Hahne, H., Wu, Z., Auer, F.J., Meng, C., Wilhelm, M., and Kuster, B. (2013). Global proteome analysis of the NCI-60 cell line panel. Cell Rep 4, 609-620.
Gibson, D.G., Young, L., Chuang, R.Y., Venter, J.C., Hutchison, C.A., 3rd, and Smith, H.O. (2009). Enzymatic assembly of DNA molecules up to several hundred kilobases. Nat Methods 6, 343-345.
Grevet, J.D., Lan, X., Hamagami, N., Edwards, C.R., Sankaranarayanan, L., Ji, X., Bhardwaj, S.K., Face, C.J., Posocco, D.F., Abdulmalik, O., et al. (2018). Domain-focused CRISPR screen identifies HRI as a fetal hemoglobin regulator in human erythroid cells. Science 361, 285-290.
Han, K., Jeng, E.E., Hess, G.T., Morgens, D.W., Li, A., and Bassik, M.C. (2017). Synergistic drug combinations for cancer identified in a CRISPR screen for pairwise genetic interactions. Nat Biotechnol 35, 463-474.
Honma, K., Iwao-Koizumi, K., Takeshita, F., Yamamoto, Y., Yoshida, T., Nishio, K., Nagahara, S., Kato, K., and Ochiya, T. (2008). RPN2 gene confers docetaxel resistance in breast cancer. Nat Med 14, 939-948.
Ma, H., Wu, Y., Dang, Y., Choi, J.G., Zhang, J., and Wu, H. (2014). Pol III Promoters to Express Small RNAs: Delineation of Transcription Initiation. Mol Ther Nucleic Acids 3, e161.
Munoz, D.M., Cassiani, P.J., Li, L., Billy, E., Korn, J.M., Jones, M.D., Golji, J., Ruddy, D.A., Yu, K., McAllister, G., et al. (2016). CRISPR Screens Provide a Comprehensive Assessment of Cancer Vulnerabilities but Generate False-Positive Hits for Highly Amplified Genomic Regions. Cancer Discov 6, 900-913.
Najm, F.J., Strand, C., Donovan, K.F., Hegde, M., Sanson, K.R., Vaimberg, E.W., Sullender, M.E., Hartenian, E., Kalani, Z., Fusi, N., et al. (2018). Orthologous CRISPR-Cas9 enzymes for combinatorial genetic screens. Nat Biotechnol 36, 179-189.
Qi, L.S., Larson, M.H., Gilbert, L.A., Doudna, J.A., Weissman, J.S., Arkin, A.P., and Lim, W.A. (2013). Repurposing CRISPR as an RNA-guided platform for sequence-specific control of gene expression. Cell 152, 1173-1183.
Reinhold, W.C., Sunshine, M., Liu, H., Varma, S., Kohn, K.W., Morris, J., Doroshow, J., and Pommier, Y. (2012). CellMiner: a web-based suite of genomic and pharmacologic tools to explore transcript and drug patterns in the NCI-60 cell line set. Cancer Res 72, 3499-3511.
Reis, A.C., Halper, S.M., Vezeau, G.E., Cetnar, D.P., Hossain, A., Clauer, P.R., and Salis, H.M. (2019). Simultaneous repression of multiple bacterial genes using nonrepetitive extra-long sgRNA arrays. Nat Biotechnol.
Shalem, O., Sanjana, N.E., Hartenian, E., Shi, X., Scott, D.A., Mikkelsen, T.S., Heckl, D., Ebert, B.L., Root, D.E., Doench, J.G., et al. (2014). Genome-scale CRISPR-Cas9 knockout screening in human cells. Science 343, 84-87.
Shen, J.P., Zhao, D., Sasik, R., Luebeck, J., Birmingham, A., Bojorquez-Gomez, A., Licon, K., Klepper, K., Pekin, D., Beckett, A.N., et al. (2017). Combinatorial CRISPR-Cas9 screens for de novo mapping of genetic interactions. Nat Methods 14, 573-576.
Shen, M.W., Arbab, M., Hsu, J.Y., Worstell, D., Culbertson, S.J., Krabbe, O., Cassa, C.A., Liu, D.R, Gifford, D.K., and Sherwood, R.I. (2018). Predictable and precise template-free CRISPR editing of pathogenic variants. Nature 563, 646-651.
Vidigal, J.A., and Ventura, A. (2015). Rapid and efficient one-step generation of paired gRNA CRISPR-Cas9 libraries. Nat Commun 6, 8083.
Wong, A.S., Choi, G.C., Cheng, A.A., Purcell, O., and Lu, T.K. (2015). Massively parallel high-order combinatorial genetics in human cells. Nat Biotechnol 33, 952-961.
Wong, A.S., Choi, G.C., Cui, C.H., Pregemig, G., Milani, P., Adam, M., Perli, S.D., Kazer, S.W., Gaillard, A., Hermann, M., et al. (2016a). Multiplexed barcoded CRISPR-Cas9 screening enabled by CombiGEM. Proc Natl Acad Sci U S A 113, 2544-2549.
Wong, A.S., Choi, G.C., and Lu, T.K. (2016b). Deciphering Combinatorial Genetics. Annu Rev Genet 50, 515-538.
Wong, S.L., Chan, W.M., and Chan, H.Y. (2008). Sodium dodecyl sulfate-insoluble oligomers are involved in polyglutamine degeneration. FASEB J 22, 3348-3357.
Wood, K., Nishida, S., Sontag, E.D., and Cluzel, P. (2012). Mechanism-independent method for predicting response to multidrug combinations in bacteria. Proc Natl Acad Sci U S A 109, 12254-12259.
Xu, L., Zhao, L., Gao, Y., Xu, J., and Han, R. (2017). Empower multiplex cell and tissue-specific CRISPR-mediated gene manipulation with self-cleaving ribozymes and tRNA. Nucleic Acids Res 45, e28.
Zimmer, A., Katzir, I., Dekel, E., Mayo, A.E., and Alon, U. (2016). Prediction of multidimensional drug dose responses based on measurements of drug pairs. Proc Natl Acad Sci U S A 113, 10442-10447.
Coscia, F., Watters, K.M., Curtis, M., Eckert, M.A., Chiang, C.Y., Tyanova, S., Montag, A., Lastra, R.R., Lengyel, E., and Mann, M. (2016). Integrative proteomic profiling of ovarian cancer cell lines reveals precursor cell associated proteins and functional status. Nat Commun 7, 12645.
Domcke, S., Sinha, R., Levine, D.A., Sander, C., and Schultz, N. (2013). Evaluating cell lines as tumour models by comparison of genomic profiles. Nat Commun 4, 2126.
Gholami, A.M., Hahne, H., Wu, Z., Auer, F.J., Meng, C., Wilhelm, M., and Kuster, B. (2013). Global proteome analysis of the NCI-60 cell line panel. Cell Rep 4, 609-620.
McClue, S.J., Blake, D., Clarke, R., Cowan, A., Cummings, L., Fischer, P.M., MacKenzie, M., Melville, J., Stewart, K., Wang, S., et al. (2002). In vitro and in vivo antitumor properties of the cyclin dependent kinase inhibitor CYC202 (R-roscovitine). Int J Cancer 102, 463-468.
Muvarak, N.E., Chowdhury, K., Xia, L., Robert, C., Choi, E.Y., Cai, Y., Bellani, M., Zou, Y., Singh, Z.N., Duong, V.H., et al. (2016). Enhancing the Cytotoxic Effects of PARP Inhibitors with DNA Demethylating Agents - A Potential Therapy for Cancer. Cancer Cell 30, 637-650.
Pulliam, N., Fang, F., Ozes, AR, Tang, J., Adewuyi, A., Keer, H., Lyons, J., Baylin, S.B., Matei, D., Nakshatri, H., et al. (2018). An Effective Epigenetic-PARP Inhibitor Combination Therapy for Breast and Ovarian Cancers Independent of BRCA Mutations. Clin Cancer Res 24, 3163-3175.
Sabers, C.J., Martin, M.M., Brunn, G.J., Williams, J.M., Dumont, F.J., Wiederrecht, G., and Abraham, R.T. (1995). Isolation of a protein target of the FKBP12-rapamycin complex in mammalian cells. J Biol Chem 270, 815-822.
Shalem, O., Sanjana, N.E., Hartenian, E., Shi, X., Scott, D.A., Mikkelsen, T.S., Heckl, D., Ebert, B.L., Root, D.E., Doench, J.G., et al. (2014). Genome-scale CRISPR-Cas9 knockout screening in human cells. Science 343, 84-87.
Wishart, D.S., Knox, C., Guo, A.C., Shrivastava, S., Hassanali, M., Stothard, P., Chang, Z., and Woolsey, J. (2006). DrugBank: a comprehensive resource for in silico drug discovery and exploration. Nucleic Acids Res 34, D668-672.
Wong, A.S., Choi, G.C., Cheng, A.A., Purcell, O., and Lu, T.K. (2015). Massively parallel high-order combinatorial genetics in human cells. Nat Biotechnol 33, 952-961.
Wong, A.S., Choi, G.C., Cui, C.H., Pregemig, G., Milani, P., Adam, M., Perli, S.D., Kazer, S.W., Gaillard, A., Hermann, M., et al. (2016). Multiplexed barcoded CRISPR-Cas9 screening enabled by CombiGEM. Proc Natl Acad Sci U S A 113, 2544-2549.
Yang, L., Zhang, Y., Shan, W., Hu, Z., Yuan, J., Pi, J., Wang, Y., Fan, L., Tang, Z., Li, C., et al. (2017). Repression of BET activity sensitizes homologous recombination-proficient cancers to PARP inhibition. Sci Transl Med 9.

The foregoing description of the specific embodiments will so fully reveal the general nature of the disclosure that others can, by applying knowledge within the skill of the relevant art(s) readily modify and/or adapt for various applications such specific embodiments, without undue experimentation, without departing from the general concept of the present disclosure. Such adaptations and modifications are therefore intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one skilled in the relevant art(s).

While various embodiments of the present disclosure have been described above, it should be understood that they have been presented by way of examples, and not limitation.

Thus, the present disclosure should not be limited by any of the above-described exemplary embodiments but should be defined only in accordance with the following claims.

## Claims

1. A system for CRISPR-based multi-gene knockout screening comprising a barcoded gRNA expression cassette comprising: (i) a first promoter operatively linked to a first gRNA; (ii) a second promoter operatively linked to a second gRNA; (iii) a third promoter operatively linked to a third gRNA; and (iv) three barcoded gRNA sequencing region, wherein the gRNA expression cassette is in a single vector;
wherein the promoters are human U6, mouse U6, and Human H1 promoters and the three barcoded gRNAs comprise wild-type and modified gRNA scaffold variants;
wherein each of the promoters comprise the same modified 3' end sequence which are complementary to the barcoded gRNA oligonucleotides, said 3' end sequence anneals to the barcoded gRNA oligonucleotides; and
wherein the system further comprises pooled digestion and ligation of the annealed 3' end sequence and the barcoded gRNA oligonucleotides to form an assembly of pooled barcoded combinatorial gRNA library.

2. The system of claim 1, wherein the expression cassettes knockout target GFP gene in OVACR8-ADR-Cas9 cells.

3. The system of any preceding claim, wherein the gRNA scaffold variants comprises: (i) higher on-target activity than wild-type scaffold; (ii) low off-target activities; and (iii) high on-to-off target activity.

4. The system of any preceding claim, wherein the vector is a lentiviral vector.

5. The system of any preceding claim, wherein a combinatorial gRNA library is assembled by CombiGEM-CRISPR v2.0.

6. The system of claim 4, wherein the lentiviral vector transfects human cells and the barcoded gRNA oligonucleotides are delivered to the human cells.

7. The system of any preceding claim, wherein the system further comprises quantitation of barcoded gRNA oligonucleotides using next-generation sequencing at a time point post transfection.

8. The system of any preceding claim, wherein the CRISPR-based multi-gene knockout screen is a high-throughput screen.

9. The system of any preceding claim, wherein the modified gRNA scaffold variants comprise v3.11, v. 3.12 or v. 3.13 gRNA scaffold variants.

## Patentansprüche

1. System zum CRISPR-basierten Multi-Gen-Knockout-Screening, umfassend eine barcodierte gRNA-Expressionskassette, umfassend: (i) einen ersten Promotor, der funktionsfähig mit einer ersten gRNA verbunden ist; (ii) einen zweiten Promotor, der funktionsfähig mit einer zweiten gRNA verbunden ist; (iii) einen dritten Promotor, der funktionsfähig mit einer dritten gRNA verbunden ist; und (iv) drei barcodierte gRNA-Sequenzierungsbereiche, wobei sich die gRNA-Expressionskassette in einem einzigen Vektor befindet;
wobei die Promotoren humane U6-, Maus-U6- und humane H1-Promotoren sind und die drei barcodierten gRNAs Wildtyp- und modifizierte gRNA-Gerüstvarianten umfassen;
wobei jeder der Promotoren die gleiche modifizierte 3'-Endsequenz umfasst, die komplementär zu den barcodierten gRNA-Oligonukleotiden ist, wobei die 3' -Endsequenz an die barcodierten gRNA-Oligonukleotide anlagert; und
wobei das System ferner eine gepoolte Verdauung und Ligation der annealisierten 3'-Endsequenz und der barcodierten gRNA-Oligonukleotide umfasst, um eine Anordnung einer gepoolten barcodierten kombinatorischen gRNA-Bibliothek zu bilden.

2. System nach Anspruch 1, wobei die Expressionskassetten das Knockout-Ziel-GFP-Gen in OVACR8-ADR-Cas9-Zellen enthält.

3. System nach einem vorhergehenden Anspruch, wobei die gRNA-Gerüstvarianten umfassen: (i) höhere Zielaktivität als Wildtyp-Gerüst; (ii) niedrige Zielabweichungsaktivitäten; und (iii) hohe Zielansprechaktivität.

4. System nach einem vorhergehenden Anspruch, wobei der Vektor ein lentiviraler Vektor ist.

5. System nach einem vorhergehenden Anspruch, wobei eine kombinatorische gRNA-Bibliothek durch CombiGEM-CRISPR v2.0 zusammengestellt wird.

6. System nach Anspruch 4, wobei der lentivirale Vektor humane Zellen transfiziert und die barcodierten gRNA-Oligonukleotide an die humanen Zellen abgegeben werden.

7. System nach einem vorhergehenden Anspruch, wobei das System ferner die Quantifizierung von barcodierten gRNA-Oligonukleotiden unter Verwendung einer Sequenzierung der nächsten Generation zu einem Zeitpunkt nach der Transfektion umfasst.

8. System nach einem vorhergehenden Anspruch, wobei der CRISPR-basierte Multi-Gen-Knockout-Screen ein Hochdurchsatz-Screen ist.

9. System nach einem vorhergehenden Anspruch, wobei die modifizierten gRNA-Gerüstvarianten v3.11, v. 3.12 oder v. 3.13 gRNA-Gerüstvarianten umfassen.

## Revendications

1. Système pour un criblage par invalidation multigénique basé sur CRISPR comprenant une cassette d'expression d'ARNg à code-barres comprenant : (i) un premier promoteur lié de manière opérationnelle à un premier ARNg ; (ii) un deuxième promoteur lié de manière opérationnelle à un deuxième ARNg ; (iii) un troisième promoteur lié de manière opérationnelle à un troisième ARNg ; et (iv) trois régions de séquençage d'ARNg à code-barres, dans lequel la cassette d'expression d'ARNg se trouve dans un seul vecteur ;
dans lequel les promoteurs sont des promoteurs U6 humain, U6 de souris et H1 humain et les trois ARNg à code-barres comprennent des variants d'échafaudage d'ARNg de type sauvage et modifiés ;
dans lequel chacun des promoteurs comprend la même séquence d'extrémité 3' modifiée qui est complémentaire aux oligonucléotides d'ARNg à code-barres, ladite séquence d'extrémité 3' s'hybride aux oligonucléotides d'ARNg à code-barres ; et
dans lequel le système comprend en outre une digestion et une ligature groupées de la séquence d'extrémité 3' hybridée et des oligonucléotides d'ARNg à code-barres pour former un ensemble de banque d'ARNg combinatoire groupée à code-barres.

2. Système de la revendication 1, dans lequel les cassettes d'expression invalident un gène GFP cible dans des cellules OVACR8-ADR-Cas9.

3. Système d'une quelconque revendication précédente, dans lequel les variants d'échafaudage d'ARNg comprennent : (i) une activité sur cible supérieure à celle d'un échafaudage de type sauvage ; (ii) de faibles activités hors cible ; et (iii) une activité sur cible/hors cible élevée.

4. Système d'une quelconque revendication précédente, dans lequel le vecteur est un vecteur lentiviral.

5. Système d'une quelconque revendication précédente, dans lequel une banque combinatoire d'ARNg est assemblée par CombiGEM-CRISPR v2.0.

6. Système de la revendication 4, dans lequel le vecteur lentiviral transfecte des cellules humaines et les oligonucléotides d'ARNg à code-barres sont administrés aux cellules humaines.

7. Système d'une quelconque revendication précédente, dans lequel le système comprend en outre la quantification d'oligonucléotides d'ARNg à code-barres en utilisant un séquençage de nouvelle génération à un instant temporel après la transfection.

8. Système d'une quelconque revendication précédente, dans lequel l'écran d'invalidation multigénique basé sur CRISPR est un écran à haut débit.

9. Système d'une quelconque revendication précédente, dans lequel les variants d'échafaudage d'ARNg modifiés comprennent des variants d'échafaudage d'ARNg v3.11, v. 3.12 ou v. 3.13.
